(19) Europäisches Patentamt
European Patent Office
Office européen des brevets

(11) EP 0 613 422 B1

(12) **EUROPEAN PATENT SPECIFICATION**

(45) Date of publication and mention of the grant of the patent: **21.08.1996 Bulletin 1996/34**

(21) Application number: **93900560.9**

(22) Date of filing: **20.11.1992**

(51) Int. Cl.$^6$: **B41M 5/40**, C07D 311/58, C09B 57/00, C07D 335/06, C07D 335/02, C07D 345/00, C07D 409/08

(86) International application number: **PCT/US92/09992**

(87) International publication number: **WO 93/09956 (27.05.1993 Gazette 1993/13)**

(54) **SQUARYLIUM DYES**

SQUARYLIUMFARBSTOFFE

COLORANTS AU SQUARYLIUM

(84) Designated Contracting States: **DE FR GB IT NL SE**

(30) Priority: **20.11.1991 US 795034**
**20.11.1991 US 795341**

(43) Date of publication of application: **07.09.1994 Bulletin 1994/36**

(73) Proprietor: **POLAROID CORPORATION Cambridge, Massachusetts 02139 (US)**

(72) Inventors:
- **ALLEN, Richard, M. Norwood, MA 02766 (US)**
- **CHU, Peter K. Acton, MA 01720 (US)**
- **LEE, John, W. Still River, MA 01467 (US)**
- **MCGOWAN, Donald, A. Bedford, MA 01730 (US)**
- **MISCHKE, Mark, R. Arlington, MA 02174 (US)**
- **RAMOS, Socorro, M. Belmont, MA 02178 (US)**
- **TELFER, Stephen, J. Arlington, MA 02174 (US)**

(74) Representative: **Reitzner, Bruno, Dr. et al Patentanwälte Dipl.-Ing. R. Splanemann Dr. B. Reitzner, Dipl.-Ing. K. Baronetzky Tal 13 80331 München (DE)**

(56) References cited:
**WO-A-92/09661** **US-A- 5 019 549**

- **DYES AND PIGMENTS vol. 11, no. 1, 1989, BARKING, ESSEX, GB pages 21 - 35 N. KURAMOTO ET AL 'Synthesis and characterization of deep-coloured squarylium dyes for laser optical recording media.'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 613 422 B1

Printed by Rank Xerox (UK) Business Services
2.13.2/3.4

## Description

This invention relates to squarylium dyes, and processes and intermediates for the preparation thereof. More specifically, it relates to such dyes and intermediates in which the squarylium ring bears a nitrogen or carbon containing group, and to certain intermediates useful for the preparation of the aforementioned dyes.

It is known that compounds in which two chromophoric groups are linked by a pentamethine chain, the three central carbon atoms of which form part of a squarylium ring, are useful as dyes, especially near infrared dyes. (The term "near infra-red" is used herein to mean electromagnetic radiation having a wavelength of about 700 to about 1200 nm.)

The term "chromophoric group" is used herein to mean a group containing a plurality of conjugated unsaturated linkages arranged so that the unsaturated linkages are conjugated with the squarylium ring via the unsaturated ($sp^2$) meso carbon atom lying between the chromophoric group and the squarylium ring, the chromophoric group being such that the squarylium dye has substantial absorption of visible or infra-red radiation.

For example, Japanese Patent Application No. 103,604/82 (Publication No. 220,143/83, published December 21, 1983), discloses a broad class of bis-heterocyclic pentamethine dyes in which the central three carbon atoms of the pentamethine chain form part of a squarylium or croconylium ring. The heterocyclic nuclei can be pyrylium, thiopyrylium, selenopyrylium, benzpyrylium, benzthiopyrylium, benzselenopyrylium, naphthopyrylium, naphthothiopyrylium or naphthoselenopyrylium nuclei, which can be substituted with alkyl, alkoxy, aryl or styryl groups.

Japanese Patent Application No. 60-8730 (Publication No. 167,681/86, published July 29, 1986), discloses bis(4-benz[b]thiopyrylium) pentamethine dyes in which the central three carbon atoms of the pentamethine chain form part of a squarylium ring. The dyes are intended for use as infra-red absorbers.

U.S. Patent No. 4,508,811, issued April 2, 1985, describes an optical recording element in which the recording layer comprises a bis(2,6-dialkyl)-pyrylium or -thiopyrylium squarylium salt.

The squarylium dyes disclosed in these Japanese applications and U.S. patent are capable of achieving high extinction coefficients in the near infra-red range. However, such squarylium dyes suffer from a number of disadvantages. Many of these prior art dyes have low solubility in most plastics and/or in semi-polar solvents (for example, methyl ethyl ketone and methylene chloride) from which they need to be deposited to form imaging media. Thus, it is difficult to dissolve or disperse the absorber in a plastic without forming large aggregates and without adversely affecting other properties of the plastic.

A related disadvantage is that, unless specific functional groups are provided on the chromophoric groups (and the presence of such functional groups on the chromophoric groups may cause problems in the synthesis of the compounds from which the chromophoric groups are derived, or in the condensation of these compounds with squaric acid or its derivatives to form the final dyes), there is no convenient site (or "handle") on the squarylium dye for attachment of functional groups. Attachment of functional groups to the squarylium ring may be desirable, for example, to change the solubility of the dye in, or its compatibility with, various media, or to permit the dye to be chemically bonded to other materials.

Thirdly, among the squarylium dyes disclosed in these Japanese applications and U.S. patent, it may be difficult to find a dye which absorbs at the precise wavelength required for a particular application. For example, when choosing infrared absorbers for use in imaging media such as those described in International Patent Application No. PCT/US91/08695 (Publication No. WO 92/09661, part of which forms prior art under Article 54(3)(4) EPC), the need for independent addressing of the three color-forming layers, coupled with the widths of the peaks (typically the full-width-half-maximum (FWHM) of these peaks is about 35-40 nm) and the limited wavelength range over which present semiconductor lasers can be produced economically, mean that it is often necessary to find an infra-red absorber which has an absorption peak within a very narrow range (say 10-15 nm) and which meets all the other requirements of stability, solubility and compatibility with other components of the imaging medium required for use in such an imaging medium. It is often difficult if not impossible to find a squarylium dye from among those disclosed in the Japanese applications and U.S. patent which absorbs within such a narrow wavelength range.

The aforementioned disadvantages of earlier prior art squarylium dyes are greatly reduced in the dyes described in the aforementioned International Application. The 2-non-aromatic substituted dyes described in this International Application are substantially more soluble than the corresponding 2-phenyl dyes, while the asymmetric dyes which can be synthesized in good yields by the processes described in European patent 516985, which forms prior art under Article 54(3)(4) EPC, greatly ease the task of finding a dye which absorbs at a desired wavelength, since the ability to change the two chromophoric groups independently gives an additional degree of freedom, as compared with earlier dyes in which the two chromophoric groups were the same. However, neither of these copending applications describes dyes in which functional groups are provided on the squarylium ring itself. Furthermore, there are still situations in which it would be advantageous to provide dyes with even greater solubility in certain media than those described in the aforementioned International and European Applications, and it would also be advantageous to provide some way in which the dyes disclosed in these applications could be "fine tuned" by shifting their infra-red absorption peaks over substantial ranges (say 30 nm or greater) in order to assist in providing dyes having absorptions within very narrow desired ranges. Furthermore, when providing infra-red dyes for use in thermal imaging media such as those described in the

aforementioned International and European Applications, in which three infra-red dyes having widely-spaced absorptions are desired, it may be advantageous from a manufacturing point of view to use a set of infra-red dyes which are chemically closely related so that they share certain synthetic intermediates, with the necessary spacing in absorption wavelength among the final infra-red dyes being provided by varying substituents in the final dye.

It has now been found that providing certain substituents in place of one of the oxygen atoms of the squarylium ring in squarylium dyes allows the absorption peak of the dye to be shifted somewhat, allows various functional groups to be incorporated conveniently into the dye, and may increase the solubility of the dye in, or its compatibility with, certain media. The substituent on the squarylium ring also provides a potential means for chemically bonding the dye to other materials. Accordingly, this invention is directed to these substituted dyes, to processes and intermediates for the preparation of such dyes, and to processes and imaging media containing these dyes.

This invention provides a squarylium compound of the formula:

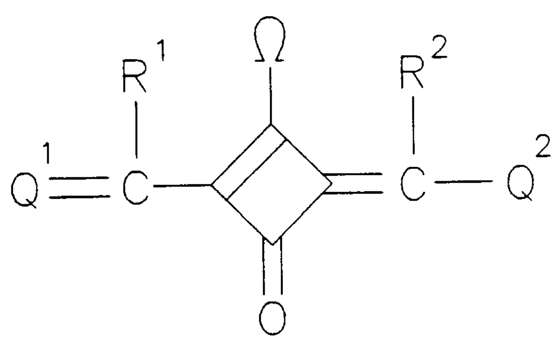

(I)

in which:

$Q^1$ and $Q^2$ are each a chromophoric group having an aromatic unsaturated system conjugated with the squarylium ring and such that in the compounds of formulae $Q^1CH_2R^1$ and $Q^2CH_2R^2$ the methylene hydrogens are active hydrogens;

$R^1$ and $R^2$ are each independently a hydrogen atom or an aliphatic or cycloaliphatic group; and

$\Omega$ is a group of the formula $-NR^3R^4$ or of the formula $-CR^5R^6R^7$, wherein:

$R^3$ and $R^4$ are each independently a hydrogen atom, or an acyl, aliphatic, cycloaliphatic, aromatic or heterocyclic group, subject to the proviso that one of $R^3$ and $R^4$ may be an amino or substituted amino group, or one of $R^3$ and $R^4$ is a hydrogen atom and the other is an organosulfonyl group, or $R^3$ and $R^4$ together with the intervening nitrogen atom form a nitrogenous heterocyclic ring; and

$R^5$, $R^6$ and $R^7$ are each independently a hydrogen atom, or an acyl, aliphatic, cycloaliphatic, aromatic or heterocyclic group, or an electron-withdrawing group able to lower the electron density at the carbon atom to which it is attached, subject to the provisoes that:

two of $R^5$, $R^6$ and $R^7$ may form a divalent group of which a single atom is double bonded to the carbon atom to which the two groups are attached, or all three of $R^5$, $R^6$ and $R^7$ may form a trivalent group of which a single atom is triple bonded to the carbon atom to which the three groups are attached, or

two of $R^5$, $R^6$ and $R^7$ may, together with the carbon atom to which they are attached, form a ring, or all three of $R^5$, $R^6$ and $R^7$ may, together with the carbon atom to which they are attached, form an unsaturated ring.

In certain cases hereinafter, it may be necessary to distinguish between the compounds of Formula I in $\Omega$ is of the formula $-NR^3R^4$ and those in which $\Omega$ is of the formula $-CR^5R^6R^7$; in such cases, the former will be referred to as the "2-N" compounds of the invention and the latter of as the "2-C" compounds of the invention.

The 2-N compounds of Formula I in which one of $R^3$ and $R^4$ is (notionally) a hydrogen atom and the other is an organosulfonyl group (i.e., in which a $-SO_2NH-$ grouping is directly attached to the squarylium nucleus) (these compounds are typically encountered in their deprotonated form) can be synthesized in a manner which is different from that employed for the other 2-N compounds of Formula I, in that introduction of the sulfonamide group can be effected directly into the unsubstituted squarylium dye. Accordingly when hereinafter it is necessary to distinguish the two groups of compounds, the 2-N compounds of Formula I in which one of $R^3$ and $R^4$ is (notionally) a hydrogen atom and the other is an organosulfonyl group will be referred to as the "2-N sulfonamide" compounds of Formula I, while the remaining 2-N compounds will be referred to as the "2-N non-sulfonamide" compounds. It should be noted that 2-N compounds in which one of $R^3$ and $R^4$ is a sulfonated alkyl group are 2-N non-sulfonamide compounds.

Similarly, the 2-C compounds of Formula I in which at least one of $R^5$, $R^6$ and $R^7$ is an electron-withdrawing group able to lower the electron density at the carbon atom to which it is attached are preferably synthesized in a somewhat different manner from the other 2-C compounds of Formula I. Accordingly, when it is necessary to distinguish between these two groups of dyes, the former may hereinafter be referred to as the "2-C electron-withdrawing" or "2-C EW-dyes"

of the invention, and the latter as the "2-C non-EW-dyes". Similarly, squaric acid derivatives of Formula II may be referred to as "2-C EW-derivatives" and "2-C non-EW-derivatives" depending upon the nature of the groups $R^5$, $R^6$ and $R^7$.

This invention also a squaric acid derivative of the formula:

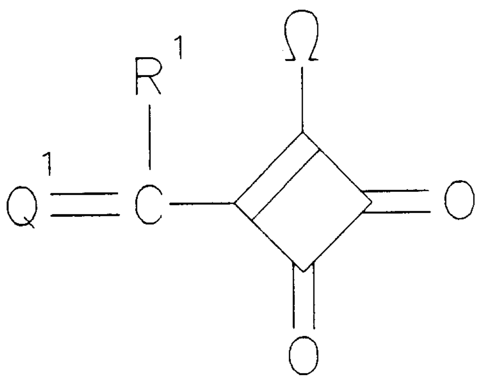

(II)

in which $Q^1$, $R^1$ and $\Omega$ are as defined above.

This invention also provides a compound of the formula:

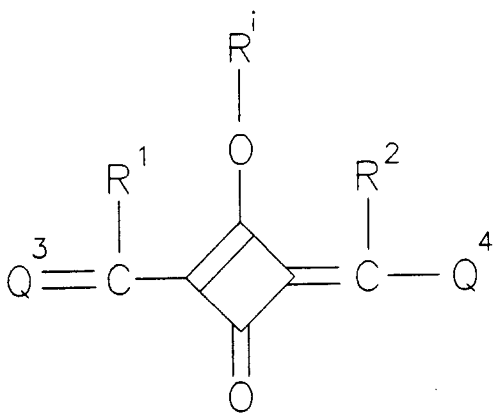

(i)

in which $R^1$ and $R^2$ are as defined above, $R^i$ is a hydrogen atom or an aliphatic or cycloaliphatic group, and $Q^3$ and $Q^4$ are each independently a pyrylium, thiopyrylium, selenopyrylium, benzpyrylium, benzthiopyrylium or benzselenopyrylium nucleus.

This invention also provides a process for the preparation of a squaric acid derivative of Formula II, which process comprises reacting a corresponding squaric acid derivative of the formula:

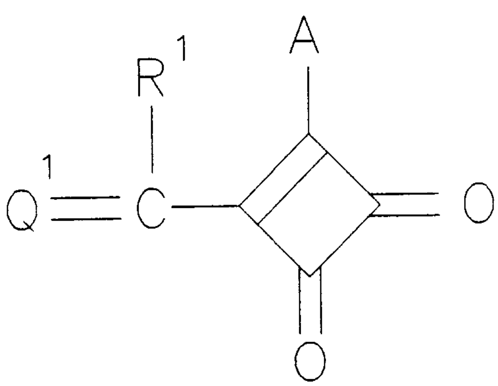

(III)

in which A is a chlorine or bromine atom, or a hydroxyl or alkoxyl group, and $Q^1$ and $R^1$ are as defined above, with a

compound of formula HΩ, in which Ω is as defined above. This process may hereinafter be referred to as the "first process of the invention".

This invention also providess a process for the preparation of a squarylium compound of Formula I above, which process comprises reacting a corresponding squaric acid derivative of Formula II above, in which $Q^1$, $R^1$ and Ω are as defined above, with a compound of the formula $Q^2CH_2R^2$, in which $Q^2$ and $R^2$ are as defined above. This process may hereinafter be referred to as the "second process of the invention".

This invention also provides a process for the preparation of a 2-N sulfonamide squarylium compound of Formula I above, which process comprises reacting the corresponding squarylium compound of the formula:

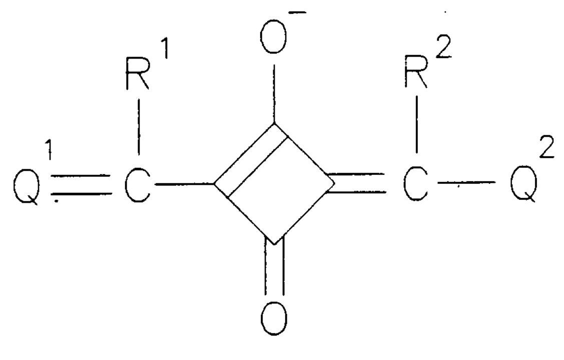

(IV)

in which $Q^1$, $Q^2$, $R^1$ and $R^2$ are as defined above, with the corresponding organosulfonyl isocyanate. This process may hereinafter be referred to as the "third process of the invention".

This invention also provides a process for the preparation of a squarylium compound of Formula I above, which process comprises reacting a corresponding Ω-substituted squaric acid monoester with at least one compound of formula $Q^1CH_2R^1$ or $Q^2CH_2R^2$. This process may hereinafter be referred to as the "fourth process of the invention".

This invention also provides a process for the preparation of a squaric acid derivative of the formula:

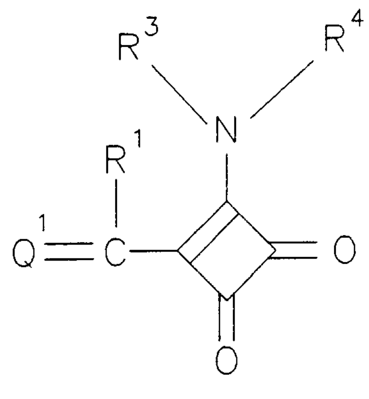

(II-N)

in which $Q^1$, $R^1$, $R^3$ and $R^4$ are as defined above, which process comprises reacting a corresponding squaric acid monoester monoamide with a compound of formula $Q^1$=$CHR^1$. This process may hereinafter be referred to as the "fifth process of the invention".

This invention also provides a process for the preparation of a 1,3-disubstituted-2-amino or substituted amino squarylium dye, which process comprises reacting the corresponding 1,3-disubstituted-2-unsubstituted squarylium dye with an alkylating agent to produce a corresponding 1,3-disubstituted-2-alkoxy squarylium compound, and thereafter reacting the 2-alkoxy compound with ammonia or a primary or secondary amine to produce the 1,3-disubstituted-2-amino or substituted amino squarylium dye. This process may hereinafter be referred to as the "sixth process of the invention".

This invention also provides a process for generating heat in a medium comprising a dye of Formula I, which process comprises exposing at least part of the medium to infra-red actinic radiation of a frequency absorbed by the dye, whereby the radiation is absorbed by the dye and heat is generated within the parts of the medium exposed to the radiation.

Finally, this invention provides a thermal imaging medium comprising at least one imaging layer, the imaging layer comprising a color-forming compound which undergoes a change of color upon heating above a color-forming temperature for a color-forming time, the imaging layer further comprising a dye of Formula I.

It will be noted that the symbol $Q^1$ has been used for both a divalent grouping in Formulae I, II, III and IV, and a monovalent grouping in the formula $Q^1CH_2R^1$. This apparent anomaly is due to the fact that the bond orders in the compounds of Formula I, II, III and IV are not integral. For example, the dye A shown in Figure 2 of the accompanying drawings is actually a resonance hybrid of the form shown and the similar form in which the positive charge resides on the oxygen atom of the other benzpyrylium nucleus (with contributions from other resonance forms). Thus, whether $Q^1$ is drawn as divalent or monovalent depends solely upon which of the contributing resonance forms is drawn, and similarly for $Q^2$. On the other hand, the compounds of formula $Q^1CH_2R^1$, such as the salt B shown in Figure 1, are not resonance hybrids to any significant extent, and thus in this formula $Q^1$ is correctly shown as monovalent. The $Q^1/Q^2$ nomenclature employed will thus be clear to skilled chemists. Similar considerations apply to $Q^3$ and $Q^4$.

When the group Ω contains one or more hydrogen atoms directly bonded to nitrogen or carbon, these hydrogen atoms are of course susceptible to being removed by bases, and the compounds may thus be encountered in deprotonated forms depending upon the pH of the medium containing the compound. Although the compounds of Formulae I and II are normally shown herein in their protonated forms, the invention extends to the deprotonated forms of these compounds. In particular, the 2-N sulfonamide compounds of Formula I, and the compounds in which Ω is a $-CH(CN)_2$ group, are so readily deprotonated that they will often be found in their deprotonated form under neutral conditions. The discussion in the following four paragraphs assumes that the compounds of Formula I are present in their protonated form, but the consequences of deprotonation of the compound will readily be apparent to skilled chemists.

The compounds of Formula I produced by the processes of the present invention may be cationic, anionic or non-ionic. When none of the groups $Q^1$, $Q^2$ and Ω carries any charged substituents, the $Q^1Q^2$-squarate moiety (hereinafter referred to simply as the "dye moiety") bears a single positive charge, and hence the dye is cationic. However, any one or more of $Q^1$, $Q^2$ and Ω may carry a negatively or positively charged group (for example a $-COO^-$ or trialkylammonium substituent), and if one or more negatively charged substituents is present, the dye will be non-ionic or anionic respectively.

When a counterion is present in a cationic or anionic dye of the present invention, the counterion may be any counterion which is not incompatible with the dye moiety and which thus provides a stable salt. The choice of counterion may be important in ensuring the solubility of the dye in various media, and reducing or preventing aggregation of the dye; minimizing aggregation of the dye is highly desirable since such aggregation can significantly reduce the apparent extinction coefficient of the dye in polymeric media.

Similarly, if the chromophoric group $Q^1$ or $Q^2$ does not carry any charged substituents (such nuclei being generally preferred in the present processes), the "compounds" $Q^1CH_2R^1$ and $Q^2CH_2R^2$ used in the present processes are actually cations. The counterion present may be any counterion which provides a stable salt and which does not interfere with the relevant reactions. Typically, large fluorinated anions, such as trifluoromethane sulfonate and tetrafluoroborate have been found to give good results in the present processes. The groups $Q^1$ and $Q^2$ may, however, bear charged substituents and thus in some cases $Q^1CH_2R^1$ and $Q^2CH_2R^2$ may be neutral compounds which do not require the presence of a counterion.

It may often be found convenient, for synthetic reasons, to prepare a desired moiety with one counterion and thereafter to effect a counterion exchange to form a different salt of the same moiety. Methods for such counterion ion exchange are well known to those skilled in the art.

Figure 1 of the accompanying drawings shows a synthetic scheme for the preparation of a starting material of Formula III used in the present invention by reactions described in the aforementioned European Application No. 92107574.3;

Figure 2 shows the conversion of a starting material of Formula III to a squaric acid derivative of Formula II and thence to a squarylium compound of Formula I by the first and second processes of the present invention;

Figure 3 shows the synthesis of a squarylium compound of Formula I from a corresponding squaric acid monoester monoamide by the fourth process of the present invention;

Figure 4 shows the preparation of an *exo*-alkylene compound of the present invention by the fifth process of the present invention, and the condensation of this *exo*-alkylene compound with a squaric acid monoester monoamide to produce the same type of intermediate as shown in Figures 2 and 3;

Figure 5 shows the conversion of a 2-unsubstituted squarylium dye to a corresponding 2-substituted aminosquarylium dye by the sixth process of the present invention;

Figure 6 shows the synthesis of a 2-C compound of Formula I by a modified form of the fourth process of the invention; and

Figure 7 shows a schematic cross-section through a preferred imaging medium of the present invention incorporating infrared dyes of the present invention.

The interrelationships among the various reactions of the present invention may best be seen from the accompanying drawings. Figures 1 and 2 show a synthetic scheme for the preparation of a 2-N squarylium compound (hereinafter referred to as "Dye A") of Formula I, in which $Q^1$ is (in the resonance hybrid drawn) a 7-diethylamino-2-(1,1-dimethylethyl)-(benz[b]-4H-pyran-4-ylidene) grouping and $Q^2$ is a 4-[7-diethylamino-2-(1,1-dimethylethyl)-benz[b]pyrylium] grouping. The reactions shown in Figure 1 are described in the European Application No. 92107574.3, while the reactions shown in Figure 2 are examples of the first and second processes of the present invention. Accordingly, the reactions shown in Figure 1 will only be briefly described herein, and for fuller details the reader is referred to the copending European Application.

One form of the synthesis begins with the condensation of a 2,6-bis-(1,1-dimethylethyl)-4-($R^1$-methyl)-7-diethylaminobenzpyrylium salt B (a compound of Formula $Q^1CH_2R^1$) with 2,3,4,4-tetrachlorocyclobut-1-en-2-one C to give the trihalosquaric acid derivative D. The tetrachloro compound C and its synthesis are described in Maahs et al., "Syntheses and Derivatives of Squaric Acid", Angew. Chem. Int. Ed., 5, 888-893 (1966). This reaction is conducted in the presence of a base, preferably triethylamine. As noted above, the anion of the salt B can be any anion which provides a stable salt and which does not interfere with the desired reaction; conveniently the tetrafluoroborate salt is used.

As may be seen from Figures 1 and 2, use of the 4-methylbenzpyrylium salt B ($R^1$ is a hydrogen atom) will produce a dye in which $R^1$ is hydrogen. If the 4-methyl group of the salt B is replaced with a different group of the formula -$CH_2R^1$, the corresponding dyes can be produced in which $R^1$ is an aliphatic or cycloaliphatic group; thus, for example, the use of a 4-ethyl salt gives a final dye in which $R^1$ is methyl. Similar variations in the group $R^2$ may be produced by varying the 4-substituent in the benzpyrylium salt of Formula M (described below). The tetrabromo homologue may be used in place of the tetrachloro compound C.

In the next step of the synthesis, the trihalosquaric acid derivative D is hydrolyzed to the corresponding non-halogenated derivative E. Desirably, this hydrolysis is effected by heating the derivative D with triflic acid, then adding water.

Alternatively, the non-halogenated derivative E may be prepared by condensing the salt B with the diacid chloride (F), an ester/acid chloride (G) or a diester (H) of squaric acid (the butyl ester/acid chloride and diester are shown in Figure 1), followed by hydrolysis of the resultant product. With both the monoacid chloride/monoester G and the diester H, this reaction requires the presence of a base to produce useful yields; with the more reactive diacid chloride F, this reaction can be conducted without base. The reaction of the diacid chloride F may also be promoted by a Lewis acid.

When the diacid chloride F is used as starting material in this reaction, the intermediate is J, the acid chloride of E, whereas when the diester H is used as starting material, the intermediate is K, the ester of E. When the ester/acid chloride G is used, both J and K are produced, but the production of this mixture poses no problems, since both compounds are readily hydrolyzed to give the derivative E. If desired, the acid chloride J may be treated with methanol to convert it to the ester K. Acid bromides may be used in place of the acid chlorides, and the group $R^1$ may be varied by changing the 4-substituent on the salt B, as described above.

In Figure 2 there is shown the first process of the invention, namely the reaction of the ester K with a compound of formula HΩ (in this case a nitrogen compound of formula $HNR^3R^4$) to produce the corresponding 2-Ω (in this case, 2-$NR^3R^4$) squaric acid derivative L. Although the ester K has been shown in Figure 2, this reaction may also be conducted using the acid chloride J shown in Figure 1.

The final step of the synthesis of the squarylium dye A is an example of the second process of the present invention, namely the condensation of the squaric acid derivative L with one mole of the appropriate compound of formula $Q^2CH_2R^2$; the compound in which $Q^2$ is a 2-(1,1-dimethylethyl)-7-diethylaminobenzpyrylium group is shown in Figure 2. The conditions required for this reaction may be substantially the same as those used for the prior art reactions in which two moles of a benzpyrylium salt are condensed with squaric acid to form a symmetric bisbenzpyrylium dye. Thus, this reaction is assisted by base, conveniently a tertiary amine, for example quinoline. The reaction is desirably conducted in solution in an alcohol, conveniently n-butanol. Alternatively, this reaction may be promoted by a Lewis acid, for example a titanium tetrahalide, conveniently in dichloromethane.

Although the reaction

$$L \rightarrow A$$

illustrated in Figure 2 produces a dye A in which $Q^1$ is the same as $Q^2$, it will readily be apparent that this need not be the case, since the group $Q^1$ derived from compound B (Figure 1) could be different from the group $Q^2$ derived from compound M. Thus, the synthesis shown in Figures 1 and 2 can be used to produce both symmetric dyes, in which $Q^1$ and $Q^2$ are the same, and asymmetric dyes in which these two groups are different.

The synthesis shown in Figure 2 may be modified to produce a 2-C EW-dye of Formula I. In this modified synthesis, the $CR^5R^6R^7$ group is introduced into the ester K using a carbanion $^-CR^5R^6R^7$ (i.e., using the compound $HCR^5R^6R^7$ in the presence of a base) to produce the corresponding substituted squaric acid derivative L. Again, the reaction may also be conducted using the acid chloride J shown in Figure 1 instead of the ester K.

In many cases, the synthesis of the final dye is most simply achieved by introducing the final Ω radical during the preparation of the compound L; for example, when Ω is an amino, alkylamino or dialkylamino group, so that the compound HΩ is ammonia or a primary or secondary amine, the reaction

$$K \rightarrow L$$

proceeds well with ammonia or a primary or secondary amine so that there is no difficulty in incorporating the final Ω radical in this step of the synthesis. However, in other cases, the final Ω radical may be such as to interfere with the reaction

$$L \rightarrow A.$$

In such circumstances it is preferred to use a different compound HΩ in the reaction

$$K \rightarrow L,$$

and then to modify Ω in the final dye A. For example, if Ω is to be an acylamino group, it is convenient to carry out the reaction

$$K \rightarrow L$$

with ammonia, thereby producing an intermediate L and a dye A in which both Ω is $NH_2$, and then to react this dye A with the appropriate acyl chloride (or other acyl halide) to attach the desired acyl group to the amino substituent.

Furthermore, as discussed in more detail below, Ω may contain various functional groups, and some of these functional groups may be capable of interfering with one or both of the reactions

$$K \rightarrow L$$

and

$$L \rightarrow A.$$

For example, the reaction

$$L \rightarrow A$$

depends upon the presence of active hydrogens in the compound $Q^2CH_2R^2$, and any active hydrogens in Ω may interfere with this reaction. In such cases, it may be necessary to modify the synthesis shown in Figure 2 either by modifying Ω in the dye A (in the same manner as discussed above for the case where Ω is an acylamino group), or by blocking the functional groups in the compound HΩ and then unblocking these groups in the dye A.

Figure 3 shows an alternative synthesis of a 2-N dye N starting from a diester P of squaric acid (the diethyl ester is illustrated in Figure 3) and using the fourth reaction of the present invention. The diester P is first condensed with a compound of formula HΩ (in this case $HNR^3R^4$) to introduce the Ω (amino) group, thereby producing the corresponding squaric acid monoester monoamide Q, which is then condensed with two moles of a compound R of formula $Q^1CH_2R^1$ (the salt in which $Q^1$ is a 2,6-bis(1,1-dimethylethyl)thiopyrylium group is illustrated in Figure 3) to produce the final symmetrical dye N. The reaction

$$Q \rightarrow N$$

may be carried out under the same conditions as the reaction

$$L \rightarrow A$$

described above with reference to Figure 2. Also, the synthesis shown in Figure 3 may be modified to include changes in Ω in the dye N, and the blocking of functional groups, as discussed above with reference to Figure 2.

At present, the synthetic route shown in Figure 2 is preferred over that shown in Figure 3 because the former tends to give better yields of dye and because the former is capable of producing asymmetric dyes is good yield. (Although theoretically the synthesis shown in Figure 3 might be modified to produce asymmetric dyes by treating the monoester monoamide Q with a mixture of two different compounds $Q^1CH_2R^1$ and $Q^2CH_2R^2$, this approach is not recommended, since the separation of the resultant mixture of three dyes (two symmetric dyes and the desired asymmetric dye) is likely to be very difficult.)

Figure 4 illustrates the preparation of an intermediate U, which is analogous to the intermediate L shown in Figure 2, by a synthetic route which exemplifies the fifth process of the present invention and proceeds via an *exo*-alkylene compound. The synthesis shown in Figure 4 begins from a chromone S; this chromone can be prepared by a variety of processes known to persons skilled in the art of organic synthesis. Certain methods for the synthesis of such chromones are exemplified below, and other are described in the aforementioned International and European Applications. The chromone S is first converted to the corresponding *exo*-methylene compound T by means of a Peterson olefination, as described in, for example, Organic Reactions, Volume 38, 1-225 (Wiley, New York, 1990); alternatively the *exo*-methylene compound may be produced by treating the corresponding compound of formula $Q^1CH_2R^1$ with a strong base. The *exo*-methylene compound T is then reacted with a squaric acid monoester monoamide to produce the intermediate S; this reaction is somewhat analogous to the reaction

$$Q + R \rightarrow N$$

shown in Figure 3, but only monocondensation occurs, so that the intermediate S is analogous to the intermediate L shown in Figure 2. The conversion of T to U is desirably catalyzed by a Lewis acid, preferably an aluminum halide such as aluminum chloride. It will readily be apparent that the intermediate L can readily be converted to a dye of the present invention by a reaction analogous to

$$L + M \rightarrow A$$

shown in Figure 2.

In at least some cases, the 2-N dyes of Formula I cannot be synthesized by reacting the corresponding dye having an unsubstituted squarylium nucleus with an amino compound, since in these cases the amino compound simply adds reversibly to the squarylium dye. However, unsubstituted squarylium dyes can be converted to 2-N dyes of the present invention by the indirect route shown in Figure 5, which illustrates the sixth process of the present invention. In this indirect route, an unsubstituted squarylium dye V is first treated with an alkylating agent, preferably a dialkyl sulfate, to produce the corresponding 3-alkoxysquarylium compound W, and this 3-alkoxy compound is then treated with a compound of formula $HNR^3R^4$ (typically ammonia or a primary or secondary amine; butylamine is shown for purposes of illustration in Figure 5) to produce the final aminosquarylium dye.

The syntheses shown in Figures 2-5 are primarily intended for preparing non-sulfonamide dyes of the present invention. The 2-N sulfonamide dyes of the present invention are conveniently synthesized by treating the corresponding squarylium dye with the appropriate organosulfonyl isocyanate, with elimination of carbon dioxide; for example tosyl isocyanate ($p\text{-}CH_3\text{-}C_6H_4\text{-}SO_2NCO$) converts a squarylium dye to the corresponding p-tolylsulfonamido squarylium dye. This reaction proceeds readily in solution in a non-polar solvent, for example toluene, no catalyst being required. The reaction is not confined to aromatic sulfonyl isocyanates and can be carried out with alkyl sulfonyl isocyanates, for example butylsulfonyl isocyanate.

Figure 6 shows an modified form of the synthesis shown in Figure 3 and intended for the synthesis of 2-C non-EW dyes of the present invention. The synthesis shown in Figure 6 is of a dye N' starting from a diester P' of squaric acid (the dibutyl ester is illustrated in Figure 6). The diester P' is first condensed with a compound containing a negatively charged species $CR^5R^6R^7$; this compound is normally an organometallic compound, and preferably an organolithium compound. The reaction adds the $-CR^5R^6R^7$ group to one of the oxo groups of the diester P' to produce the squaric acid derivative Q'; to avoid disubstitution into both oxo groups, not more than the stoichiometric amount of the organometallic reagent should be used.

After being separated from unreacted starting material P' and other by-products, the squaric acid derivative Q' is treated with an acid, for example hydrochloric acid to convert it to the squaric acid derivative R'. Although it is possible to simply add acid to the reaction mixture resulting from the treatment of the diester P' with the organometallic reagent, this course is not recommended, since the squaric acid derivative R' produced may be contaminated with unreacted ester P', and P' and R' are so similar that it is extremely difficult to separate them, even by chromatography.

In one version of the synthesis shown in Figure 6, the squaric acid derivative R' is then condensed with two moles of a compound S' of formula $Q^1CH_2R^1$ (the salt in which $Q^1$ is a 2,6-bis(1,1-dimethylethyl)thiopyrylium group is illustrated in Figure 6) to produce the final symmetrical dye N'. The reaction

$$R' \rightarrow N'$$

may be carried out under the same conditions as the reaction

$$L \rightarrow A$$

described above with reference to Figure 2. Also, the synthesis shown in Figure 6 may be modified to include changes in the group $R^5$ and/or $R^6$ in the dye N', and the blocking of functional groups, as discussed above with reference to Figure 2.

Alternatively, the squaric acid derivative R' may be condensed with only one mole of the salt S' of formula $Q^1CH_2R^1$ to produce a monocondensed intermediate T'; the conditions required for this reaction are substantially the same as those for the reaction

$$B + H \rightarrow K$$

described above with reference to Figure 1. This intermediate T' may then be condensed with a second mole of the appropriate salt S' to produce the final dye N', using the same conditions as for the reaction

$$R \rightarrow N.$$

Obviously, although Figure 6 shows the same salt being condensed with both R' and T,' two different salts could be used in the two reactions, thereby permitting synthesis of asymmetric dyes in which $Q^1$ and $Q^2$ are different.

The syntheses shown in Figure 6 may provide a more unambiguous synthesis of 2-C non-EW dyes of the invention than that shown in Figure 2.

As already indicated, a wide range of groups $R^3$, $R^4$, $R^5$, $R^6$ and $R^7$ can be present in the squarylium dyes of the present invention. Thus, for example, these groups can each independently be a hydrogen atom or an acyl, aliphatic, cycloaliphatic, aromatic or heterocyclic group, subject to the aforementioned provisoes. For examples these groups can each be an alkyl, alkenyl, alkynyl, cycloalkyl (for example, a cyclohexyl group) or cycloalkenyl group, a polycyclic saturated group such as a decalinyl or adamantyl group, or a similar group containing ethylenic unsaturation (for example a 6,6-dimethylbicyclo[3.1.1.]hept-2-en-2-yl or bicyclo[2.2.1]hept-2-en-5-yl group) an aromatic group (for example, a phenyl group), or a saturated or unsaturated heterocyclic group (for example, a piperidino group). In addition, these groups, together with the intervening nitrogen or carbon atom, may form a heterocyclic ring; thus, for example, the $NR^3R^4$ grouping could be a piperidino or indolino group, and may contain additional hetero atoms, as for example in a morpholino or pyrimidino group. One of $R^3$ and $R^4$ can also be an amino or substituted (for example, alkyl or cycloalkyl substituted) amino group, so that the $NR^3R^4$ grouping may be a substituted or unsubstituted hydrazine grouping, for example a trimethylhydrazine grouping.

Any of these groups may be unsubstituted or substituted; as indicated above, it is one of the advantages of the dyes of the present invention that the 2-substituent on the squarylium ring provides a convenient site to which a variety of groups may be attached in order to modify the properties of the dye, without having to change the chromophoric groups $Q^1$ and $Q^2$. Thus, the group $\Omega$ may contain substituents which affect the solubility of the dye in various media. For example, if it desired to increase the solubility of the dye in highly polar solvents, $\Omega$ may contain sulfonic acid, carboxyl or quaternary ammonium groups. On the other hand, if it desired to increase the solubility of the dye in non-polar solvents, $\Omega$ may be unsubstituted long-chain alkyl groups. The group $\Omega$ may also contain substituents such as halo, cyano, amino, oxo and phenyl groups, ether, amide and urethane linkages.

The group $\Omega$ may also contain groups which permit linking the dye to other materials, thereby permitting, for example, the dye to be incorporated into a polymer.

The nature of $\Omega$ also affects the spectrum of the squarylium dye. In general, the wavelength ($\lambda_{max}$) of maximum absorption of the dye in the near infra-red becomes longer as $\Omega$ becomes more electron donating. For example, in the 2-N dyes A shown in Figure 2, the variation of $\lambda_{max}$ with changes in $\Omega$ is as follows:

| $\Omega$ | $\lambda_{max}$, nm |
|---|---|
| $NH_2$ | 797 |
| $NHCH_2CH_2SO_3^-$ | 810 |
| $N(C_2H_5)_2$ | 828 |

The corresponding unsubstituted squarylium dye, in which $\Omega$ is replaced by $O^-$, has $\lambda_{max}$ = 808 nm. Thus, in this series of dyes, modification of the unsubstituted squarylium dye by incorporation of these amino groups permits modification ("fine tuning") of $\lambda_{max}$ over a range of -11 to +20 nm. Greater wavelength shifts can be achieved using the 2-C dyes of the present invention; for example, the analogue of dye A shown in Figure 2 in which $\Omega$ is an ethyl group has $\lambda_{max}$ = 752 nm, while the corresponding dye in which $\Omega$ is a dicyanomethyl group has $\lambda_{max}$ = 850 nm, in its deprotonated form.

The value of $\lambda_{max}$ is also affected by deprotonation of the dye. As mentioned above, if $\Omega$ contains an acidic hydrogen atom, this hydrogen atom may be removed by bases. As shown in Example 61 below, such deprotonation of the dye typically shifts $\lambda_{max}$ about 60 nm longer. Thus, in cases where it is desired to provide absorption at longer wavelengths, it may be advantageous to incorporate a dye of the present invention in a basic medium, either by using a basic binder in such a medium or by providing a separate base in the medium, such that the dye exists in the medium in its deprotonated form.

Although the invention has been shown in the accompanying drawings and described above with reference to compounds in which $Q^1$ and $Q^2$ are each a pyrylium or benzpyrylium nucleus, it will be apparent that both $Q^1$ and $Q^2$ can each independently be any chromophoric group such that in the compounds of formulae $Q^1CH_2R^1$ and $Q^2CH_2R^2$ the methylene hydrogens are active hydrogens, so that these methylene hydrogen atoms can undergo the condensations with squaric acid derivatives already described. It is preferred that the atoms of $Q^1$ and $Q^2$ which are bonded directly to the $CR^1$ and $CR^2$ groupings respectively each be part of an aromatic ring. For example, $Q^1$ and $Q^2$ may each independently be an imidazole, benzimidazole, thiazole, benzthiazole, oxazole, benzoxazole, 2- or 4-pyridinium, 2- or 4-quinolinium or indolinium nucleus. Desirably, at least one, and preferably both, of $Q^1$ and $Q^2$ is a non-nitrogenous heterocyclic nucleus, especially preferred nuclei being pyrylium, thiopyrylium, selenopyrylium, benzpyrylium, benzthiopyrylium and benzselenopyrylium nuclei. Such nuclei be either the 2- or 4-isomers, although the latter are preferred. $Q^3$ and $Q^4$ can be any of the groups already mentioned for $Q^1$ and $Q^2$.

In one preferred group of dyes of Formula I, $Q^1$ and/or $Q^2$ is a 2,6-dialkylpyrylium, -thiopyrylium or -selenopyrylium nucleus, in which each of the alkyl groups contains not more than about 8 carbon atoms, especially those in which $Q^1$ and/or $Q^2$ is a 2,6-di-tertiary butylpyrylium, -thiopyrylium or -selenopyrylium nucleus. The presence of these nuclei in the dyes has been found to provide good solubility in polymeric media and high extinction coefficients.

Another preferred group of dyes of Formula I are those in which $Q^1$ and/or $Q^2$ is a 4-benzpyrylium, 4-benzthiopyrylium or 4-benzselenopyrylium nucleus, desirably such a nucleus which carries at its 2-position a substituent in which a non-aromatic carbon atom is bonded directly to the benzpyrylium nucleus, subject to the proviso that if this 2-substituent contains an aromatic nucleus, this aromatic nucleus is not conjugated with the benzpyrylium nucleus. The 2-substituent may be, for example:

a. an alkyl group, for example an isopropyl, sec-butyl, tert-butyl, 2-ethyl-2-methylbutyl or 2,2-dimethylbutyl group;
b. an alkenyl group, for example a vinyl group;
c. an alkynyl group, for example an ethine group;
d. a cycloalkyl group, for example a cyclohexyl group;
e. a cycloalkenyl group, for example a cyclohexenyl group;
f. a polycyclic saturated hydrocarbon group, for example a decalinyl or adamantyl group;
g. a polycyclic, ethylenically unsaturated hydrocarbon group, for example a 6,6-dimethylbicyclo[3.1.1]hept-2-en-2-yl or bicyclo[2.2.1]hept-2-en-5-yl group;
h. any of the foregoing substituents substituted with aryl, halo, cyano, amino or oxo groups, or containing ether, amine or urethane linkages. The 2-substituent is desirably one in which the carbon atom which is directly attached to the benzpyrylium nucleus carries not more than one hydrogen atom.

The benzpyrylium nucleus may also carry at its 7-position a substituent in which an element of Group 5A, 6A or 7A of the Periodic Table is directly connected to the benzpyrylium nucleus, subject to the proviso that when this element of Group 5A or 6A, the 7-substituent may be at least one saturated ring containing this element of Group 5A or 6A, this saturated ring optionally being fused to the phenyl ring of the associated benzpyrylium nucleus. Preferred 7-substituents are alkoxy groups containing not more than about 12 carbon atoms, or disubstituted amino or disubstituted phosphino groups, wherein each of the substituents on the or each disubstituted group comprises an alkyl group containing not more than about 6 carbon atoms, or the two substituents on any one disubstituted group together form, with the nitrogen or phosphorus atom thereof, a heterocyclic ring system, this ring system optionally being fused to the benzpyrylium nucleus which carries the disubstituted amino or phosphino substituent. Examples of suitable 7-substituents include dialkylamino wherein each of the alkyl groups contains not more than about 4 carbon atoms, piperidino, indolinyl, morpholino and $-N[-(CH_2)_3-]_2$ groups, subject to the proviso that when one or both of the amino groups is a $-N[-(CH_2)_3-]_2$ group, the ends of the trimethylene groups remote from the nitrogen atom are joined to the 6- and 8-positions of the benzpyrylium nucleus carrying the nitrogen atom, so that the $-N[-(CH_2)_3-]_2$ group and the benzene ring of the benzpyrylium nucleus together form a julolidine ring system. As described in the aforementioned International and European Applications, dyes containing such 4-benzpyrylium nuclei have desirable properties, including solubility in polymeric media and high extinction coefficients.

Alternatively, the or each benzpyrylium nucleus may carry at its 6-position an alkoxy, alkenyloxy or alicyclyloxy group; the alkyl, cycloalkyl or alicyclic portion of this group may be any of the groups (other than alkynyl) mentioned above as the 2-substituent. Desirably the 6-substituent is an alkoxy or cycloalkoxy group, preferably a branched chain alkoxy group. Specific preferred branched chain alkoxy groups are propoxy, but-2-oxy and 2-ethylbutoxy groups. Preferably, the benzene ring of the benzpyrylium nucleus bears no substituents other than the 6-substituent. It has been found that providing such a branched-chain 6-substituent increases the solubility of the dye in polymeric media. In addition, such 6-substituted dyes tend to have relatively low visible extinction and are thus advantageous for use in applications (such as thermal imaging media) where visible absorption is undesirable. It is believed (although the invention is in no way limited by this belief) that the advantageous solubility properties of the aforementioned 6-substituted dyes are due at least in part to the 6-substituent extending out of the plane of the aromatic nucleus to which it is attached, and thus the choice of a 6-substituent may be influenced by the stereochemistry of the substituent, not merely its chemical nature.

Although $R^1$ and $R^2$ may be other groups, for example cycloalkyl groups or any of the other aliphatic and cycloaliphatic groups discussed above as potential 2-substituents on benzpyrylium groups $Q^1$ and $Q^2$, it is preferred that these two groups each independently be a hydrogen atom or an alkyl group containing not more than about 6 carbon atoms.

The dyes produced by the processes of the present invention may be used in any of the applications in which prior art near infra-red absorbers have been used. Thus, the dyes may be used as dyes in printing inks intended to provide markings which can be read under near infra-red radiation, for example, on packages of consumer items intended to be scanned by near infra-red laser scanners. At least some of the present dyes may also be useful as charge transfer materials for use in xerography, electrophotography and similar processes, and as laser dyes.

However, because of their high extinction coefficients in the near infra-red region, the dyes produced by the present processes are especially useful in processes for generating heat in a medium; in such a process at least part of the medium is exposed to near infra-red actinic radiation of a frequency absorbed by the dye, so that the radiation is absorbed by the dye and heat is generated within the parts of the medium exposed to the radiation. Typically, in such a process, the radiation is provided by a laser. The medium may also comprise a thermally sensitive material capable of undergoing a color change upon exposure to heat; the medium is exposed imagewise to the radiation, and the heat generated by the dye is sufficient to effect a color change in the thermally sensitive material, so that an image is formed in the medium. Thus, for example, the present dyes may be used as the near infra-red absorbers in the thermal imaging processes described in U.S. Patents Nos. 4,602,263 and 4,826,976, and in the aforementioned International and European Applications. These imaging processes rely upon the irreversible unimolecular fragmentation of one or more thermally unstable carbamate moieties of an organic compound to effect a visually discernible color shift from colorless to colored, from colored to colorless or from one color to another.

In such a process, preferably the thermally sensitive material is originally substantially colorless and is converted by the heat generated to a colored material in exposed areas of the image. Multi-colored images may be produced using a heat-sensitive element containing an imaging layer of colorless imaging compound (leuco dye) for forming a yellow image, an imaging layer of colorless imaging compound for forming a cyan image, and an imaging layer of colorless imaging compound for forming a magenta image. Preferred leuco dyes, and processes for their preparation, are described in U.S. Patent No. 4,663,518.

In the production of such multi-color images, each imaging layer contains, in addition to the leuco dye, an infra-red absorber selected such that the three infra-red absorbers absorb radiation at different predetermined wavelengths above 700 nm sufficiently separated so that each imaging layer may be exposed separately and independently of the others by using infra-red radiation at the particular wavelengths selectively absorbed by the respective infra-red absorbers. As an illustration, the yellow, magenta and cyan precursors may have infrared absorbers associated therewith that absorb radiation at (say) 760 nm, 820 nm and 880 nm, respectively, and may be addressed by laser sources, for example, infra-red laser diodes emitting radiation at these respective wavelengths so that the three imaging layers can be exposed independently of one another. While each layer may be exposed in a separate scan, it is usually preferred to expose all of the imaging layers simultaneously in a single scan using multiple laser sources of the appropriate wavelengths. Instead of using superimposed imaging layers, the heat-sensitive compounds and associated infra-red absorbers may be arranged in an array of side-by-side dots or stripes in a single recording layer.

The dyes of the present invention may also be used in the acid-generating imaging process described in copending Application Serial No. 07/965,161 of Stephen J. Telfer, filed October 23, 1992.

A preferred thermal imaging medium of this invention will now be described, though by way of illustration only, with reference to Figure 7 of the accompanying drawings, which is a schematic cross-section through the imaging medium. The thicknesses of the various layers shown in the drawing are not to scale.

The imaging medium (generally designated 10) shown in Figure 7 is intended for use in the production of transparencies and comprises a substantially transparent support 12 formed of 4 mil (101 μm) poly(ethylene terephthalate) (PET) film incorporating an ultra-violet absorber. Appropriate PET films are readily available commercially, for example as P4C1A film from DuPont de Nemours., Wilmington, Delaware, United States of America.

The imaging medium 10 also comprises a diffusion-reducing subcoat 14 approximately 1 μm thick formed from a 10:1 w/w mixture of a water-dispersible styrene acrylic polymer (Joncryl 538 sold by S.C. Johnson & Son, Inc., Racine WI 53403, United States of America) and a water-soluble acrylic polymer (Carboset 526 sold by The B.F. Goodrich Co., Akron Ohio 44313, United States of America). The presence of the minor proportion of water-soluble acrylic polymer reduces the tendency for the layer 14 to crack during the coating process. The diffusion-reducing subcoat 14, which has a glass transition temperature of approximately 55°C, serves the function of a conventional subcoat, namely increasing the adhesion of the imaging layer 16 (described in detail below) to the support 12. The subcoat 14 also serves to reduce

or eliminate migration of dye compound from the imaging layer 16 after imaging; if a conventional subcoat were employed in place of the diffusion-reducing subcoat 14, diffusion of the dye compound from the layer 16 into the subcoat after imaging might cause loss of sharpness of the image. The subcoat 14 is coated onto the support 12 from an aqueous medium containing the water-dispersible and water-soluble polymers.

A yellow imaging layer 16 is in contact with the diffusion-reducing subcoat 14. This imaging layer 16 is approximately 5 µm thick and comprises approximately 47.5 parts by weight of a leuco dye of the formula:

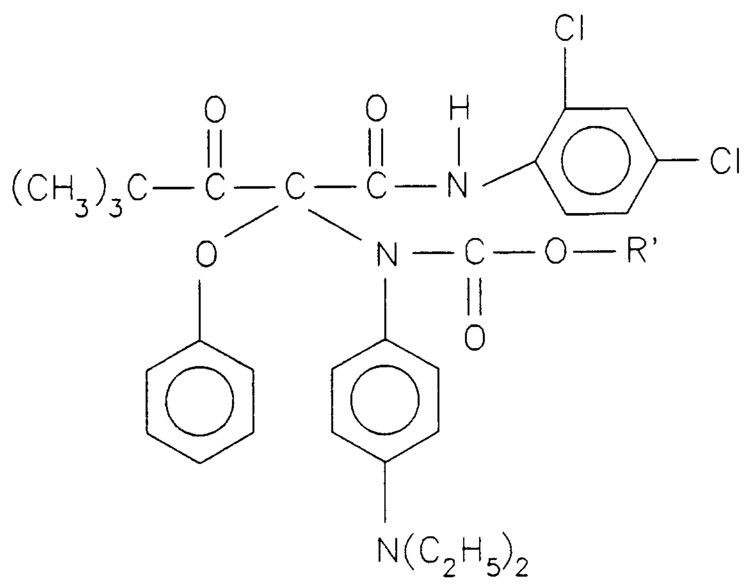

(LD1)

in which R' is a tertiary butyl group (the compounds in which R' is an isobutyl or benzyl group may alternatively be used), 1.6 parts by weight of an infra-red dye of the formula:

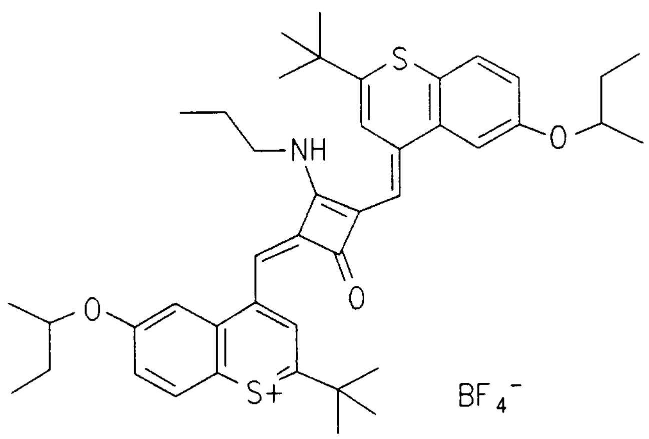

(IR1)

(which may be produced in a manner exactly analogous to the reaction of Example 9 substituting the 6-[2-butoxy] salt for the 6-methoxy salt used in that Example), 3.3 parts by weight of a hindered amine stabilizer (HALS-63, sold by Fairmount Chemical Co.), and 47.5 parts by weight of a poly(methyl methacrylate) binder (Elvacite 2021, sold by DuPont de Nemours, Wilmington, Delaware, United States of America; this material is stated by the manufacturer to be a methyl methacrylate/ethyl acrylate copolymer, but its glass transition temperature approximates that of poly(methyl methacrylate)). This binder has a glass transition temperature of approximately 110°C. The imaging layer 16 is applied by coating from a mixture of heptanes and methyl ethyl ketone.

(Alternatively, the infra-red dye of Formula IR1 above may be replaced by an infra-red dye of the formula:

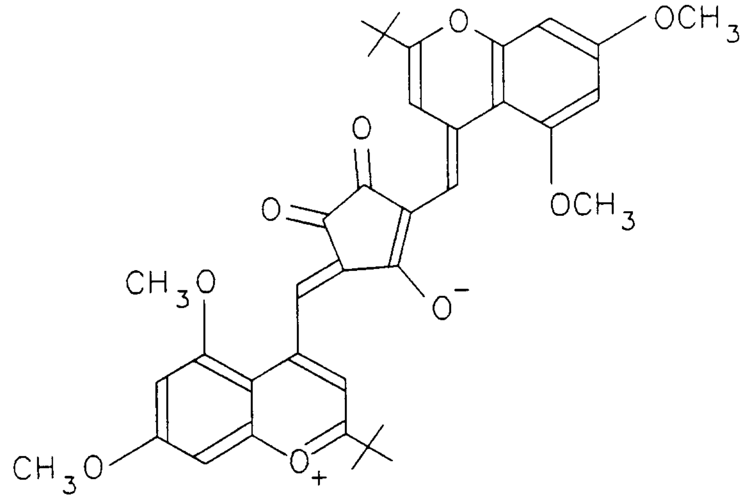

(IR2)

which may be prepared as described in the aforementioned European Application; essentially, this dye is produced by condensing two moles of a 2-(1,1-dimethylethyl)-5,7-dimethoxy-4-methylbenzpyrylium salt with a croconate salt.

Superposed on the yellow imaging layer 16 is a diffusion-reducing layer 18, which, like the first diffusion-reducing layer 14, serves to prevent migration of dye compound from the yellow imaging layer 16 on storage after imaging. The diffusion-reducing layer 18, which is approximately 2 μm thick, is formed of a water-dispersible styrene acrylic polymer (Joncryl 138 sold by S.C. Johnson & Son, Inc., Racine WI 53403, United States of America), and is coated from an aqueous dispersion. This layer has a glass transition temperature of approximately 60°C.

The next layer of the imaging medium 10 is a solvent-resistant interlayer 20 approximately 4.6 μm thick and composed of a major proportion of partially cross-linked polyurethane (NeoRez XR-9637 polyurethane sold by ICI Resins US, Wilmington, Massachusetts, United States of America) and a minor proportion of poly(vinyl alcohol) (Airvol 540, sold by Air Products and Chemicals, Inc., Allentown PA 18195, United States of America). This solvent-resistant interlayer 20 is coated from an aqueous dispersion. The interlayer 20 not only helps to thermally insulate the imaging layers 14 and 22 (described below) from one another during imaging, but also prevents disruption and/or damage to the yellow imaging layer 16 and the diffusion-reducing layer 18 during coating of the magenta imaging layer 22. Since the yellow imaging layer 16 and the magenta imaging layer 22 are both coated from organic solution, if a solvent-resistant interlayer were not provided on the layer 16 before the layer 22 was coated, the organic solvent used to coat the layer 22 might disrupt, damage or extract leuco dye or infra-red absorber from the layer 16. Provision of the solvent-resistant interlayer 20, which is not dissolved by and does not swell in the organic solvent used to coat the layer 22, serves to prevent disruption of or damage to the layer 16 as the layer 22 is coated. Furthermore, the solvent-resistant interlayer 20 serves to prevent the magenta leuco dye, infra-red dye and hindered amine light stabilizer from the layer 22 sinking into the diffusion-reducing layer 18 and the yellow imaging layer 16 as the layer 22 is being coated.

Superposed on the solvent-resistant interlayer 20 is the magenta imaging layer 22, which is approximately 3 μm thick and comprises approximately 47.25 parts by weight of a leuco dye of the formula:

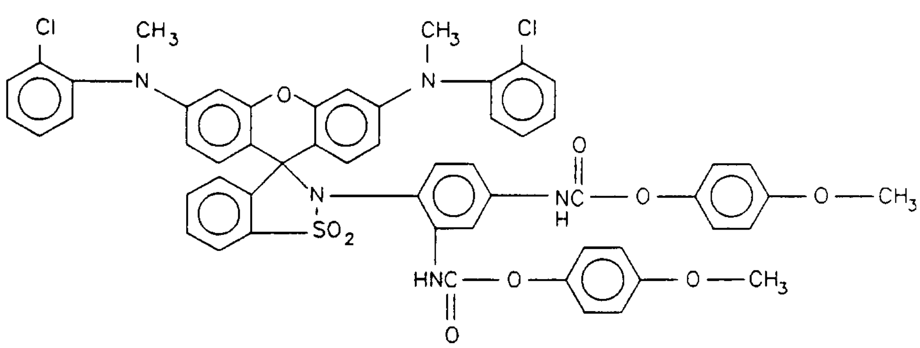

(LD2)

(this leuco dye may be prepared by the methods described in U.S. Patents Nos. 4,720,449 and 4,960,901), approximately 3.4 parts by weight of zinc acetate (thus giving a leuco dye: zinc cation molar ratio of about 1:0.4), 1.62 parts by weight of an infra-red dye of the formula:

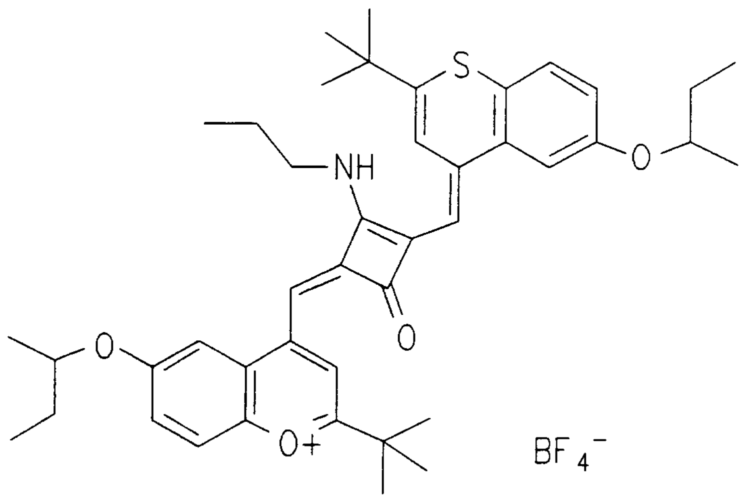

(IR3)

(which may be produced in a manner exactly analogous to the reaction of Example 5 substituting the 6-[2-butoxy] salt for the 6-methoxy salt used in that Example), 3.6 parts by weight of a hindered amine stabilizer (HALS-63), 0.27 parts by weight of a wetting agent, and 47.25 parts by weight of a polyurethane binder (Estane 5715, supplied by The B.F. Goodrich Co., Akron Ohio 44313). The imaging layer 22 is applied by coating from a cyclohexanone/methyl ethyl ketone mixture.

Alternatively, the infra-red dye of Formula IR3 above may be replaced by the dye of formula:

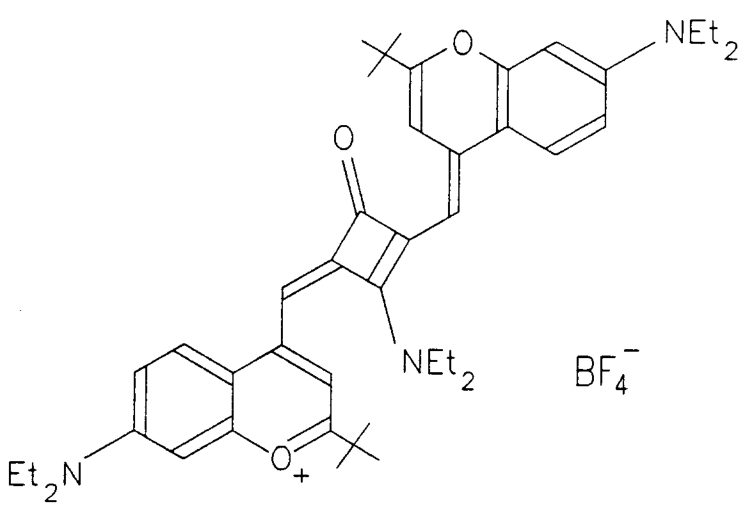

(IR4)

(prepared in a manner similar to that of the dye of Formula IR3 above), or by the dye of formula:

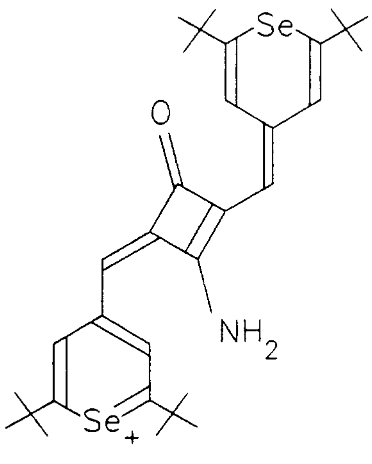

(IR5)

(which can be prepared by the method of Figure 3), or by a dye of formula:

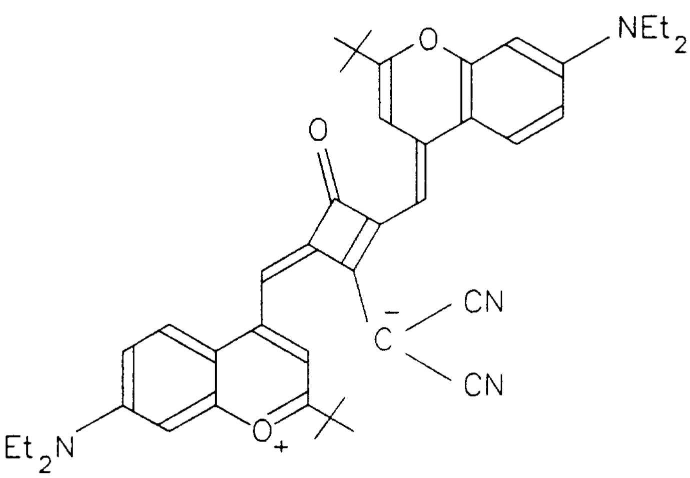

(IR6)

(prepared in Example 11 below).

On the imaging layer 22 is coated a second solvent-resistant interlayer 24 which is formed from the same material, and coated in the same manner as, the solvent-resistant interlayer 20.

Superposed on the second solvent-resistant interlayer 24 is a cyan imaging layer 26, which is approximately 3 μm thick and comprises approximately 49.5 parts by weight of a leuco dye of the formula:

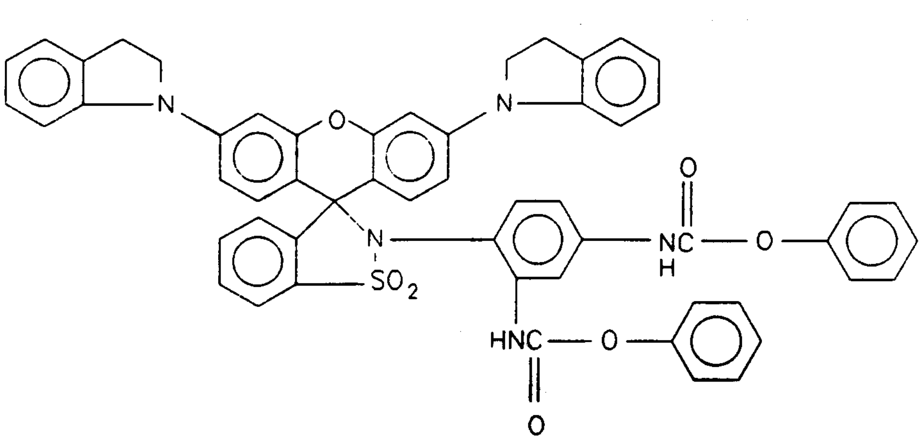

(LD3)

(this leuco dye may be prepared by the methods described in the aforementioned U.S. Patents Nos. 4,720,449 and 4,960,901), approximately 3.97 grams of zinc acetate (thus giving a leuco dye: zinc cation molar ratio of about 1:0.4), 1.62 parts by weight of an infra-red dye of the formula:

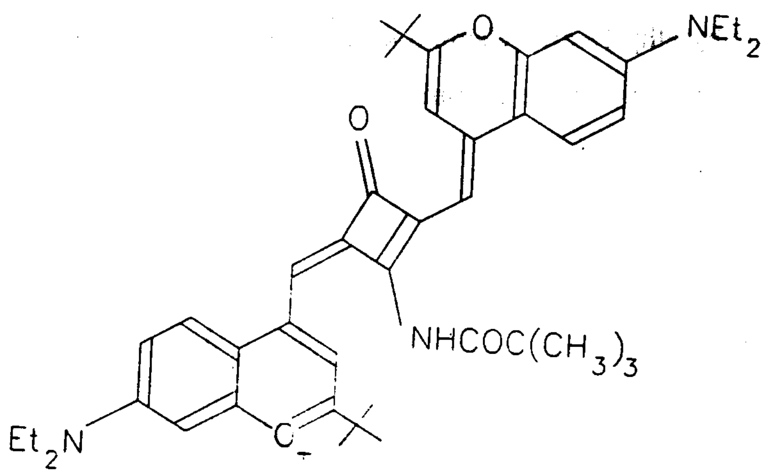

**(IR7)**

(prepared in Example 4 below), 0.2 parts of a wetting agent, and 49.5 parts by weight of a polyurethane binder (Estane 5715). The imaging layer 26 is applied by coating from methyl ethyl ketone.

(Alternatively, the infra-red dye of Formula IR7 above may be replaced by the dye of formula:

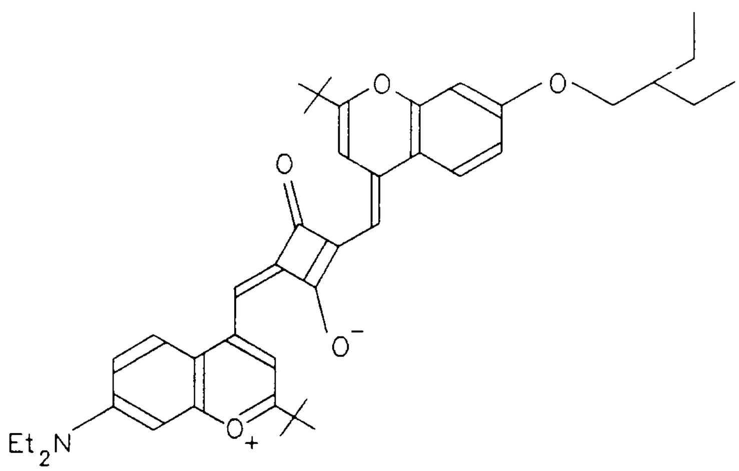

**(IR8)**

which is preferably prepared by the process described in the aforementioned European Application; essentially this process comprises reacting a diester, diacid chloride or monoester monoacid chloride of squaric acid with a 2-(1,1-dimethylethyl)7-diethylamino-4-methylbenzpyrylium salt and hydrolysing to produce a compound of the formula:

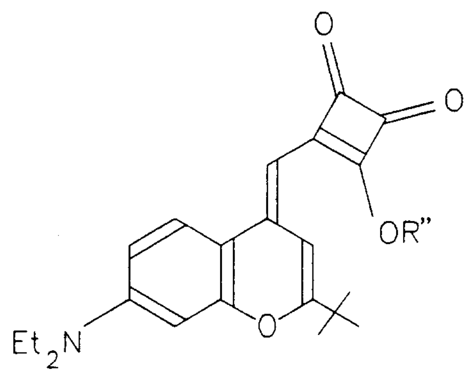

(V)

and then reacting this compound with a 7-alkoxy-2-(1,1-dimethylethyl)-4-methylbenzpyrylium salt to give the final infrared dye of Formula IR8.

As already indicated, the layers 14-26 of the imaging medium 10 are produced by coating on to the transparent support 12. However, the remaining layers of the imaging medium 10, namely the transparent bubble-suppressant layer 32, the ultraviolet filter layer 30 and the adhesive layer 28 are not coated on to the layer 26 but rather are prepared as a separate unit and then laminated to the remaining layers of the medium.

The transparent bubble-suppressant layer 32 is a 1.75 mil (44µm) PET film, a preferred film being that sold as ICI 505 film by ICI Americas, Inc., Wilmington, Delaware. The bubble-suppressant layer 32 prevents the formation of bubbles in the imaging layers 16, 22 and 26 of the imaging medium 10 during imaging.

The ultraviolet filter layer 30 serves to protect the imaging layers 16, 22 and 26 from the effects of ambient ultraviolet radiation. It has been found that the leuco dyes are susceptible to undergoing color changes when exposed to ultraviolet radiation during storage before or after imaging; such color changes are obviously undesirable since they increase the $D_{min}$ of the image and may distort the colors therein. The ultraviolet filter layer 30 is approximately 5 µm thick and comprises approximately 83 percent by weight of a poly(methyl methacrylate) (Elvacite 2043, sold by DuPont de Nemours, Wilmington, Massachusetts), 16.6 percent by weight of an ultraviolet filter (Tinuvin 328 sold by Ciba-Geigy, Ardsdale NY) and 0.4 percent by weight of a wetting agent. The ultraviolet filter layer 30 is prepared by coating on to the bubble-suppressant layer 32 from a solution in methyl ethyl ketone.

The adhesive layer, which is approximately 2 µm thick, is formed of a water-dispersible styrene acrylic polymer (Joncryl 138 sold by S.C. Johnson & Son, Inc., Racine WI 53403) and is coated on to the ultraviolet filter layer 30 from an aqueous dispersion.

After the layers 30 and 28 have been coated on to the bubble-suppressant layer 32, the entire structure containing these three layers is laminated under heat (approximately 225°F, 107°C) and pressure to the structure containing the layers 12-26 to form the complete imaging medium 10.

If desired, the bubble-suppressant layer 32 may be formed by coating, rather than by lamination of a pre-formed film on to the layers 12-26. If the bubble-suppressant layer 32 is to be formed by coating, it is convenient to incorporate an ultra-violet absorber into the bubble-suppressant layer, thereby avoiding the need for a separate ultra-violet absorber layer. Thus, in this case, the layer 28 is coated on to the layer 26 using the solvent already described, and then the bubble-suppressant layer 32 containing the ultra-violet absorber may be coated on to the layer 28 from an aqueous medium.

The medium 10 is imaged by exposing it simultaneously to the beams from three infra-red lasers having wavelengths of approximately 790, 850 and 920 nm. The 920 nm beam images the yellow imaging layer 16, the 850 nm beam images the magenta imaging layer 22 and the 790 nm beam images the cyan imaging layer 26. Thus, a multicolor image is formed in the imaging medium 10, and this multicolor image requires no further development steps. Furthermore, the medium 10 may be handled in normal room lighting prior to exposure, and the apparatus in which the imaging is performed need not be light-tight.

Alternatively, the present dyes may be used in a thermal imaging process in which the medium comprises one layer of a multi-layer structure, this structure further comprising a support layer disposed on one side of the medium and a colored layer adhering to the opposed side of the medium. In this type of thermal imaging process, the heat generated on exposure of the dye to actinic radiation causes increased adhesion of the colored layer to the support layer, such that upon application of a peeling force to the colored layer, the colored layer will peel from the support layer in areas which have not been exposed to the radiation, but in areas which have been exposed to radiation the colored layer will remain attached to the support layer. A preferred thermal imaging process of this type is described and claimed in International Patent Application No. PCT/US87/03249.

From the foregoing description, it will be seen that the present invention provides near infra-red dyes with enhanced compatibility with a variety of media and which can be arranged to have absorptions within narrow wavelength ranges. Furthermore, these dyes can contain a variety of functional groups. The processes of the present invention enable asymmetric infra-red dyes of the invention to be synthesized without the need to separate mixtures of asymmetric and symmetric dyes.

The following Examples are now given, though by way of illustration only, to show details of particularly preferred reagents, conditions and techniques used in the processes of the present invention.

**Examples 1-3 : First process of the invention**

The following Examples 1-3 illustrate the first process of the invention, that is to say reactions analogous to reaction

$$K \rightarrow L,$$

shown in Figure 2, together with certain reactions used for the preparation of the necessary starting materials, and conversion of compounds of Formula II to other compounds of Formula II.

Example 1 : Preparation of 4-[[7-diethylamino-2-(1,1-dimethylethyl)benz[b]-4H-pyran-4-ylidene]methyl]-3-butoxycyclobut-3-en-1,2-dione

This Example illustrates the preparation, by a reaction analogous to

$$B + H \rightarrow K$$

shown in Figure 1, of the squaric acid derivative K in which $R^1$ is a hydrogen atom.

A solution of 7-diethylamino-2-(1,1-dimethylethyl)-4-methylbenzpyrylium tetrafluoroborate (3.57 g, 10 mmol, prepared as described in the aforementioned International Application) in dichloromethane (20 mL) was added dropwise over two hours to a solution of di-n-butyl squarate (2.5 g, 11 mmol, available from Aldrich Chemical Company, Milwaukee, Wisconsin) and triethylamine (2.02 g, 20 mmol) in dichloromethane (30 mL) at room temperature. After the addition had been completed, the reaction mixture was heated under reflux for three hours. The solvent was then removed and diethyl ether (50 mL) was added. The ether solution was filtered and the solid residue was washed with more ether (50 mL). The combined ether extracts were concentrated, and the crude product thus obtained was purified by flash chromatography on silica gel with 30% ether/hexanes as eluent to give 4-[[7-diethylamino-2-(1,1-dimethylethyl)benz[b]-4H-pyran-4-ylidene]methyl]-3-butoxy-cyclobut-3-en-1,2-dione as a red solid (1.35 g, 29% yield) which melted at 145-146°C. The structure of this compound was confirmed by mass spectroscopy and by $^1H$ and $^{13}C$ NMR spectroscopy.

(The filtrate from the ether extraction was collected, dissolved in dichloromethane, washed sequentially with 1M hydrochloric acid, a saturated solution of sodium hydrogen carbonate and brine, and dried over magnesium sulfate. Removal of solvent yielded 3,4-bis[[7-diethylamino-2-(1,1-dimethylethyl)benz[b]-4H-pyran-4-ylidene]methyl]cyclobut-3-en-1,2-dione as a green solid (1.14 g, 37% yield) which did not melt below 300°C. The structure of this compound was confirmed by mass spectroscopy and by $^1H$ and $^{13}C$ NMR spectroscopy.)

Example 2 : Preparation of 2-(3-sulfonatoeth-1-ylamino)-4-[[7-diethylamino-2-[1,1-dimethylethyl]benz[b]-4H-pyran-4-ylidene]methyl]cyclobut-3-ene-1,2-dione

This is the aminosquaric acid derivative L in which $R^3$ is (notionally) a $-CH_2CH_2SO_3H$ grouping, and $R^1$ and $R^4$ are each a hydrogen atom, this derivative being the derivative of Formula II in which $Q^1$ is a 2-(1,1-dimethylethyl)-7-diethylaminobenz[b]-4H-pyran-4-ylidene grouping, $R^3$ is (notionally) a $-CH_2CH_2SO_3H$ grouping, and $R^1$ and $R^4$ are each a hydrogen atom.

4-[[7-Diethylamino-2-(1,1-dimethylethyl)benz[b]-4H-pyran-4-ylidene]methyl]-3-butoxycyclobut-3-ene-1,2-dione (155 mg, 0.36 mmol, prepared in Example 1 above), taurine (45 mg, 0.36 mmol) and triethylamine (40 mg, 0.4 mmol) were stirred overnight at room temperature in water/diglyme (5 mL/10 mL). The mixture was then concentrated under reduced pressure, removing the water and some of the diglyme. Ether was added, and a solid residue was formed, which was separated and washed with more ether. The remaining material was then treated with dichloromethane, which dissolved the desired product but not excess taurine, which was removed by filtration. Removal of dichloromethane gave orange crystals (80 mg) which were used in Example 4 below without further purification. The structure of this compound was confirmed by mass spectroscopy and by $^1H$ and $^{13}C$ NMR spectroscopy.

Example 3 : Preparation of 4-[[2-[1,1-dimethylethyl]-6-methoxybenz[b]-4Hthiopyran-4-ylidene]methyl]-3-propylaminocyclobut-3-ene-1,2-dione

This Example illustrates the preparation, by reactions analogous to

$$B + F \rightarrow J$$

shown in Figure 1 and

$$J \rightarrow L$$

(i.e., a reaction analogous to

$$K \rightarrow L$$

shown in Figure 2, but starting with the monoacid chloride rather than the monoester), of the aminosquaric acid derivative of Formula II in which $Q^1$ is a 2-[1,1-dimethylethyl]-6-methoxybenz[b]-4H-thiopyran-4-ylidene grouping, $R^3$ is an propyl group, and $R^2$ and $R^4$ are each a hydrogen atom.

A solution of 2-[1,1-dimethylethyl]-6-methoxy-4-methylbenzthiopyrylium tetrafluoroborate (31.0 g, 93 mmol, prepared in Example 8 below) in dry dimethylformamide (DMF, 200 mL) was added dropwise at room temperature under nitrogen to a solution of 3,4-dichlorocyclobut-3-ene-1,2-dione (15.7 g, 104 mmol, prepared as described in Schmidt, A. H., Synthesis, 1980, 963) in dry DMF (200 mL) over a period of 5 hours. A red-brown color developed. The reaction mixture was then cooled with an ice/water bath, and propylamine (71.9 g, 1.22 mol) was added in a slow stream over 10 minutes (a mildly exothermic reaction occurred). The dark red mixture was then stirred at room temperature for 17 hours, after which it was poured into a rapidly stirred mixture of ice/brine (1.5 L) and concentrated hydrochloric acid (100 mL). The solid which separated was collected by filtration, washed with water and air-dried to give the crude product as a red-brown powder. Purification was effected by dissolving this material in dichloromethane (1 L) and slurrying with silica gel (500 g of Selecto 142824 40 micron) for 10 minutes. The mixture was filtered, and the solid residue was washed with dichloromethane (10 100 mL aliquots). The solid residue was then set aside. The blue filtrate was treated in a similar manner with a further 250 g of the silica gel, and the cycle of filtration and washing was repeated. The solid residue was combined with that set aside earlier, and the desired product was extracted from the silica gel by slurrying with methanol for 10 minutes, followed by filtration. The filtrate was reserved, and the silica gel was washed with more methanol (10 100 mL aliquots). The combined methanol extracts were concentrated under reduced pressure, and the resultant residue was dissolved in dichloromethane (500 mL). Excess silica gel was removed by filtration, after which the solvent was removed under reduced pressure to give the pure aminosquarate derivative (21.17 g, 59.4% yield) as a brown powder. The structure of this compound was confirmed by mass spectroscopy and by $^1H$ and $^{13}C$ NMR spectroscopy.

**Examples 4 and 5 : Second process of the invention**

The following Examples 4 and 5 illustrate the second process of the invention, that is to say a reaction analogous to reaction

$$L \rightarrow A,$$

shown in Figure 2, together with a reaction used for the conversion of one compound of Formula I to another compound of Formula I.

Example 4 : Preparation of 4-[[2-[3-sulfonatoeth-1-ylamino]-3-[[7-diethylamino-2-[1,1-dimethylethyl]benz[b]-4H-pyran-4-ylidene]methyl]-4-oxo-2-cyclobuten-1-ylidene]methyl]-7-diethylamino-2-[1,1-dimethylethyl]benz[b]pyrylium inner salt dye

This Example illustrates the preparation, by the reaction

$$L \rightarrow A$$

shown in Figure 2, of the dye A in which $R^3$ is (notionally) a $-CH_2CH_2SO_3H$ grouping, and $R^1$, $R^2$ and $R^4$ are each a hydrogen atom, this being the dye of Formula I in which $Q^1$ is a 2-(1,1-dimethylethyl)-7-diethylaminobenz[b]-4H-pyran-

4-ylidene grouping, $Q^2$ is a 4-[2-(1,1-dimethylethyl)-7-diethylaminobenz[b]-4H-pyrylium] grouping, $R^3$ is (notionally) a -$CH_2CH_2SO_3H$ grouping, and $R^1$, $R^2$ and $R^4$ are each a hydrogen atom.

A solution of the orange material prepared in Example 2 above, (70 mg, 0.13 mmol), 7-diethylamino-2-[1,1-dimethylethyl]-4-methylbenzpyrylium tetrafluoroborate (50 mg, 0.14 mmol) and quinoline (17 mg, 0.13 mmol) was heated at reflux in butanol (5 mL) for 5 hours, then cooled and allowed to stand overnight at 5°C. The crude product was separated by filtration and washed with ether to afford red crystals of the dye (36 mg, 38% yield) which had a principal infrared absorption at 814 nm in dichloromethane solution, $\epsilon$ = 333,000. The structure of this dye was confirmed by mass spectroscopy and by $^1$H NMR spectroscopy.

Example 5 : Preparation of 6-[but-2-oxy]-4-[[3-[[2-[1,1-dimethylethyl]-6-methoxybenz[b]-4H-thiopyran-4-ylidene]methyl]-4-oxo-2-[propylamino]-2-cyclobuten-1-ylidene]methyl]-2-[1,1-dimethylethyl]benz[b]pyrylium tetrafluoroborate

This Example illustrates the preparation of the dye of Formula I in which $Q^1$ is a 2-[1,1-dimethylethyl]-6-methoxybenz[b]-4H-thiopyran-4-ylidene grouping, $Q^2$ is a 6-[but-2-oxy]-2-[1,1-dimethylethyl]benz[b]pyrylium grouping, $R^1$ and $R^2$ are each a hydrogen atom, and $\Omega$ is a propylamino group. In this Example, the reaction is promoted with a Lewis acid, namely titanium tetrachloride.

Titanium tetrachloride (8 mL of a 1M solution in dichloromethane, 8 mmole) was added to a solution of 4-[[2-[1,1-dimethylethyl]-6-methoxybenz[b]-4H-thiopyran-4-ylidene]methyl]-3-propylaminocyclobut-3-ene-1,2-dione (1.0 g, 2.6 mmol, prepared in Example 3 above), 6-[but-2-oxy]-2-[1,1-dimethylethyl]-4-methylbenzpyrylium tetrafluoroborate (1.0 g, 2.8 mmole, prepared in Example 10 below) and quinoline (2.8 g, 22 mmole) in dichloromethane (150 mL) at room temperature under nitrogen, and the mixture was stirred at room temperature for 2 hours. The mixture was then washed sequentially with 200 mL quantities of:

i) water containing tetrafluoroboric acid (10 mL of a 48% aqueous solution);
ii) water;
iii) 10% aqueous sodium hydrogen carbonate solution;
iv) water; and
v) 10% aqueous sodium tetrafluoroborate solution.

The organic layer was then dried over anhydrous sodium sulfate and concentrated. The residue was partially purified by flash chromatography on silica gel with 0-2% methanol/ dichloromethane as eluent. Further purification was effected by stirring the chromatographed material with methyl t-butyl ether (MTBE) (5 mL), storing the resultant suspension in a freezer (at about 5°C) for 17 hours, removing the solvent by decantation and triturating the residue with hexanes (25 mL). Air-drying following this purification afforded a brown powder, which still contained impurities. Final purification was achieved by stirring the brown powder for 5 minutes in a mixture of acetone (5 mL) and hexanes (10 mL). The brown, suspended material was collected by filtration, washed with a further small amount of 1:2 acetone/hexanes, and air dried to afford the dye (0.47 g, 25% yield). The dye had an absorption in the near infra-red in dichloromethane solution at 848 nm, $\varepsilon$ = 230,000. The structure of this compound was confirmed by mass spectroscopy and by $^1$H and $^{13}$C NMR spectroscopy.

**Example 6 : Third process of the invention**

The following Example 6 illustrates the third process of the invention, that is to say the preferred reaction for the preparation of the sulfonamide dyes of the invention.

Example 6 : Preparation of 4-[[3-[[2,6-bis[1,1-dimethylethyl]thio-4H-pyran-4-ylidene]methyl]-2-butanesulfonylamino-4-oxo-2-cyclobuten-1-ylidene]methyl]-2,6-bis-[1,1-dimethylethyl]thiopyrylium hydroxide inner salt dye

This Example illustrates the preparation of a 2-N dye of Formula I, in which $Q^1$ is a 2,6-bis(1,1-dimethylethyl)-4H-thiopyran-4-ylidene grouping, $Q^2$ is a 4-[2,6-bis(1,1-dimethylethyl)-4H-thiopyrylium] grouping, $R^3$ is a $CH_3CH_2CH_2CH_2SO_2$- grouping, and $R^1$, $R^2$ and $R^4$ are each a hydrogen atom, starting from the corresponding dye in which the squarylium nucleus is unsubstituted.

Butanesulfonyl isocyanate (3 drops, approx. 30 mg) was added to a solution of 4-[[3-[[2,6-bis[1,1-dimethylethyl]thio-4H-pyran-4-ylidene]methyl]-2-hydroxy-4-oxo-2-cyclobuten-1-ylidene]methyl]-2,6-bis[1,1-dimethylethyl]thiopyrylium hydroxide inner salt dye (100 mg, 0.19 mmol, prepared as described in U.S. Patent No. 4,508,811) in toluene (15 mL) and the reaction mixture was heated to 95°C for 2 hours. Additional butanesulfonyl isocyanate (3 drops) was added and heating was continued for a further 2 hours. A final addition of butanesulfonyl isocyanate (3 drops) then took place, followed by heating for 2 more hours. The reaction mixture was then diluted with ether and washed with water. The organic

layer was dried over sodium sulfate and concentrated under reduced pressure, then the residual dark green oil produced was purified by flash chromatography on silica gel. The material which eluted first from the column was the dye (3.4 mg, 3% yield) which had a principal infra-red absorption at 828 nm in dichloromethane solution. The structure of this compound was confirmed by $^1H$ NMR spectroscopy and mass spectroscopy.

**Examples 7-9 : Fourth process of the invention**

The following Examples 7-9 illustrate a fourth process of the invention, that is to say a reaction analogous to

$$Q \rightarrow R$$

shown in Figure 3, together with a reaction analogous to

$$P \rightarrow Q$$

shown in Figure 3 and another reaction necessary for the preparation of starting material.

Example 7 : Preparation of 2-[butoxy]-4-[propylamino]cyclobut-3-ene-1,2-dione

This Example illustrates a reaction analogous to

$$P \rightarrow Q$$

shown in Figure 3, but which produces the butyl homologue of the compound P in which the $NR^3R^4$ is a propylamino group.

A solution of propylamine (26 mL, 0.31 mmol) in ether (100 mL) was added to a solution of dibutyl squarate (57.68 g, 0.255 mmol) in ether (300 mL), and the mixture was allowed to stand at room temperature for two hours, then filtered and the filtrate concentrated under reduced pressure to yield a yellow, oily solid (52.46 g, 97.5% yield). The structure of this compound was confirmed by mass spectroscopy and by $^1H$ and $^{13}C$ NMR spectroscopy.

Example 8 : Preparation of 2-[1,1-dimethylethyl]-6-methoxy-4-methylbenz[b]thiopyrylium tetrafluoroborate

This Example illustrates the preparation of the 6-methoxy-7-unsubstituted thiopyrylium analogue of the compound M shown in Figure 2 in which $R^2$ is a hydrogen atom (i.e., the 4-substituent is a methyl group).

Part A : Preparation of ethyl 4,4-dimethylpent-2-ynoate

This is an improved preparation of a compound described in E. A. Halonen, Acta Chem. Scand., **9**, 1492-1497 (1955).

t-Butylacetylene (15.38 g, 0.188 mol) was dissolved in tetrahydrofuran (100 mL) in a 500 mL three-necked round-bottomed flask fitted with a nitrogen bubbler, rubber septum and dropping funnel. The resultant solution was cooled to -70°C using a dry ice/acetone bath, and butyl lithium (72 mL of a 2.5 M solution in hexanes, 0.18 mol) was added dropwise via a syringe. The cooling bath was then removed and the reaction mixture was stirred for 30 minutes, during which time the temperature in the flask rose to 10-15°C. The flask was then again cooled to -70°C and a solution of ethyl chloroformate (19.5 g, 0.18 mol) was added dropwise. The cooling bath was again removed, and the reaction mixture was stirred for 3 hours. Cold water (75 mL) was next added to quench the reaction, and the aqueous and organic phases were separated. The aqueous phase was extracted with THF (50 mL) and the combined organic phases were washed with 0.1 M hydrochloric acid (75 mL) and brine (100 mL), and dried over magnesium sulfate. Removal of the solvent under reduced pressure afforded a pale yellow oil (28 g) which was distilled under reduced pressure to provide the propiolate ester (23.5 g, 85% yield) as a colorless liquid which boiled at 70-75°C at 18-20 mm Hg. The structure of this compound was confirmed by mass spectroscopy and by $^1H$ and $^{13}C$ NMR spectroscopy.

Part B : Preparation of 4-dimethyl-3-(4-methoxyphenylthio)pent-2-enoic acid

This and the next step are modifications of the methods described in M. R. Detty and B. J. Murray, J. Am. Chem. Soc., 105, 883-890 (1983).

A solution of 4-methoxybenzene thiol (14.0 g, 0.1 mol) in methanol (25 mL) was added in one portion to sodium methoxide (21.6 g of a 25% solution in methanol, 0.1 mol) with ice/water bath cooling. The flask was warmed to room temperature, stirred for 15 minutes, and then cooled again using the ice/water bath. A solution of ethyl 4,4-dimethylpent-2-ynoate (15.4 g, 0.1 mol, prepared in Part A above) in methanol (25 mL) was next added in one portion. The reaction mixture was warmed to room temperature, stirred for one hour, and then diluted with ethanol (95%, 75 mL). Potassium hydroxide (30 mL of a 40% aqueous solution) was added, and the resultant solution was heated to 50-60 °C using a water bath, and stirred at this temperature for 2 hours. The mixture was then cooled to room temperature and diluted with cold water *(circa* 400 mL). The resultant cloudy suspension was extracted with carbon tetrachloride (3 x 100 mL) and the aqueous layer was acidified with ice-cold 6M hydrochloric acid (to about pH 3), whereupon a precipitate of the desired carboxylic acid separated. The product was extracted with dichloromethane (3 100 mL aliquots), and the solution was dried over magnesium sulfate. Removal of solvent under reduced pressure afforded the acid (21.4 g, 80% yield) as a white solid. The structure of this compound was confirmed by mass spectroscopy and by $^1H$ and $^{13}C$ NMR spectroscopy.

Part C : Preparation of 2-[1,1-dimethylethyl]-6-methoxybenz[b]-4H-thiopyran-4-one

Methanesulfonic acid (2.6 g, 27 mmol) was added to a suspension of 4-dimethyl-3-(4-methoxyphenylthio)pent-2-enoic acid (6.72 g, 25 mmol, prepared in Part B above) in acetic anhydride (20 mL). The reaction flask was then stirred and heated with a preheated oil bath at 80-85°C for about 30 minutes, during which time the color of the reaction mixture changed from brown to deep green. The mixture was then cooled and the reaction was quenched by addition of crushed ice/water (50 g). The resultant mixture was stirred for 20 minutes, then extracted with hexanes (5 50 mL aliquots). The combined hexane extracts were washed with a saturated aqueous solution of sodium hydrogen carbonate and with brine, and dried over magnesium sulfate. Removal of solvent under reduced pressure afforded the thiochromone (5.74 g). $^1H$ NMR indicated that the material was almost pure, so this material was used without further purification in the reaction of Part D below.

Part D : Preparation of 2-[1,1-dimethylethyl]-6-methoxy-4-methylbenz[b]thiopyrylium tetrafluoroborate

Methyl magnesium bromide (50 mL of a 3M solution in ether, 0.15 mmol) was added to a solution of the crude thiochromone (20 g, prepared in Part C above) in THF (100 mL) with cooling to 10°C. The reaction mixture was then allowed to warm to room temperature and stirred for 6 hours. The mixture was then added, with vigorous stifling, to tetrafluoroboric acid (125 mL of a 50% aqueous solution) which had been diluted with ice/water (600 mL). A yellow precipitate formed, which was collected by vacuum filtration, washed thoroughly with hexanes and dried under reduced pressure to yield the desired salt (18.5 g, 55% yield over three steps from the 4-methoxybenzene thiol). The structure of this compound was confirmed by mass spectroscopy and by $^1H$ and $^{13}C$ NMR spectroscopy.

Example 9 : Preparation of 4-[[3-[[2-[1,1-dimethylethyl]-6-methoxybenz[b]-4H-thiopyran-4-ylidene]methyl]-4-oxo-2-[propylamino]-2-cyclobuten-l-ylidene]methyl]-2-[1,1-dimethylethyl]-6-methoxybenz[b]thiopyrylium tetrafluoroborate

A solution of 2-[butoxy]-4-[propylamino]-cyclobut-3-ene-1,2-dione (5.16 g, 24.4 mmol, prepared in Example 7 above) in dichloromethane (100 mL) was placed in a 2 L, three-necked round-bottomed flask equipped with a mechanical stiffer, under a nitrogen atmosphere. The flask was then charged with a suspension of 2-[1,1-dimethylethyl]-6-methoxy-4-methylbenzthiopyrylium tetrafluoroborate (8.17 g, 24.4 mmol, prepared in Example 8 above) in dichloromethane (400 mL), followed by a solution of quinoline (25.1 g, 0.2 mol) in dichloromethane (50 mL), and finally by a solution of titanium tetrachloride (26.7 mL of a 1M solution in dichloromethane, added using a syringe); a somewhat exothermic reaction followed the final addition. The resultant reaction mixture was stirred for 17 hours, after which time tetrafluoroboric acid (150 mL of a 55% aqueous solution) was added. After a further one hour's stirring, the mixture was extracted with water (2 100 mL aliquots), and the dichloromethane layer was washed with saturated sodium bicarbonate solution (2 100 mL aliquots) and saturated sodium tetrafluoroborate solution (2 100 mL aliquots), dried, and concentrated under reduced pressure. The crude material thus obtained was dissolved in dichloromethane (50 mL) and precipitated with MTBE (400 mL). Repeating this procedure provided the dye as a red-brown solid (5.21 g, 61% yield). The dye exhibited a near infrared absorption in dichloromethane solution at 902 nm, $\varepsilon$ = 218,000. The structure of this compound was confirmed by mass spectroscopy and by $^1H$ and $^{13}C$ NMR spectroscopy.

**Example 10**

The following Example illustrates the fourth process of the invention carried out with promotion by a Lewis acid.

Example 10 : Preparation of 4-[[2-amino-3-[[6-[but-2-oxy]-2-[1,1-dimethylethyl]benz[b]-4H-pyran-4-ylidene]methyl]-4-oxo-2-cyclobuten-l-ylidene]methyl]-6-[but-2-oxy]-2-[1,1-dimethylethyl]benz[b]pyrylium tetrafluoroborate

This example illustrates the preparation of the dye of Formula I in which $Q^1$ is a 6-[but-2-oxy]-2-[l,l-dimethylethyl]benz[b]-4H-pyran-4-ylidene grouping, $Q^2$ is a 6-[but-2-oxy]-2-[1,1-dimethylethyl]benz[b]pyrylium grouping, $R^1$ and $R^2$ are each a hydrogen atom, and Ω is an amino group.

Part A : Preparation of 2-[1,1-dimethylethyl]-6-hydroxybenz-4H-pyran-4-one

Trifluoromethanesulfonic acid (150 g) was added in a slow stream over a period of 30 minutes to a mixture of hydroquinone (30.0 g, 0.272 mol) and methyl 4,4-dimethyl-3-oxopentanoate (48.0 g, 0.304 mol) with ice/water cooling to control the mildly exothermic reaction which ensued. The reaction mixture was then warmed to 50-55°C and held at that temperature for 3 hours, during which time a red solution developed and, later, some solid material separated. The reaction mixture was then cooled and poured into stirred ice/water (1500 mL) containing saturated brine (100 mL), whereupon a gum separated, which solidified with scratching. This material was collected by filtration, washed with water and air-dried to give the desired compound as a pale yellow powder (46.7 g, 79% yield). The structure of this compound was confirmed by mass spectroscopy and by $^1H$ and $^{13}C$ NMR spectroscopy.

Part B : Preparation of 6-[but-2-oxy]-2-[1,1-dimethylethyl]benz-4H-pyran-4-one

A mixture of 2-[1,1-dimethylethyl]-6-hydroxybenz-4H-pyran-4-one (25.0 g, 0.115 mol, prepared in Part A above), 2-bromobutane (25.95 g, 0.189 mol), potassium carbonate (50.0 g, 0.36 mol) and potassium iodide (20.0 g, 0.12 mol) in MEK (250 mL) was stirred and heated at reflux under nitrogen for 8 hours. A further quantity of 2-bromobutane (8.65 g, 0.063 mol) was then added, and heating was continued for another 16 hours. The mixture was cooled and poured into stirred ice/water (1000 mL) and the mixture so formed was extracted with dichloromethane (2 400 mL aliquots). The combined organic extracts were washed with water (200 mL) and brine (200 mL), dried over magnesium sulfate and concentrated under reduced pressure to give the desired compound as a viscous, golden-brown oil (27.98 g, 89% yield). The structure of this compound was confirmed by mass spectroscopy and by $^1H$ and $^{13}C$ NMR spectroscopy.

Part C : Preparation of 6-[but-2-oxy]-2-[1,1-dimethylethyl]-4-methylbenzpyrylium tetrafluoroborate

Methyl magnesium bromide (100 mL of a 3M solution in ether, 0.3 mol) was added over a period of 20 minutes to a solution of 6-[but-2-oxy]-2-[1,1-dimethylethyl]benz-4H-pyran-4-one (27.22 g, 0.099 mol, prepared in Part B above) in dry THF (250 mL) maintained below 10°C with an ice/water bath; some solid material was observed to separate from the yellow-brown solution. The ice bath was removed, and the reaction mixture was stirred at room temperature for 16 hours. The mixture was then poured cautiously into a rapidly stirred solution of tetrafluoroboric acid (90 mL of a 48% aqueous solution) in ice/water (1000 mL). Vigorous effervescence was observed, and the precipitate which formed was collected by filtration, washed with water, and air-dried to yield the salt as a pale yellow powder (30.08 g, 84% yield). The structure of this compound was confirmed by mass spectroscopy and by $^1H$ and $^{13}C$ NMR spectroscopy.

Part D : Preparation of 4-amino-3-[but-1-oxy]cyclobut-3-ene-1,2-dione

Ammonia gas was bubbled into a solution of dibutyl squarate (22.6 g, 100 mmol) in ether (500 mL) contained in a 1 L three-necked, round-bottomed flask equipped with a mechanical stirrer and an acidic scrubber. Precipitation of product ceased after 3 hours, so the reaction was halted and the ether solution was filtered. The solid residue was washed with ether and set aside. The filtrate was allowed to stand at room temperature for a further 3 hours, after which time more solid was observed to have been precipitated. This solid was collected by filtration, washed with ether, and combined with the material set aside previously. Drying in vacuo afforded the product as a white solid (15.7 g, 92.9% yield). The structure of this compound was confirmed by mass spectroscopy and by $^1H$ and $^{13}C$ NMR spectroscopy.

Part E : Preparation of 4-[[2-amino-3-[[6-[but-2-oxy]-2-[1,1-dimethylethyl]benz[b]-4H-pyran-4-ylidene]methyl]-4-oxo-2-cyclobuten-1-ylidene]methyl]-6-[but-2-oxy]-2-[1,1-dimethylethyl]benz[b]pyrylium tetrafluoroborate

A solution of 6-[but-2-oxy]-2-[1,1-dimethylethyl]-4-methylbenzpyrylium tetrafluoroborate (180 mg, 0.5 mmol, prepared in Part C above), 4-amino-3-butoxycyclobut-3-ene-1,2-dione (43 mg, 0.25 mmol, prepared in Part D above), quinoline (762 mg, 6 mmol), and trimethylsilyl chloride (325 mg, 3 mmol) in dichloromethane (5 mL) was heated at reflux for 17 hours. The reaction mixture was then cooled to room temperature, and tetrafluoroboric acid (5 mL of a 48% aqueous solution) was added. Following this addition, the mixture was stirred rapidly for 30 minutes, during which time a precipitate formed. The precipitate was removed by filtration and reserved, while the filtrate was extracted with

dichloromethane (2 25 mL aliquots). The combined organic layers were washed with water (25 mL) and concentrated under reduced pressure to yield a residue which was combined with the precipitate reserved earlier. Acetone (5 mL) was added to this combined crude material, and the resultant suspension was stirred for 17 hours. The solid material remaining was collected by filtration, washed with acetone, and air-dried to afford the dye (75 mg, 42% yield) as coppery crystals. The dye exhibited a near infra-red absorption in dichloromethane solution at 782 nm, $\varepsilon$ = 319,000. The structure of this compound was further confirmed by mass spectroscopy.

Example 11 : Preparation of 4-[[4-dicyanomethyl-3-[[7-diethylamino-2-[1,1-dimethylethyl]benz[b]-4H-pyran-4-ylidene]methyl]-cyclobut-3-ene-2-one-1-ylidene]methyl]-7-diethylamino-2-[1,1-dimethylethyl]benz[b]pyrylium hydroxide inner salt dye

This is the 2-dicyanomethyl analogue of the dye of Formula A shown in Figure 2, this being the compound of Formula I in which $Q^1$ is a 7-diethylamino-2-[1,1-dimethylethyl]benz[b]-4H-pyran-4-ylidene grouping, $Q^2$ is a 7-diethylamino-2-[1,1-dimethylethyl]benz[b]-4H-pyrylium grouping, $R^1$ and $R^2$ are each a hydrogen atom, and $\Omega$ is a dicyanomethyl grouping in the protonated form of the dye. Under neutral conditions, the $R^7$ proton is removed, yielding an uncharged dye.

Part A : Preparation of 4-dicyanomethyl-3-[[7-diethylamino-2-[1,1-dimethylethyl]benz[b]-4H-pyran-4-ylidene]methyl]cyclobut-3-ene-1,2-dione, diazabicycloundecene salt

This is the compound of Formula II in which $Q^1$ is a 7-diethylamino-2-[1,1-dimethylethyl]benz[b]-4H-pyran-4-ylidene grouping, $R^1$ is a hydrogen atom, and $\Omega$ is a dicyanomethyl grouping. The compound was produced in the form of a salt, in which the hydrogen atom of the dicyanomethyl grouping is removed, giving the squarylium moiety a net negative charge, the hydrogen atom being transferred to a diazabicycloundecene base.

A solution of 1,8-diazabicyclo[5.4.0]undec-7-ene (DBU, 36 mg, 0.24 mmol) in tetrahydrofuran (THF, 0.5 mL) was added to a solution of 3-butoxy-4-[[7-diethylamino-2-[1,1-dimethylethyl]benz[b]-4H-pyran-4-ylidene]methyl]-cyclobut-3-ene-1,2-dione (100 mg, 0.24 mmol, prepared in Example 1 above) and malononitrile (16 mg, 0.24 mmol) in THF (2 mL). After one hour, further quantities of DBU (36 mg, 0.24 mmol) and malononitrile (16 mg, 0.24 mmol) were added, and the reaction mixture was stirred for a further 2 hours. The solvent was then removed under reduced pressure and the residue was washed with ether, causing it to crystallize. The crystals were collected and washed with ether to give the desired salt (62 mg, 46% yield) as a red solid. The structure of this compound was confirmed by mass spectroscopy and by $^1H$ and $^{13}C$ NMR spectroscopy.

Part B : Preparation of 4-[[4-dicyanomethyl-3-[[7-diethylamino-2-[1,1-dimethylethyl]benz[b]-4H-pyran-4-ylidene]methyl]-cyclobut-3-ene-2-one-1-ylidene]methyl]-7-diethylamino-2-[1,1-dimethylethyl]benz[b]pyrylium hydroxide inner salt dye

A solution of 4-dicyanomethyl-3-[[7-diethylamino-2-[1,1-dimethylethyl]benz[b]-4H-pyran-4-ylidene]methyl]-cyclobut-3-ene-1,2-dione hydroxide, diazabicycloundecene salt (50 mg, 0.09 mmol, prepared in Part A above), 7-diethylamino-2-[1,1-dimethylethyl]-4-methylbenzpyrylium tetrafluoroborate (prepared as described in the aforementioned International Application No. PCT/US91/08695), 50 mg, 0.14 mmol) and quinoline (5 drops) was heated at reflux in n-butanol (5 mL) for 5 hours, then cooled and allowed to stand overnight. The crude product was separated by filtration and washed with ether to afford brown crystals of the desired dye (30 mg, 50% yield) which had a principal infrared absorption at 851 nm in dichloromethane solution, $\epsilon$ = 312,000. The structure of this compound was confirmed by mass spectroscopy and by $^1H$ and $^{13}C$ NMR spectroscopy.

**Examples 12-14 : Fifth process of the invention**

The following Examples 12-14 illustrate the fifth process of the invention, that is to say reactions analogous to

$$T \rightarrow U$$

shown in Figure 4, together with a reaction analogous to

$$S \rightarrow T$$

shown in Figure 4.

Example 12 : Preparation of 6-[but-2-oxy]-2-[1,1-dimethylethyl]-4-methylidenebenz[b]-4H-pyran

This Example illustrates the reaction

$$T \rightarrow U$$

shown in Figure 4 carried out by means of a Peterson olefination.

Trimethylsilylmethylmagnesium chloride (5 mL of a 1 M in ether, 5 mmol) was added to a solution of 6-[but-2-oxy]-2-[1,1-dimethylethyl]benz-4H-pyran-4-one (1.37 g, 5 mmol, prepared in Example 10, Part B above) in anhydrous THF (10 mL) under a nitrogen atmosphere, and the solution was heated to reflux for 3 hours. Thin layer chromatography of an aliquot indicated that the reaction was not complete, so an additional amount of trimethylsilylmethylmagnesium chloride (5 mL of a 1 M in ether, 5 mmol) was added and the reaction solution was heated at reflux for a further 17 hours. An aqueous solution of sodium hydroxide was then added, and the mixture was heated at reflux for a further 1 hour, cooled, and filtered through a short plug of Celite (manufactured by Johns-Manville Corporation, Denver, Colorado 80217). The upper, organic layer was separated, dried over anhydrous magnesium sulfate and concentrated to afford the crude product as a light yellow oil (1.05 g, 77% yield). The structure of this compound was confirmed by mass spectroscopy and by $^1H$ and $^{13}C$ NMR spectroscopy.

Example 13 : Preparation of 6-[but-2-oxy]-2-[1,1-dimethylethyl]-4-methylidenebenz[b]-4H-pyran

This Example illustrates the same reaction as in Example 12 above, but carried out by use of a strong base on the corresponding 4-methyl salt.

Part A : Using potassium t-butoxide

A solution of potassium t-butoxide (1 mL of a 1.0 M solution in 2-methyl-2-propanol, 1 mmol) was added dropwise to a suspension of 6-[but-2-oxy]-2-[1,1-dimethylethyl]-4-methylbenzpyrylium tetrafluoroborate (0.36 g, 1 mmol, prepared in Example 10, Part C above) in heptanes (10 mL) under a nitrogen atmosphere. The green suspension was converted to a brown solution after stirring at room temperature for 30 minutes. The reaction mixture was stirred for an additional 30 minutes, then poured into ice/water. The organic layer was separated, dried over anhydrous magnesium sulfate and concentrated under reduced pressure to afford the product as a brown oil (0.27 g, 100% yield). This material was spectroscopically identical to that prepared in Example 12 above.

Part B : Using potassium hydride

A small amount of dry heptanes was added to a dispersion of potassium hydride in mineral oil. The resultant suspension was centrifuged and the upper layer was decanted to give a gray solid. This solid was dried in vacuo to afford 0.16 g of potassium hydride, which was then placed under a nitrogen atmosphere. Dry heptanes (10 mL) and 6-[but-2-oxy]-2-[1,1-dimethylethyl]-4-methylbenzpyrylium tetrafluoroborate (1.44 g, 4 mmol, prepared in Example 10, Part C above) were then added sequentially at room temperature. The solid suspension dissolved within one hour. After stirring at room temperature for an additional 1 hour, the solution was poured into ice/water, and the organic layer was separated, dried over anhydrous magnesium sulfate and concentrated under reduced pressure to afford the desired product as a brown oil (0.9 g, 82.5% yield). This material was spectroscopically identical to that prepared in Example 12 above.

Example 14 : Preparation of 3-amino-4-[[2,6-bis-[1,1-dimethylethyl]seleno-4H-pyran-4-ylidene]methyl]cyclobut-3-ene-1,2-dione

This Example illustrates reactions analogous to

$$S \rightarrow T \rightarrow U$$

shown in Figure 4 but in which the starting material is the seleno analogue of the salt R shown in Figure 2, in which $R^1$ is a hydrogen atom.

A solution of potassium t-butoxide (1 mL of a 1.0 M solution in 2-methyl-2-propanol, 1 mmol) was added dropwise to a suspension of 2,6-bis-[1,1-dimethylethyl]-4-methylselenopyrylium tetrafluoroborate (0.36 g, 1 mmol, prepared as described in the aforementioned European Application) in heptanes (10 mL) under a nitrogen atmosphere. The brown solid suspension was converted to a clear solution after stirring at room temperature for 30 minutes. The reaction mixture was stirred for an additional 30 minutes, then poured into ice/water. The organic layer was separated, dried over anhydrous magnesium sulfate and concentrated under reduced pressure to afford 0.27 g of a reddish-brown oil.

A solution of this brown oil in THF (14 mL) was added dropwise to a suspension of 4-amino-3-[but-1-oxy]cyclobut-3-ene-1,2-dione (0.114 g, 1 mmol, prepared in Example 10, Part D above) and aluminum chloride (132 mg, 1 mmol) in THF (10 mL) under a nitrogen atmosphere. A red solution formed immediately after the addition. After stirring at room temperature for 17 hours, the reaction solution was quenched into ice/water. The organic layer was separated, dried over anhydrous magnesium sulfate, and concentrated under reduced pressure to afford an impure product. This product was purified by preparative thin-layer chromatography on silica gel with 10% methanol/dichloromethane as eluent to afford the desired product (30 mg, 9% yield). The structure of this compound was confirmed by mass spectroscopy and by $^1H$ and $^{13}C$ NMR spectroscopy.

**Example 15, 56 : Sixth process of the invention**

The following Example 15, 56 illustrates the sixth process of the invention, that is to say reactions analogous to

$$V \rightarrow W$$

and

$$W \rightarrow X$$

shown in Figure 5.

Example 15 : Preparation of 4-[[3-[[2,6-bis[1,1-dimethylethyl]thio-4H-pyran-4-ylidene]methyl]-2-carboxymethylamino-4-oxo-2-cyclobuten-1-ylidene]methyl]-2,6-bis[1,1-dimethylethyl]thiopyrylium hydroxide inner salt dye

This example illustrates the preparation, by reactions analogous to

$$V \rightarrow W \rightarrow X$$

shown in Figure 5, of the dye of Formula N (Figure 3) in which $R^3$ is a carboxymethylamino group and $R^1$ and $R^4$ are each a hydrogen atom, this being the 2-N dye of Formula I in which $Q^1$ is a 2,6-bis[1,1-dimethylethyl]thio-4H-pyran-4-ylidene grouping, $Q^2$ is a 2,6-bis[1,1-dimethylethyl]thiopyrylium grouping, $R^3$ is a carboxymethyl group and $R^1$, $R^2$ and $R^4$ are each a hydrogen atom. Due to deprotonation of the carboxyl group, this dye exists as an inner salt. The amine used to form the amino group is glycine.

Part A : Preparation of 4-[[3-[[2,6-bis[1,1-dimethylethyl]thio-4H-pyran-4-ylidene]methyl]-2-methoxy-4-oxo-2-cyclobuten-1-ylidene]methyl]-2,6-bis[1,1-dimethylethyl]thiopyrylium tetrafluoroborate

Dimethyl sulfate (4.5 mL, 6.0 g, 47.6 mmol) was added to a solution of 4-[[3-[[2,6-bis[1,1-dimethylethyl]thio-4H-pyran-4-ylidene]methyl]-2-hydroxy-4-oxo-2-cyclobuten-1-ylidene]methyl]-2,6-bis[1,1-dimethylethyl]thiopyrylium hydroxide inner salt dye (4.50 g, 8.62 mmol, prepared as described in U.S. Patent No. 4,508,811) in dichloromethane (75 mL). The resultant solution was stirred at reflux for 6 hours, then allowed to stand for 48 hours at 20°C, after which it was examined by thin layer chromatography to ensure complete reaction. (Silica gel plates eluted with 5% methanol in methylene chloride show the starting dye at $R_f$ 0.75 (brown fading to green) and the desired product streaking at $R_f$ 0.1-0.35. A blue impurity at $R_f$ 0.9 corresponds to an impurity in the starting material.) Sodium methoxide (7.56 g of a 25% methanolic solution, 35.0 mmol) was then added dropwise over a period of two minutes and the resulting dark brown solution was left at 20° for 3 hours (this treatment with sodium methoxide is intended to destroy residual dimethyl sulfate, a toxic methylating agent). The reaction mixture was then poured into 60 mL of ice-water containing tetrafluoroboric acid (15 mL of a 48% aqueous solution), and the organic layer was washed with dilute aqueous sodium tetrafluoroborate, stirred over solid sodium tetrafluoroborate (3.0 g) and silica gel (1.0 g) and then filtered. The filter cake was washed with methylene chloride (20 mL) and the combined filtrates were evaporated to *circa* 15 mL. The concentrated solution was diluted with methyl t-butyl ether (MTBE, 50 mL) and stirred at 20° for one hour, during which time a precipitate formed, which was collected by filtration. The product was washed with MTBE (20 mL) and dried in vacuo to provide the methylated dye as its tetrafluoroborate salt (4.69 g, 87.2% yield; HPLC analysis indicated a purity of 99.7% by area at 365 nm). Further dilution of the liquors with MTBE (*circa* 100 mL) provided an additional crop of 0.232 g (4.3%) of the methylated dye. The dye exhibited an infra-red absorption at 787 nm in dichloromethane solution, $\varepsilon$ = 330,000. The structure of this compound was confirmed by mass spectroscopy and by $^1H$ and $^{13}C$ NMR spectroscopy.

Part B : Preparation of 4-[[3-[[2,6-bis[1,1-dimethylethyl]thio-4H-pyran-4-ylidene]methyl]-2-carboxymethylamino-4-oxo-2-cyclobuten-1-ylidene]methyl]-2,6-bis[1,1-dimethylethyl]thiopyrylium hydroxide inner salt dye

Tetrabutylammonium hydroxide (1.5 mL of a 1M methanol solution, 1.50 mmol) was added to a suspension of glycine (112 mg, 1.5 mmol) in methanol (4.0 mL) and the resulting clear solution was charged at 5-10°C with 4-[[3-[[2,6-bis-[1,1-dimethylethyl]thio-4H-pyran-4-ylidene]methyl]-2-methoxy-4-oxo-2-cyclobuten-1-ylidene]methyl]-2,6-bis[1,1dimethylethyl]thiopyrylium tetrafluoroborate (624 mg, 0.10 mmol, prepared in Part A above); a brown color developed. After 35 minutes the reaction mixture was quenched into 5% aqueous tetrafluoroboric acid (55 mL). The resulting black gum was washed with water (40 mL), taken up in methylene chloride (20 mL) and washed again with water, then evaporated to a red-black solid which was chromatographed on silica gel, eluting with a 15% methanol, 25% acetone, 60% dichloromethane mixture, to give 1.0 g of a brick-red partial solid, partial gum. A 300 mg portion of this gum was deprotonated by dissolution in dichloromethane, washing with dilute sodium bicarbonate, evaporation to 2.5 mL, and dilution with MTBE (12 mL). The resulting precipitate was collected by filtration and washed with MTBE (10 mL), then concentrated under reduced pressure to furnish the desired product as brick-red, fine prisms (91.2 mg, 52.4% yield). Further dilution of the mother liquors with MTBE (50 mL) provided a second crop of 53.2 mg (30.6%) of product.

The dye exhibited an infra-red absorption at 828 nm in dichloromethane solution, $\varepsilon = 285{,}000$. The structure of this compound was confirmed by mass spectroscopy and by $^{1}H$ and $^{13}C$ NMR spectroscopy.

**Examples 16-60**

The following additional compounds of the present invention, and intermediates useful in the synthesis thereof, were prepared by the same techniques as described in the preceding Examples. In the columns headed "Characterization", "MS" indicates characterization by mass spectroscopy, "$^{1}H$" characterization by proton nuclear magnetic resonance spectroscopy, and "$^{13}C$" characterization by carbon-13 nuclear magnetic resonance spectroscopy. In the following Table, "Me", "Et", "Pr" and "Bu" denote methyl, ethyl, propyl and butyl respectively. Unless otherwise stated, all salts are present as tetrafluoroborates, and all absorption measurements are in dichloromethane.

## INTERMEDIATES

| Example # | Formula | Characterization |
|---|---|---|
| 16 | 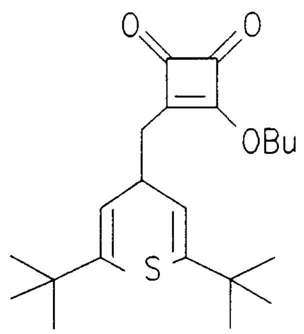 | - |
| 17 | 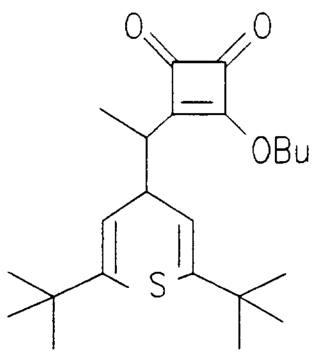 | - |
| 18 | 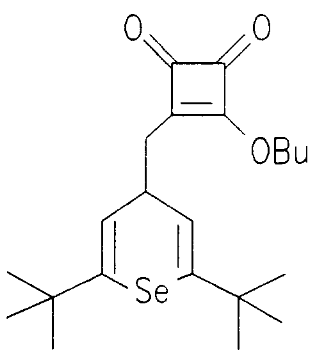 | - |

| Example # | Formula | Characterization |
|---|---|---|
| 19 | 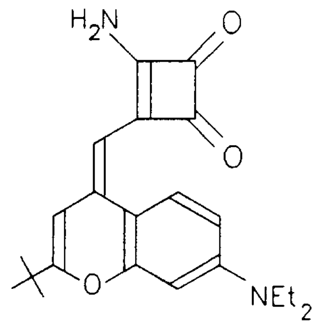 | melting point 261-264°C, $^{13}C$, $^{1}H$, MS |
| 20 | 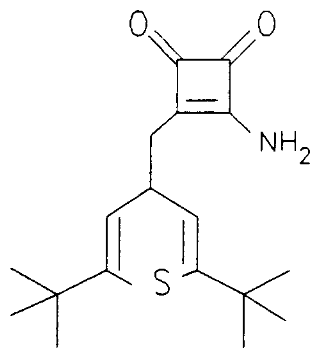 | $^{13}C$, $^{1}H$, MS |
| 21 | 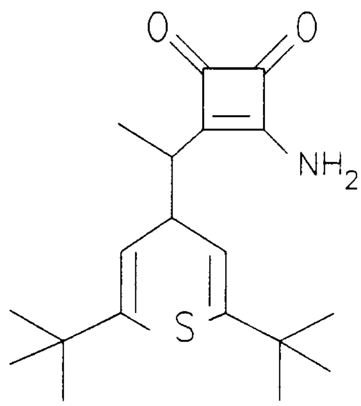 | $^{13}C$, $^{1}H$, MS |

| Example # | Formula | Characterization |
|---|---|---|
| 22 | 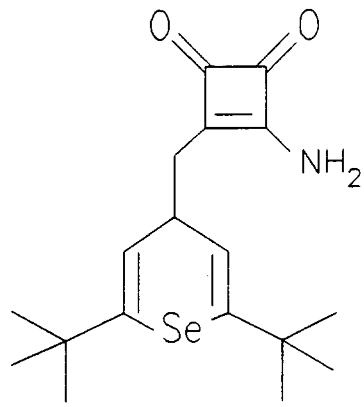 | $^{13}C$, $^{1}H$, MS |
| 23 | 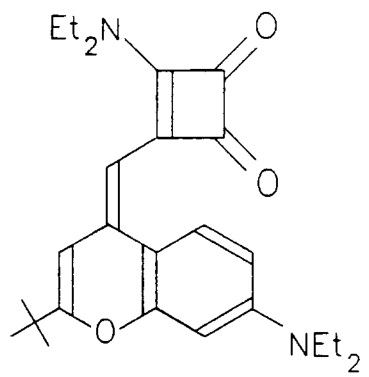 | $^{13}C$, $^{1}H$, MS |
| 24 | 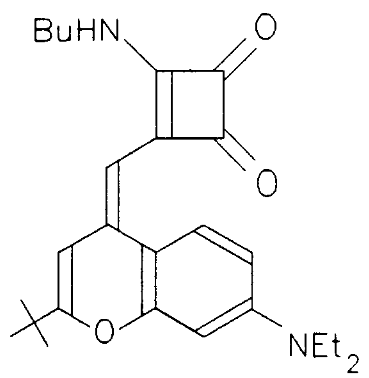 | $^{13}C$, $^{1}H$, MS |

| Example # | Formula | Character-ization |
| --- | --- | --- |
| 25 | 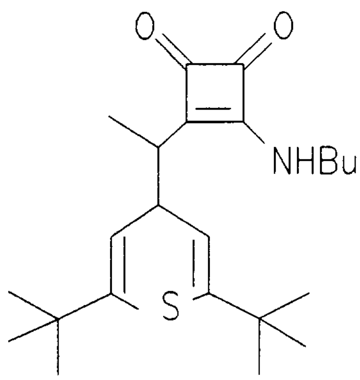 | $^{13}C$, $^{1}H$, MS |
| 26 | 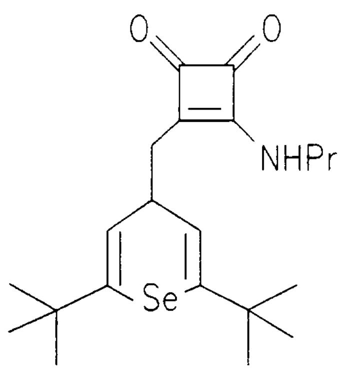 | $^{1}H$, MS |
| 27 | 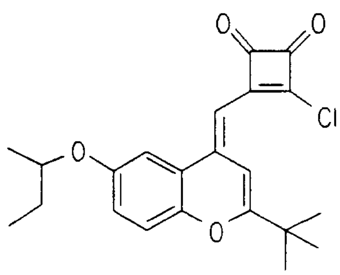 | $^{13}C$, $^{1}H$, MS |
| 28 | 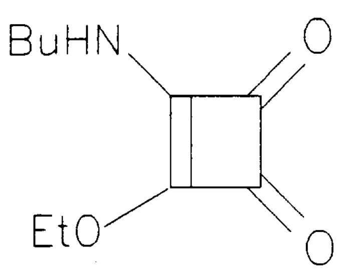 | $^{13}C$, $^{1}H$, MS |

| Example # | Formula | Character-ization |
|---|---|---|
| 29 | 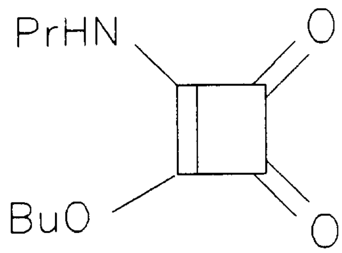 | $^{13}C$, $^{1}H$ |
| 30 | 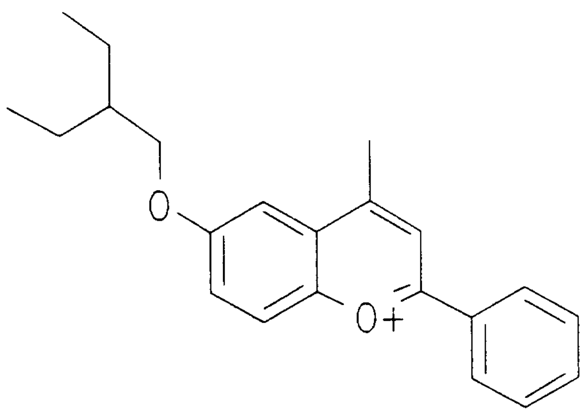 | $^{13}C$, $^{1}H$, MS |
| 31 | 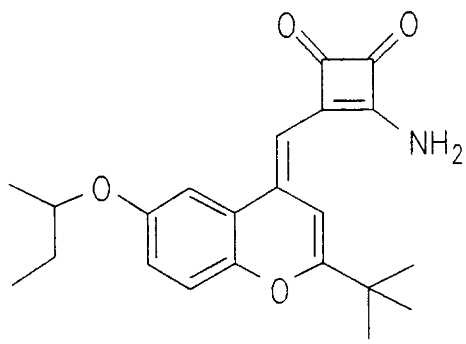 | $^{13}C$, $^{1}H$, MS |

| Example # | Formula | Character-ization |
|---|---|---|
| 32 | 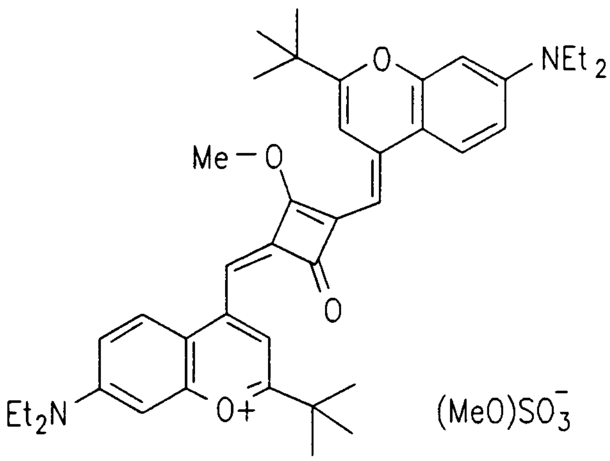 | $^{1}H$, MS, $\lambda_{max}$ 786 nm, $\varepsilon$ = 426,000 |
| 33 | 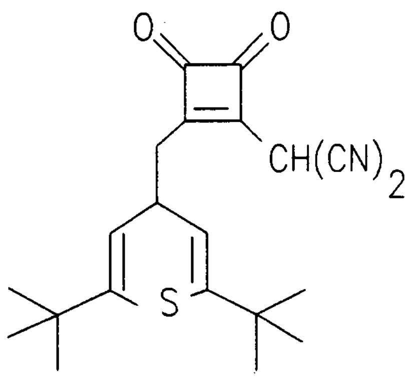 (as diazabicycloundecene salt) | $^{13}C$, $^{1}H$, MS |
| 34 | 2-Phenyl analogue of Example 19 above | $^{13}C$, $^{1}H$, MS |
| 35 | 2-Ethyl analogue of Example 19 above | $^{13}C$, $^{1}H$, MS |

## DYES

| Example # | Formula | Characterization |
|---|---|---|
| 36 | 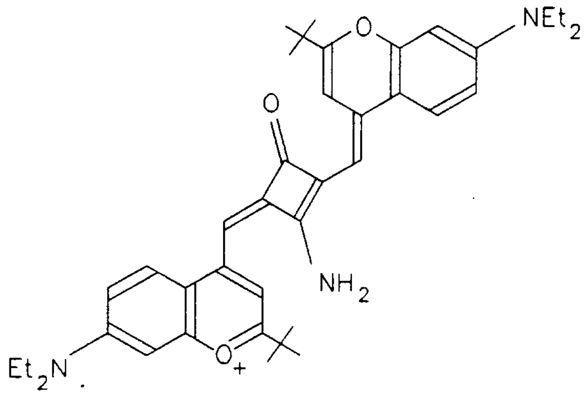 | $^{13}C$, $^{1}H$, MS, $\lambda_{max}$ 797 nm, ε=334,000 |
| 37 | 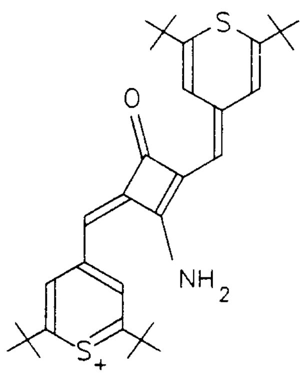 | $^{1}H$, MS, $\lambda_{max}$ 805 nm, ε=224,000 |
| 38 | 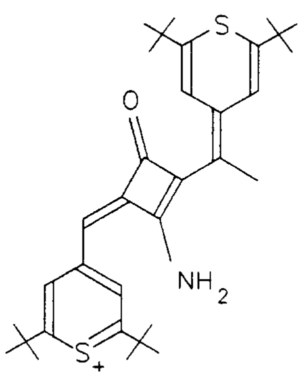 | MS, $\lambda_{max}$ 834 nm (impure) |

| Example # | Formula | Characterization |
|---|---|---|
| 39 | 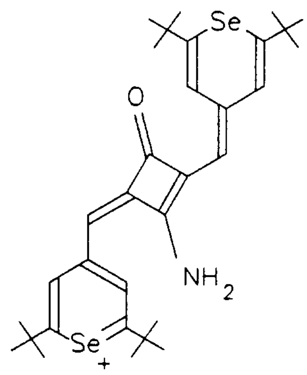 | $^{13}C$, $^{1}H$, MS, $\lambda_{max}$ 844 nm, ε=287,000 |
| 40 | 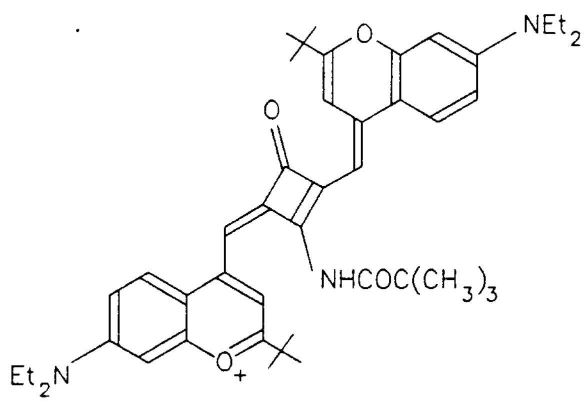 | $^{1}H$, MS, $\lambda_{max}$ 782 nm, ε=282,000 |
| 41 | 2-Toluenesulfonylamino analogue of Example 36 above | $^{13}C$, $^{1}H$, MS, $\lambda_{max}$ 814 nm, ε=264,000 |
| 42 | 2-Diethylamino analogue of Example 36 above | $^{13}C$, $^{1}H$, MS, $\lambda_{max}$ 828 nm, ε=315,000 |
| 43 | 2-Butylamino analogue of Example 36 above | $^{1}H$, MS, $\lambda_{max}$ 810 nm |
| 44 | 2-Propylamino analogue of Example 39 above | MS, $\lambda_{max}$ 860 nm |
| 45 | 2-Butylamino analogue of Example 37 above | $^{13}C$, $^{1}H$, MS, $\lambda_{max}$ 816 nm, ε=135,000 |

| Example # | Formula | Characterization |
|---|---|---|
| 46 | 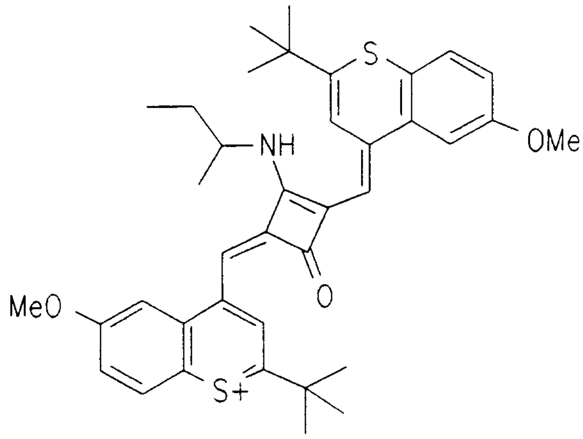 | $^{13}C$, $^{1}H$, MS, $\lambda_{max}$ 902 nm, ε=179,200 |
| 47 | 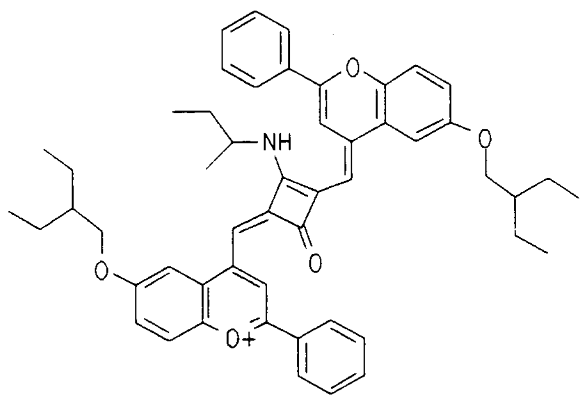 | MS, $\lambda_{max}$ 856 nm, ε=201,200 |
| 48 | 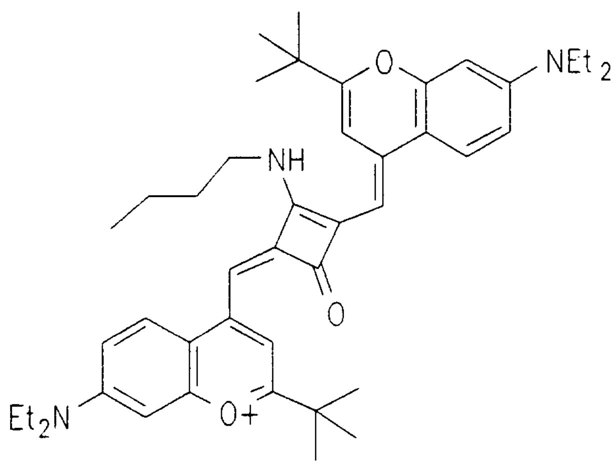 | $^{13}C$, $^{1}H$, MS, $\lambda_{max}$ 810 nm, ε=410,000 |

| Example # | Formula | Character-ization |
|---|---|---|
| 49 | 2-Amino analogue of Example 48 above | MS (m/e 621), $\lambda_{max}$ 795 nm |
| 50 | 2-Methylamino analogue of Example 48 above | MS (m/e 635), $\lambda_{max}$ 808 nm |
| 51 | 2-n-Pentylamino analogue of Example 48 above | MS (m/e 691), $\lambda_{max}$ 808 nm |
| 52 | 2-Dimethylamino analogue of Example 48 above | MS (m/e 649), $\lambda_{max}$ 827 nm |
| 53 | 2-Diethylamino analogue of Example 48 above | MS (m/e 677), $\lambda_{max}$ 828 nm |
| 54 | 2-Dibutylamino analogue of Example 48 above | MS (m/e 733), $\lambda_{max}$ 828 nm |
| 55 | 2-Phenylamino analogue of Example 48 above | MS (m/e 696), $\lambda_{max}$ 801 nm |
| 56 | 2-Carboxymethylamino analogue of Example 37 above | $^{13}C$, $^{1}H$, MS, $\lambda_{max}$ 828 nm, $\varepsilon$=285,000 |
| 57 | 2-[2-Phenylethyl]amino analogue of Example 37 above | $^{13}C$, $^{1}H$, MS, $\lambda_{max}$ 816 nm, $\varepsilon$=276,000 |

| Example # | Formula | Characterization |
|---|---|---|
| 58 | 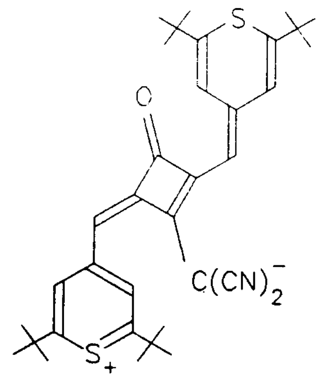 | $^{1}H$, MS, $\lambda_{max}$ 869 nm, ε=147,000 |
| 59 | 2-Phenyl analogue of Example 36 above | $^{13}C$, $^{1}H$, MS, $\lambda_{max}$ 758 nm, ε=336,000 |
| 60 | 2-Ethyl analogue of Example 36 above | $^{13}C$, $^{1}H$, MS, $\lambda_{max}$ 752 nm, ε=325,000 |

Example 61 : Effect of deprotonation on position of principal absorptions of cationic amino dyes

Near infra-red absorption spectra were measured for certain cationic dyes of this invention in a neutral solvent (approximately 3 mL), and the measurements was repeated for a solution of the dye in the same solvent with the addition of a strong organic base, DBU (approximately 10 mg). The positions of the principal infra-red absorptions are indicated in Table 1 below.

Table 1

| Dye of Example # | Solvent | $\lambda_{max}$, neutral | $\lambda_{max}$, base |
|---|---|---|---|
| 36 | dichloromethane | 797 | 854 |
| 37 | dichloromethane | 802 | 866 |
| 38 | dimethyl sulfoxide | 829 | 897 |

From the data in Table 1, it will be seen that deprotonation of the dye, presumably from the nitrogen attached to the squarate ring, causes a shift to longer wavelength of the principal infra-red absorption by about 60 nm.

Example 62 : Imaging

This Example illustrates the use of a dye of the present invention in a thermal imaging medium and process.

The thermal imaging media used in this Example was a simplified model of that described above with reference to Figure 6. A coating fluid A was prepared by combining the infra-red dye of Formula IR4 (1.8 mg) with acetone (0.73 mL), the leuco dye of Formula LD3 above (110 mg) and a polymeric binder (polyurethane Estane 5715, supplied by B. F. Goodrich, 0.73 mL of a 15% solution in acetone). Similarly, a coating fluid B was prepared by combining the infra-red dye of Formula IR7 above (1.8 mg) with dichloromethane (0.18 mL), the leuco dye of Formula LD3 above (103 mg) and

Estane 5715 (0.72 mL of the acetone solution). Also, a coating fluid C was prepared by combining the infrared dye of Formula IR6 above (1.8 mg) with dichloromethane (0.18 mL), the leuco dye of Formula LD3 above (110 mg) and Estane 5715 (0.73 mL of the acetone solution). The fluids were coated onto a 4 mil (101 μm) transparent poly(ethylene terephthalate) base using a #12 coating rod. The films thus formed were laminated at 180°F (88°C) and 60 psi (0.4 MPa) to additional sheets of 4 mil (101 μm) poly(ethylene terephthalate) which had been coated with Joncryl 138 (supplied by S.C. Johnson & Son, Inc., Racine WI 53403, United States of America) to a thickness of approximately 2 μm. The resultant imaging media (hereinafter designated Media A, B and C respectively) exhibited peak absorptions in the near infra-red at 848 nm, absorbance 0.93 (coating A); 805 nm, absorbance 1.51 (coating B); and 863.5 nm, absorbance 0.62 (coating C). Storage of samples of these structures at 60°C for 4 days resulted in losses of only 9.7%, 2.4% and 1.6% respectively, of near infra-red absorptions for Media A, B and C.

A portion of Medium A which had not been heated was exposed to infra-red radiation from a GaAlAs semiconductor diode laser emitting at 867 nm, which delivered 62 mW to the medium. The laser output was focussed to a spot approximately 33 x 3 microns in size. The medium was wrapped around a drum whose axis was perpendicular to the incident laser beam. Rotation of the drum about its axis and simultaneous translation of the drum in the direction of the axis caused the laser spot to write a helical pattern on the medium. The pitch of the helix was 33 microns, chosen so that none of the medium was left unexposed between adjacent turns of the helix. In this arrangement, the exposure received by the medium was inversely proportional to the speed of rotation of the drum (here measured as a linear writing speed at the medium surface). Table 2 below shows the relationship between writing speed and red optical density (measured using an X-Rite 310 photographic densitometer, supplied by X-Rite, Inc., Grandville, Michigan, with the appropriate filter) achieved. The unexposed medium had a red density of 0.07.

Similarly, a portion of Medium B which had not been heated was exposed to infra-red radiation from a GaAlAs semiconductor diode laser emitting at 792 nm, which delivered 151 mW to the medium. Table 2 shows the relationship between writing speed and red optical density achieved. The unexposed medium had a red density of 0.075.

Similarly, a portion of Medium C which had not been heated was exposed to infra-red radiation from a GaAlAs semiconductor diode laser emitting at 867 nm, which delivered 621 mW to the medium. Table 2 shows the relationship between writing speed and red optical density achieved. The unexposed medium had a red density of 0.08.

Table 2

| Medium A | |
|---|---|
| **Writing speed, m/s** | **Red optical density** |
| 0.14 | 0.48 |
| 0.125 | 0.62 |
| **Medium B** | |
| **Writing speed, m/s** | **Red optical density** |
| 0.43 | 0.22 |
| 0.32 | 1.54 |
| 0.25 | 2.95 |
| 0.18 | 4.08 |
| **Medium C** | |
| **Writing speed, m/s** | **Red optical density** |
| 0.14 | 0.51 |
| 0.125 | 0.75 |

From these results, it will be seen that these thermal imaging media were capable of producing images when exposed to near infra-red radiation. Medium B produced images with optical densities as high as those needed in commercial transparencies. Medium B produced images with optical densities as high as those needed in commercial transparencies.

## Claims

1. A squarylium compound of the formula:

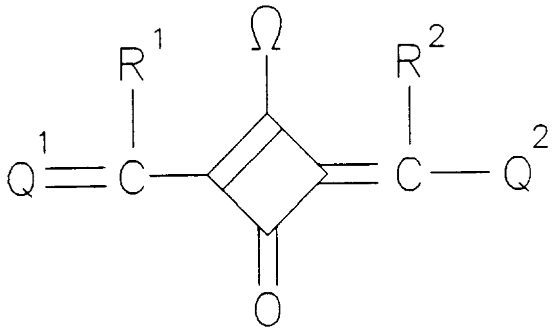

in which:

$Q^1$ and $Q^2$ are each a chromophoric group having an aromatic unsaturated system conjugated with the squarylium ring and such that in the compounds of formulae $Q^1CH_2R^1$ and $Q^2CH_2R^2$ the methylene hydrogens are active hydrogens;

$R^1$ and $R^2$ are each independently a hydrogen atom or an aliphatic or cycloaliphatic group; and

Ω is a group of the formula $-NR^3R^4$ or of the formula $-CR^5R^6R^7$, wherein:

$R^3$ and $R^4$ are each independently a hydrogen atom, or an acyl, aliphatic, cycloaliphatic, aromatic or heterocyclic group, subject to the proviso that one of $R^3$ and $R^4$ may be an amino or substituted amino group, or one of $R^3$ and $R^4$ is a hydrogen atom and the other is an organosulfonyl group, or $R^3$ and $R^4$ together with the intervening nitrogen atom form a nitrogenous heterocyclic ring; and

$R^5$, $R^6$ and $R^7$ are each independently a hydrogen atom, or an acyl, aliphatic, cycloaliphatic, aromatic or heterocyclic group, or an electron-withdrawing group able to lower the electron density at the carbon atom to which it is attached, subject to the provisoes that:

two of $R^5$, $R^6$ and $R^7$ may form a divalent group of which a single atom is double bonded to the carbon atom to which the two groups are attached, or all three of $R^5$, $R^6$ and $R^7$ may form a trivalent group of which a single atom is triple bonded to the carbon atom to which the three groups are attached, or

two of $R^5$, $R^6$ and $R^7$ may, together with the carbon atom to which they are attached, form a ring, or all three of $R^5$, $R^6$ and $R^7$ may, together with the carbon atom to which they are attached, form an unsaturated ring.

2. A squaric acid derivative of the formula:

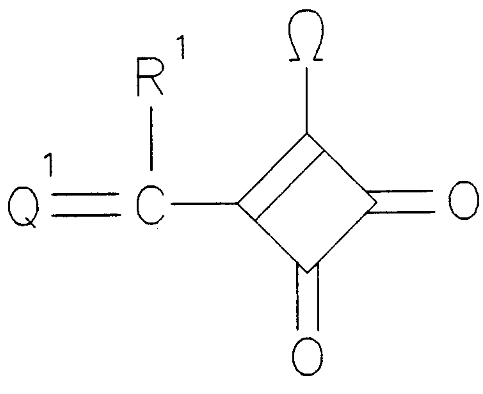

in which $Q^1$, $R^1$ and Ω are as defined in claim 1.

**3.** A compound of the formula:

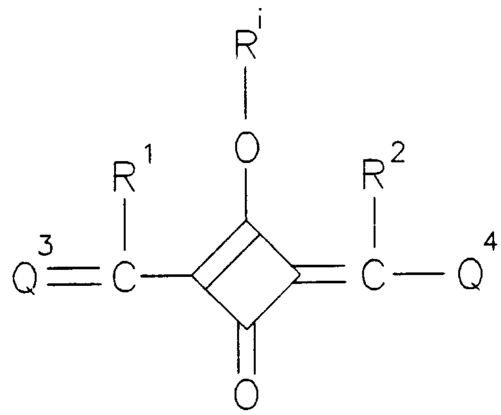

in which $R^1$ and $R^2$ are as defined in claim 1, $R^i$ is a hydrogen atom or an aliphatic or cycloaliphatic group, and $Q^3$ and $Q^4$ are each independently a pyrylium, thiopyrylium, selenopyrylium, benzpyrylium, benzthiopyrylium or benzselenopyrylium nucleus.

**4.** A compound according to claim 1 or 2 characterized in that $Q^1$, or at least one of $Q^1$ and $Q^2$, is a pyrylium, thiopyrylium, selenopyrylium, benzpyrylium, benzthiopyrylium or benzselenopyrylium nucleus.

**5.** A compound according to any one of the preceding claims characterized in that $Q^1$, or at least one of $Q^1$ and $Q^2$, or at least one of $Q^3$ and $Q^4$ is a benzpyrylium nucleus carrying at its 2-position a substituent in which a non-aromatic carbon atom is bonded directly to the benzpyrylium nucleus, subject to the proviso that if said 2-substituent contains an aromatic nucleus, this aromatic nucleus is not conjugated with the benzpyrylium nucleus.

**6.** A compound according to claim 5 characterized in that at least $Q^1$, or one of $Q^1$ and $Q^2$, or at least one of $Q^3$ and $Q^4$, carries at its 6-position an alkoxy or cycloalkoxy group.

**7.** A compound according to claim 6 characterized in that the 6-alkoxy group is a branched chain alkoxy group, preferably a propoxy, but-2-oxy or 2-ethylbutoxy group.

**8.** A compound according to claim 7 which is:

a 4-[[3-[[7-diethylamino-2-[1,1-dimethylethyl]benz[b]-4H-pyran-4-ylidene]methyl]-4-oxo-2-pivaloylamino-2-cyclobuten-1-ylidene]methyl]-7-diethylamino-2-[1,1-dimethylethyl]benz[b]pyrylium salt;

b. $Q^1$ is a 6-[but-2-oxy]-2-(1,1-dimethylethyl)benz[b]-4H-thiopyran-4-ylidene grouping, $Q^2$ is a 4-[2-(1,1-dimethylethyl)-6-[but-2-oxy]-benz[b]-4H-thiopyrylium] grouping, $R^1$, $R^2$ and $R^4$ are each a hydrogen atom, and $R^3$ is a propyl group, namely, a 6-[but-2-oxy]-4-[[3-[[6-[but-2-oxy]-2-[1,1-dimethylethyl]benz[b]-4H-thiopyran-4-ylidene]-methyl]-4-oxo-2-[propylamino]-2-cyclobuten-l-ylidene]methyl]-2-[1,1-dimethylethyl]-benz[b]thiopyrylium salt; and

c. $Q^1$ is a 6-[but-2-oxy]-2-(1,1-dimethylethyl)benz[b]-4H-thiopyran-4-ylidene grouping, $Q^2$ is a 4-[6-[but-2-oxy]-2-(1,1-dimethylethyl)benz[b]-4H-pyrylium] grouping, $R^1$, $R^2$ and $R^4$ are each a hydrogen atom, and $R^3$ is a propyl group, namely a 6-[but-2-oxy]-4-[[2-[propylamino]-3-[[6-[but-2-oxy]2-[1,1-dimethylethyl]-benz[b]-4H-thiopyran-4-ylidene]methyl]-4-oxo-2-cyclobuten-1-ylidene]methyl]-2-[1,1-dimethylethyl]benz[b]pyrylium salt.

**9.** A process for the preparation of a squaric acid derivative of the formula:

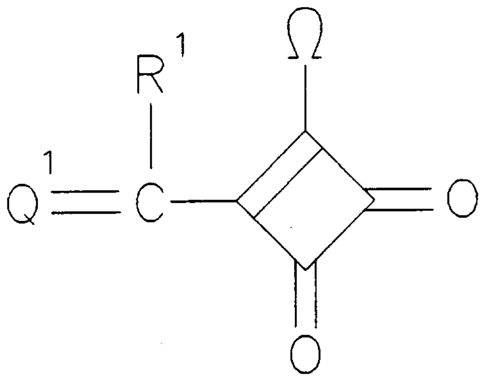

in which $Q^1$, $R^1$ and $\Omega$ are as defined in claim 1, which process comprises reacting a corresponding squaric acid derivative of the formula:

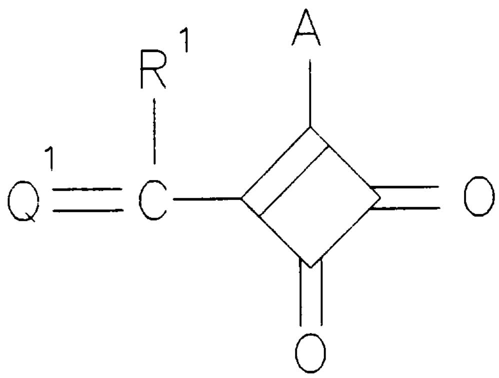

in which A is a chlorine or bromine atom, or a hydroxyl or alkoxyl group, and $Q^1$ and $R^1$ are as defined above, with a compound of formula $H\Omega$, in which $\Omega$ is as defined above.

**10.** A process for the preparation of a squarylium compound of the formula:

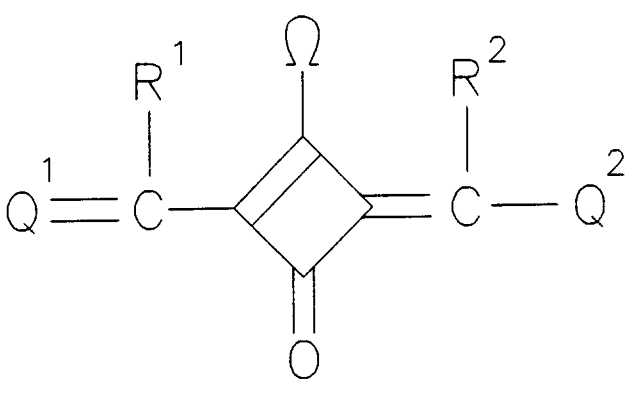

in which $Q^1$, $Q^2$, $R^1$, $R^2$ and $\Omega$ are as defined in claim 1, which process comprises reacting a corresponding squaric acid derivative of the formula:

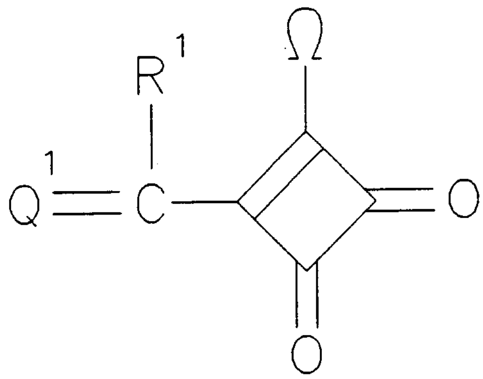

in which $Q^1$, $R^1$ and $\Omega$ are as defined above, with a compound of the formula $Q^2CH_2R^2$, in which $Q^2$ and $R^2$ are as defined above.

11. A process for the preparation of a squarylium compound of the formula:

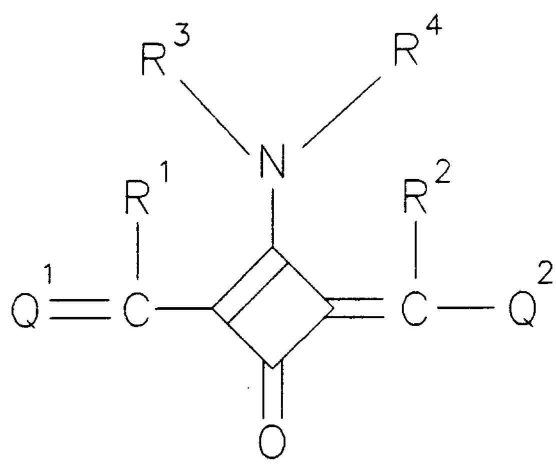

in which $Q^1$, $Q^2$, $R^1$ and $R^2$ are as defined in claim 1, and one of $R^3$ and $R^4$ is a hydrogen atom and the other is an organosulfonyl group, which process comprises reacting the corresponding squarylium compound of the formula:

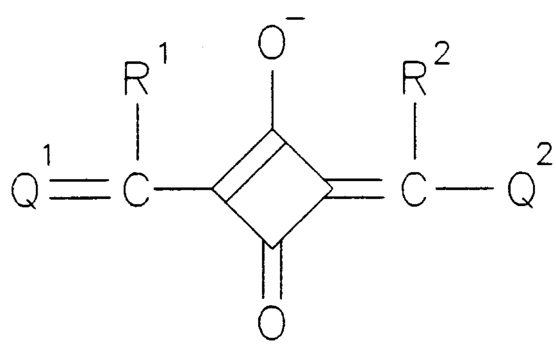

in which $Q^1$, $Q^2$, $R^1$ and $R^2$ are as defined above, with the corresponding orgaosulfonyl isocyanate.

12. A process for the preparation of a squarylium compound of the formula:

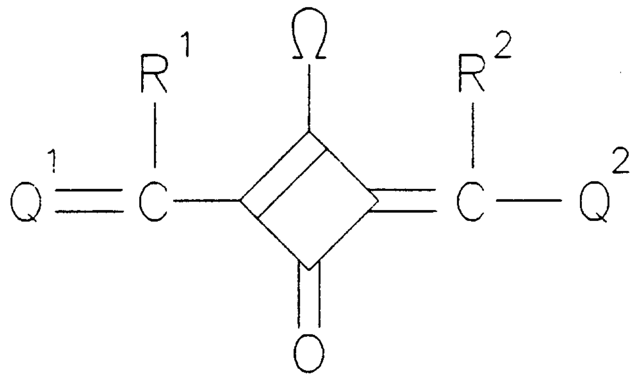

in which $Q^1$, $Q^2$, $R^1$, $R^2$ and Ω are as defined in claim 1, which process comprises reacting a corresponding Ω-substituted squaric acid monoester with at least one compound of formula $Q^1CH_2R^1$ or $Q^2CH_2R^2$.

13. A process for the preparation of a squaric acid derivative of the formula:

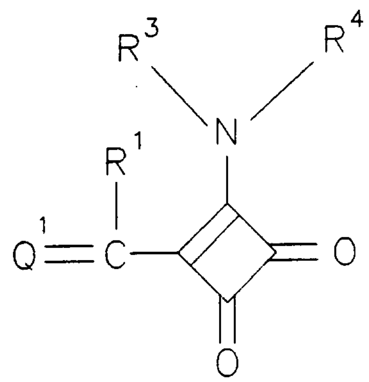

in which $Q^1$, $R^1$, $R^3$ and $R^4$ are as defined in claim 1, which process comprises reacting a corresponding squaric acid monoester monoamide with a compound of formula $Q^1$=$CHR^1$.

14. A process for the preparation of a 1,3-disubstituted-2-amino or substituted amino squarylium dye, which process comprises reacting the corresponding 1,3-disubstituted-2-unsubstituted squarylium dye with an alkylating agent to produce a corresponding 1,3-disubstituted-2-alkoxy squarylium compound, ad thereafter reacting the 2-alkoxy compound with ammonia or a primary or secondary amine to produce the 1,3-disubstituted-2-amino or substituted amino squarylium dye.

15. A process for generating heat in a medium comprising a dye according to claim 1, which process comprises exposing at least part of the medium to infra-red actinic radiation of a frequency absorbed by the dye, whereby the radiation is absorbed by the dye and heat is generated within the parts of the medium exposed to the radiation.

16. A process according to claim 15 wherein the medium further comprises a thermally sensitive material capable of undergoing a color change upon exposure to heat, the medium is exposed imagewise to the radiation, and the heat generated by the dye is sufficient to effect a color change in the thermally sensitive material, whereby an image is formed in the medium.

17. A thermal imaging medium comprising at least one imaging layer, the imaging layer comprising a color-forming compound which undergoes a change of color upon heating above a color-forming temperature for a color-forming time, the imaging layer further comprising a dye according to claim 1.

## Patentansprüche

1. Squaryliumverbindung der Formel:

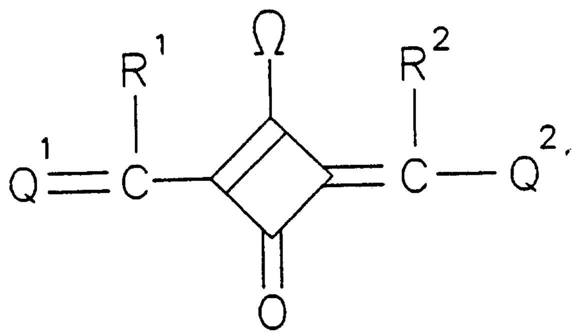

worin bedeuten:
$Q^1$ und $Q^2$ bedeuten jeweils eine chromophore Gruppe mit einem aromatischen ungesättigten System, das mit dem Squaryliumring konjugiert ist, wobei in den Verbindungen der Formeln $Q^1CH_2R^1$ und $Q^2CH_2R^2$ die Methylen-Wasserstoffatome aktive Wasserstoffatome darstellen;
$R^1$ und $R^2$ bedeuten jeweils unabhängig voneinander ein Wasserstoffatom oder eine aliphatische oder cycloaliphatische Gruppe; und
$\Omega$ bedeutet eine Gruppe der Formel $-NR^3R^4$ oder der Formel $-CR^5R^6R^7$, worin bedeuten:
$R^3$und $R^4$ bedeuten jeweils unabhängig voneinander ein Wasserstoffatom oder eine Acyl-, eine aliphatische, cycloaliphatische, aromatische oder heterocyclische Gruppe, mit der Maßgabe, daß eines von $R^3$ und $R^4$ eine Amino- oder substituierte Aminogruppe sein kann, oder eines von $R^3$ und $R^4$ ein Wasserstoffatom und das andere eine Organosulfonylgruppe darstellt, oder $R^3$ und $R^4$ zusammen mit dem dazwischenliegenden Stickstoffatom einen stickstoffhaltigen heterocyclischen Ring bilden; und
$R^5$, $R^6$ und $R^7$ bedeuten jeweils unabhängig voneinander ein Wasserstoffatom oder eine Acyl-, eine aliphatische, cycloaliphatische, aromatische oder heterocyclische Gruppe oder eine elektronenziehende Gruppe, die in der Lage ist, die Elektronendichte am Kohlenstoffatom, an das sie gebunden ist, zu erniedrigen, mit der Maßgabe, daß: zwei von $R^5$, $R^6$ und $R^7$ eine zweiwertige Gruppe bilden können, von der ein einziges Atom doppelt an das Kohlenstoffatom gebunden ist, an das die beiden Gruppen gebunden sind, oder alle drei $R^5$, $R^6$ und $R^7$ können eine dreiwertige Gruppe bilden, von der ein einziges Atom dreifach an das Kohlenstoffatom gebunden ist, an das die drei Gruppen gebunden sind, oder
zwei von $R^5$, $R^6$ und $R^7$ können zusammen mit dem Kohlenstoffatom, an das sie gebunden sind, einen Ring bilden, oder alle drei $R^5$, $R^6$ und $R^7$ können zusammen mit dem Kohlenstoffatom, an das sie gebunden sind, einen ungesättigten Ring bilden.

2. Squarylsäurederivat der Formel:

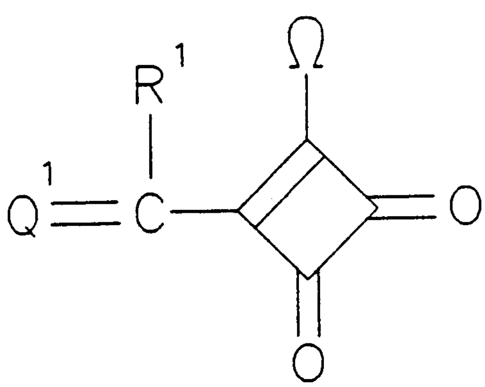

worin $Q^1$, $R^1$ und $\Omega$ wie in Anspruch 1 definiert sind.

3. Verbindung der Formel:

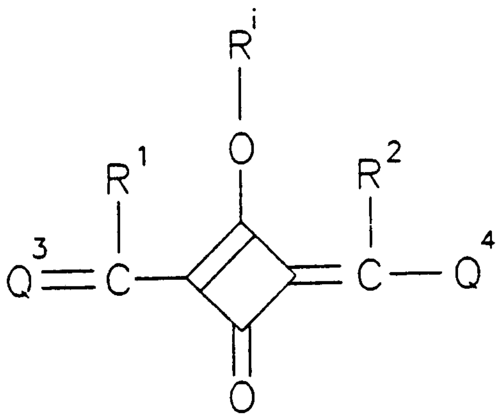

worin $R^1$ und $R^2$ wie in Anspruch 1 definiert sind, $R^i$ ein Wasserstoffatom oder eine aliphatische oder cycloaliphatische Gruppe darstellt und $Q^3$ und $Q^4$ jeweils unabhängig voneinander einen Pyrylium-, Thiopyrylium-, Selenopyrylium-, Benzpyrylium-, Benzthiopyrylium- oder Benzselenopyrylium-Kern darstellen.

4. Verbindung nach Anspruch 1 oder 2, dadurch gekennzeichnet, daß $Q^1$ oder mindestens eines von $Q^1$ und $Q^2$ einen Pyrylium-, Thiopyrylium-, Selenopyrylium-, Benzpyrylium-, Benzthiopyrylium- oder Benzselenopyrylium-Kern darstellen.

5. Verbindung nach einem der vorhergehenden Ansprüche, dadurch gekennzeichnet, daß $Q^1$ oder mindestens eines von $Q^1$ oder $Q^2$ oder mindestens eines von $Q^3$ und $Q^4$ einen Benzpyrylium-Kern darstellen, der in seiner 2-Stellung einen Substituenten trägt, in welchem ein nicht-aromatisches Kohlenstoffatom direkt an den Benzpyryliumkern gebunden ist, mit der Maßgabe, daß, wenn der 2-Substituent einen aromatischen Kern enthält, dieser aromatische Kern nicht mit dem Benzpyryliumkern konjugiert ist.

6. Verbindung nach Anspruch 5, dadurch gekennzeichnet, daß mindestens ein $Q^1$ oder eines von $Q^1$ und $Q^2$ oder mindestens eines von $Q^3$ und $Q^4$ in seiner 6-Stellung eine Alkoxy- oder Cycloalkoxy-Gruppe trägt.

7. Verbindung nach Anspruch 6, dadurch gekennzeichnet, daß die 6-Alkoxy-Gruppe eine verzweigtkettige Alkoxygruppe, vorzugsweise eine Propoxy-, But-2-oxy oder 2-Ethylbutoxy-Gruppe darstellt.

8. Verbindung nach Anspruch 7, welche ein:

   a. 4-[[3-[[7-Diethylamino-2-[1,1-dimethyl]benz[b]-4H-pyran-4-yliden]methyl]-4-oxo-2-pivaloylamino-2-cyclobuten-1-yliden]methyl]-7-diethylamino-2-[1,1-dimethylethyl]benz[b]pyryliumsalz darstellt;

   b. $Q^1$ eine 6-[But-2-oxy]-2-(1,1-dimethylethyl)benz[b]-4H-thiopyran-4-yliden-Gruppierung, $Q^2$ eine 4-[2-(1,1-Dimethylethyl)-6-[but-2-oxy]-benz[b]-4H-thiopyrylium]-Gruppierung, $R^1$, $R^2$ und $R^4$ jeweils ein Wasserstoffatom und $R^3$ eine Propylgruppe, nämlich ein 6-[But-2-oxy]-4-[[3-[[6-[but-2-oxy]-2-[1,1-dimethylethyl]benz[b]-4H-thiopyran-4-yliden[-methyl]-4-Oxo-2-[propylamino]-2-cyclobuten-1-yliden]methyl]-2-[1,1-dimethylethyl]-benz[b]thiopyryliumsalz darstellen; und

   c. $Q^1$ eine 6-[But-2-oxy]-2-(1,1-dimethylethyl)benz[b]-4H-thiopyran-4-yliden-Gruppierung, $Q^2$ eine 4-[6-[But-2-oxy]-2-(1,1-dimethylethyl)benz[b]-4H-pyrylium]-Gruppierung, $R^1$, $R^2$ und $R^4$ jeweils ein Wasserstoffatom und $R^3$ eine Propyl-Gruppe, nämlich ein 6-[But-2-oxy]-4-[[2-[propylamino]-3-[[6-[but-2-oxy]2-[1,1-dimethylethyl]-benz[b]-4H-thiopyran-4-yliden]methyl]-4-oxo-2-cyclobuten-1-yliden]methyl]-2-[1,1-dimethylethyl]benz[b]pyryliumsalz darstellen.

9. Verfahren zur Herstellung eines Squarylsäurederivats der Formel:

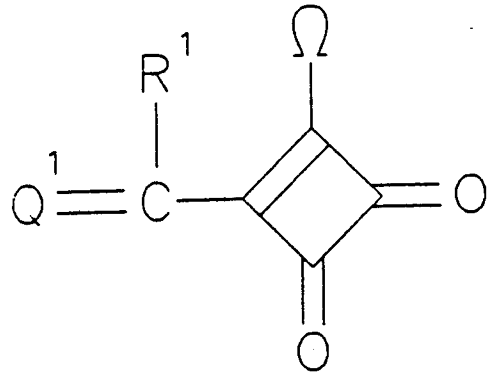

worin $Q^1$, $R^1$ und $\Omega$ wie in Anspruch 1 definiert sind, welches die Umsetzung eines entsprechenden Squarylsäurederivats der Formel:

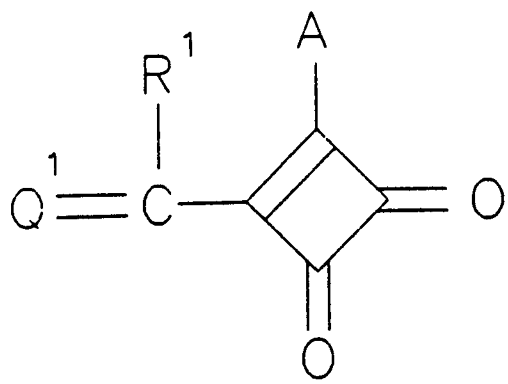

worin A ein Chlor- oder Bromatom oder eine Hydroxyl- oder Alkoxylgruppe darstellt, und $Q^1$ und $R^1$ wie vorstehend definiert sind, mit einer Verbindung der Formel $H\Omega$, worin $\Omega$ wie vorstehend definiert ist, umfaßt.

10. Verfahren zur Herstellung einer Squaryliumverbindung der Formel:

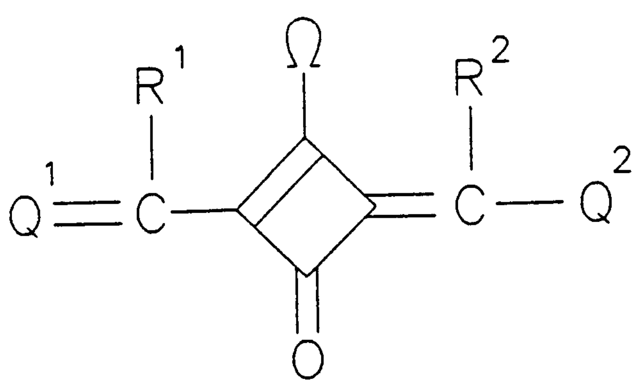

worin $Q^1$, $Q^2$, $R^1$ $R^2$ und $\Omega$ wie in Anspruch 1 definiert sind, welches die Umsetzung eines entsprechenden Squarylsäurederivats der Formel:

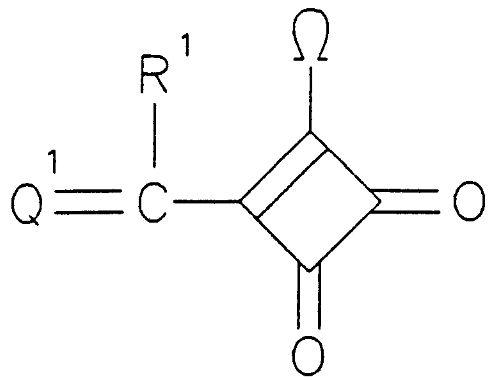

worin $Q^1$, $R^1$ und Ω wie vorstehend definiert sind, mit einer Verbindung der Formel $Q^2CH_2R^2$, worin $Q^2$ und $R^2$ wie vorstehend definiert sind, umfaßt.

**11.** Verfahren zur Herstellung einer Squaryliumverbindung der Formel:

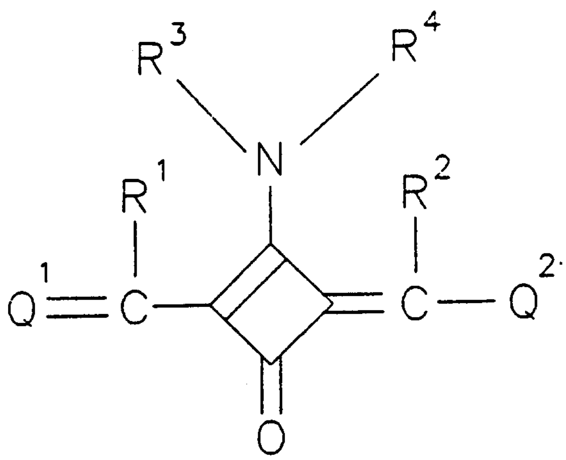

worin $Q^1$, $Q^2$, $R^1$ und $R^2$ wie in Anspruch 1 definiert sind und eines von $R^3$ und $R^4$ ein Wasserstoffatom und das andere eine Organosulfonylgruppe darstellen, welches die Umsetzung der entsprechenden Squaryliumverbindung der Formel

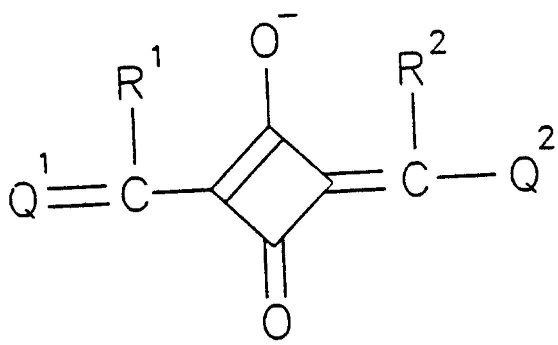

worin $Q^1$, $Q^2$, $R^1$ und $R^2$ wie vorstehend definiert sind, mit den entsprechenden Organosulfonyl-Isocyanat, umfaßt.

**12.** Verfahren zur Herstellung einer Squaryliumverbindung der Formel:

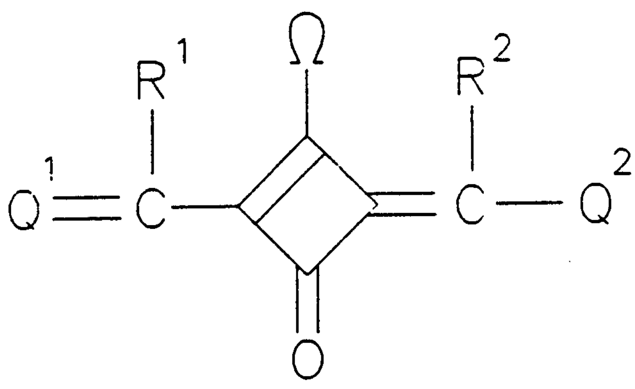

worin $Q^1$, $Q^2$, $R^1$, $R^2$ und $\Omega$ wie in Anspruch 1 definiert sind, welches die Umsetzung eines entsprechenden $\Omega$-substituierten Squarylsäure-Monoesters mit mindestens einer Verbindung der Formel $Q^1CH_2R^1$ oder $Q^2CH_2R^2$ umfaßt.

**13.** Verfahren zur Herstellung eines Squarylsäurederivats der Formel:

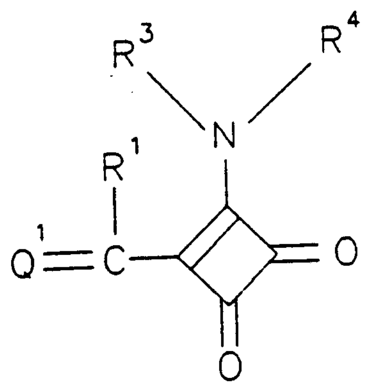

worin $Q^1$, $R^1$, $R^3$ und $R^4$ wie in Anspruch 1 definiert sind, welches die Umsetzung eines entsprechenden Squarylsäure-Monoester-Monoamids mit einer Verbindung der Formel $Q^1$=$CHR^1$ umfaßt.

**14.** Verfahren zur Herstellung eines 1,3-disubstituierten-2-Amino- oder substituierten Amino-Squaryliumfarbstoffs, welches die Umsetzung des entsprechenden 1,3-disubstituierten-2-unsubstituierten Squaryliumfarbstoffs mit einem Alkylierungsmittel und der Bildung einer entsprechenden 1,3-disubstituierten-2-Alkoxy-Squaryliumverbindung und die anschließende Umsetzung der 2-Alkoxy-Verbindung mit Ammoniak oder einem primären oder sekundären Amin unter Bildung des 1,3-disubstituierten-2-Amino oder substituierten Amino-Squaryliumfarbstoffs umfaßt.

**15.** Verfahren zur Erzeugung von Wärme in einem Medium, enthaltend einen Farbstoff nach Anspruch 1, wobei mindestens ein Teil des Mediums einer aktinischen Infrarotstrahlung mit einer Frequenz, die von dem Farbstoff absorbiert wird, ausgesetzt wird, wodurch die Strahlung von dem Farbstoff absorbiert und Wärme in den Teilen des Mediums, die der Strahlung ausgesetzt waren, erzeugt wird.

**16.** Verfahren nach Anspruch 15, wobei das Medium weiterhin ein thermisch empfindliches Material enthält, das bei der Erwärmung eine Farbänderung erleidet, wobei das Medium der Strahlung bildmäßig ausgesetzt wird und die durch den Farbstoff erzeugte Wärme ausreichend ist, um eine Farbänderung in dem thermisch empfindlichen Material zu erzeugen, wodurch in dem Medium ein Bild erzeugt wird.

**17.** Thermisches Bilderzeugungsmedium, enthaltend mindestens eine Bilderzeugungsschicht, wobei die Bilderzeugungsschicht eine farbbildende Verbindung enthält, die beim Erwärmen oberhalb einer Farbbildungstemperatur

und über eine Farbbildungszeit eine Farbänderung erleidet, wobei die Bilderzeugungsschicht weiterhin einen Farbstoff nach Anspruch 1 enthält.

## Revendications

1. Composé de type squarylium, répondant à la formule :

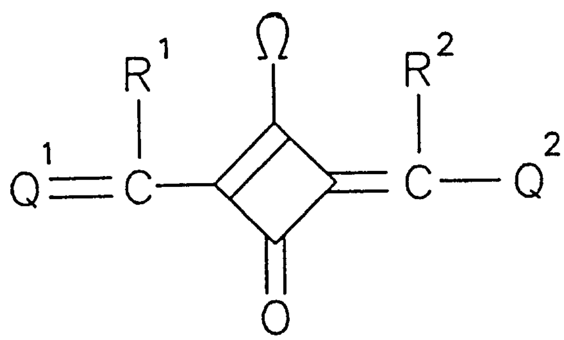

dans laquelle :

$Q^1$ et $Q^2$ représentent chacun un groupe chromophore possédant un système insaturé aromatique, conjugué avec le cycle squarylium, et tel que, dans les composés de formules $Q^1CH_2R^1$ et $Q^2CH_2R^2$, les atomes d'hydrogène des groupes méthylène sont des atomes d'hydrogène actif,

$R^1$ et $R^2$ représentent chacun, indépendamment, un atome d'hydrogène ou un groupe aliphatique ou cycloaliphatique, et

Ω représente un groupe de formule $-NR^3R^4$ ou de formule $-CR^5R^6R^7$, formules dans lesquelles :

$R^3$ et $R^4$ représentent chacun, indépendamment, un atome d'hydrogène, ou un groupe acyle, aliphatique, cycloaliphatique, aromatique ou hétérocyclique, étant entendu que l'un de $R^3$ et $R^4$ peut être un groupe amino ou amino substitué, ou bien l'un de $R^3$ et $R^4$ représente un atome d'hydrogène et l'autre représente un groupe organosulfonyle, ou bien encore $R^3$ et $R^4$ forment ensemble avec l'atome d'azote intervenant un cycle hétérocyclique azoté, et

$R^5$, $R^6$ et $R^7$ représentent chacun, indépendamment, un atome d'hydrogène, un groupe acyle, aliphatique, cycloaliphatique, aromatique ou hétérocyclique, ou un groupe attracteur d'électrons, capable de diminuer la densité électronique autour de l'atome de carbone auquel il est attaché, étant entendu que deux de $R^5$, $R^6$ et $R^7$ peuvent former un groupe divalent dont un seul atome est doublement lié à l'atome de carbone auquel les deux groupes sont attachés, ou bien $R^5$, $R^6$ et $R^7$ peuvent former tous les trois un groupe trivalent dont un seul atome est triplement lié à l'atome de carbone auquel les trois groupes sont attachés, ou bien encore deux de $R^5$, $R^6$ et $R^7$ peuvent former un cycle avec l'atome de carbone auquel ils sont attachés, ou bien encore $R^5$, $R^6$ et $R^7$ peuvent former tous les trois, avec l'atome de carbone auquel ils sont attachés, un cycle insaturé.

**2.** Dérivé de l'acide squarique de formule :

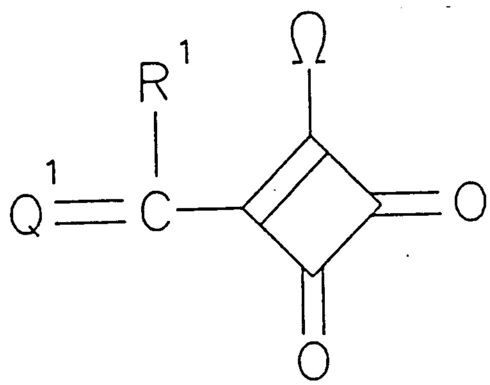

dans laquelle $Q^1$, $R^1$ et Ω sont tels que définis à la revendication 1.

**3.** Composé de formule :

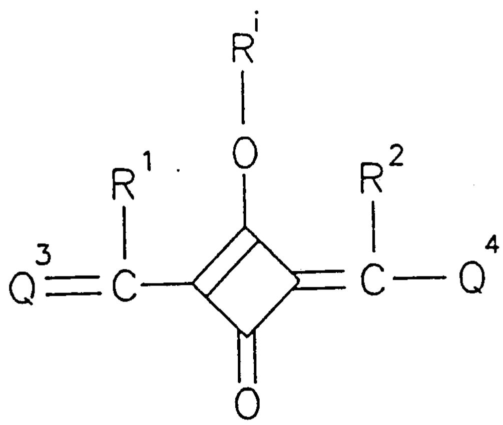

dans laquelle $R^1$ et $R^2$ sont tels que définis à la revendication 1, $R^i$ représente un atome d'hydrogène ou un groupe aliphatique ou cycloaliphatique, et $Q^3$ et $Q^4$ représentent chacun, indépendamment, un noyau pyrylium, thiopyrylium, sélénopyrylium, benzopyrylium, benzothiopyrylium ou benzosélénopyrylium.

**4.** Composé selon la revendication 1 ou 2, caractérisé en ce que $Q^1$ ou au moins l'un de $Q^1$ et $Q^2$ représente un noyau pyrylium, thiopyrylium, sélénopyrylium, benzopyrylium, benzothiopyrylium ou benzosélénopyrylium.

**5.** Composé selon l'une quelconque des revendications précédentes, caractérisé en ce que $Q^1$ ou au moins l'un de $Q^1$ et $Q^2$ ou au moins l'un de $Q^3$ et $Q^4$ représente un noyau benzopyrylium portant en position 2 un substituant dont un atome de carbone non-aromatique est lié directement au noyau benzopyrylium, étant entendu que, si ledit substituant en position 2 contient un noyau aromatique, ce noyau aromatique n'est pas conjugué avec le noyau benzopyrylium.

**6.** Composé selon la revendication 5, caractérisé en ce que $Q^1$ ou au moins l'un de $Q^1$ et $Q^2$ ou au moins l'un de $Q^3$ et $Q^4$ porte en position 6 un groupe alcoxy ou cycloalcoxy.

**7.** Composé selon la revendication 6, caractérisé en ce que le groupe alcoxy en position 6 est un groupe alcoxy à chaîne ramifiée, de préférence un groupe propoxy, but-2-oxy ou 2-éthylbutoxy.

**8.** Composé selon la revendication 7, qui est :

a) un sel de 4-[[3-[[7-diéthylamino-2-[1,1-diméthyléthyl]benzo [b]-4H-pyran-4-ylidène]méthyl]-4-oxo-2-pivaloylamino-2-cyclobutèn-1-ylidène]méthyl]-7-diéthylamino-2-[1,1-diméthyléthyl]benzo[b] pyrylium;

b) un sel de 6-[but-2-oxy)-4-[[3-[[6-[but-2-oxy]-2-[1,1-diméthyléthyl]benzo[b]-4H-thiopyran-4-ylidène]-méthyl]-4-oxo-2-[propylamino]-2-cyclobutèn-1-ylidène]méthyl]-2-[1,1-diméthyléthyl]-benzo [b]thiopyrylium, $Q^1$ représentant un groupement 6-[but-2-oxy]-2-(1,1-diméthyléthyl)benzo[b]-4H-thiopyran-4-ylidène, $Q^2$ représentant un groupement 4-[2-(1,1-diméthyléthyl)-6-[but-2-oxy]-benzo[b]-4H-thiopyrylium, $R^1$, $R^2$ et $R^4$ représentant chacun un atome d'hydrogène et $R^3$ représentant un groupe propyle; ou

c) un sel de 6-[but-2-oxy]-4-[[2-[propylamino]-3-[[6-[but-2-oxy] 2-[1,1-diméthyléthyl]-benzo[b]-4H-thiopyran-4-ylidène]méthyl]-4-oxo-2-cyclobutèn-1-ylidène]méthyl]-2-[1,1- diméthyléthyl]benzo[b] pyrylium, $Q^1$ représentant un groupement 6-[but-2-oxy]-2-(1,1-diméthyléthyl)benzo[b]-4H-thiopyran-4-ylidène, $Q^2$ représentant un groupement 4-[6-[but-2-oxy]-2-(1,1-diméthyléthyl)benzo[b]-4H-pyrylium, $R^1$, $R^2$ et $R^4$ représentant chacun un atome d'hydrogène et $R^3$ représentant un groupe propyle.

**9.** Procédé de préparation d'un dérivé de l'acide squarique de formule :

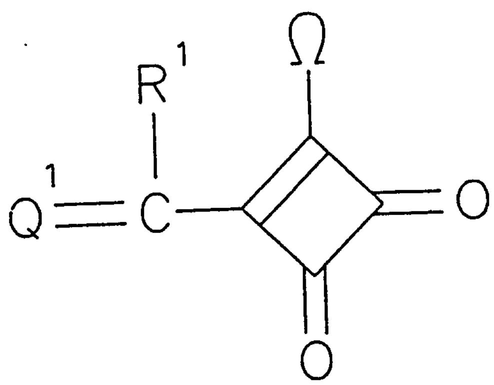

dans laquelle $Q^1$, $R^1$ et $\Omega$ sont tels que définis dans la revendication 1, qui comprend la réaction du dérivé correspondant de l'acide squarique de formule :

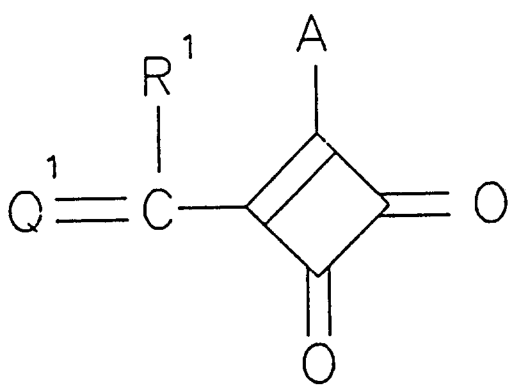

dans laquelle A représente un atome de chlore ou de brome, ou un groupe hydroxyle ou alcoxyle, et $Q^1$ et $R^1$ sont tels que définis précédemment, avec un composé de formule $H\Omega$, dans laquelle $\Omega$ est tel que défini précédemment.

**10.** Procédé de préparation d'un composé squarylium de formule :

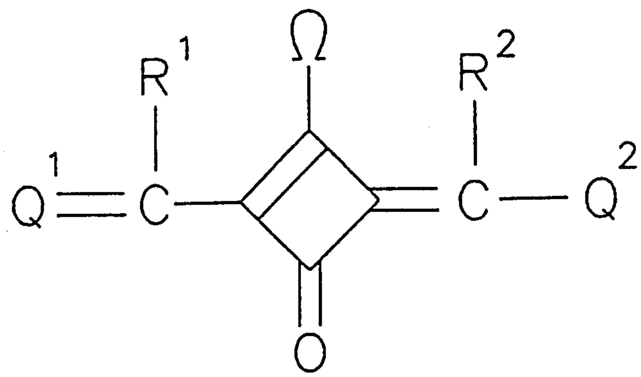

dans laquelle $Q^1$, $Q^2$, $R^1$, $R^2$ et $\Omega$ sont tels que définis dans la revendication 1, qui comprend la réaction du dérivé correspondant de l'acide squarique de formule :

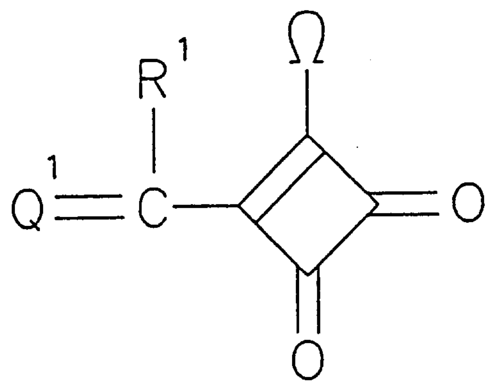

dans laquelle $Q^1$, $R^1$ et $\Omega$ sont tels que définis précédemment, avec un composé de formule $Q^2CH_2R^2$, dans laquelle $Q^2$ et $R^2$ sont tels que définis précédemment.

**11.** Procédé de préparation d'un composé squarylium de formule :

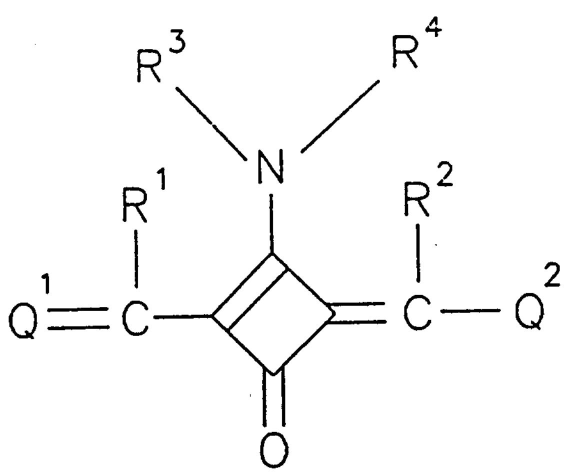

dans laquelle $Q^1$, $Q^2$, $R^1$ et $R^2$ sont tels que définis dans la revendication 1, l'un de $R^3$ et $R^4$ représente un atome d'hydrogène et l'autre représente un groupe organosulfonyle, qui comprend la réaction du composé squarylium correspondant de formule :

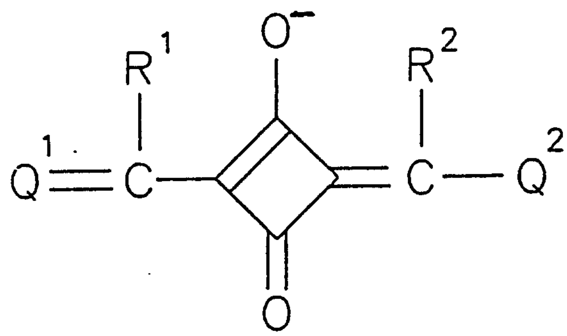

dans laquelle $Q^1$, $Q^2$, $R^1$ et $R^2$ sont tels que définis précédemment, avec l'isocyanate d'organosulfonyle correspondant.

**12.** Procédé de préparation d'un composé squarylium de formule :

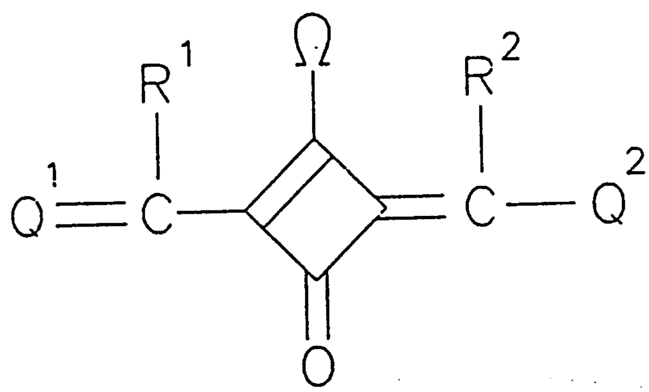

dans laquelle $Q^1$, $Q^2$, $R^1$, $R^2$ et Ω sont tels que définis dans la revendication 1, qui comprend la réaction du monoester correspondant de l'acide squarique substitué par Ω, avec au moins un composé de formule $Q^1CH_2R^1$ ou $Q^2CH_2R^2$.

**13.** Procédé de préparation d'un dérivé de l'acide squarique de formule :

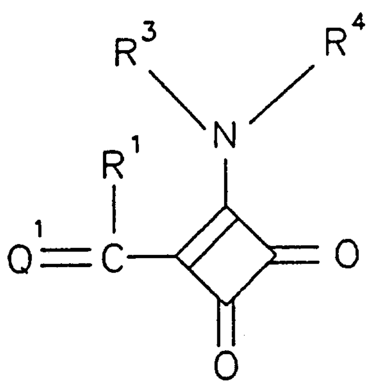

dans laquelle $Q^1$, $R^1$, $R^3$ et $R^4$ sont tels que définis dans la revendication 1, qui comprend la réaction du monoamide monoester correspondant de l'acide squarique, avec un composé de formule $Q^1$=$CHR^1$.

**14.** Procédé de préparation d'un colorant squarylium disubstitué en positions 1 et 3 et substitué en position 2 par un groupe amino ou amino substitué, qui comprend la réaction du colorant squarylium 1,3-disubstitué, non substitué en position 2, correspondant, avec un agent d'alkylation pour la production du composé 1,3-disubstitué-2-alcoxy

squarylium correspondant, puis la réaction du composé 2-alcoxy avec de l'ammoniac ou une amine primaire ou secondaire, pour la production du colorant squarylium disubstitué en positions 1 et 3 et substitué en position 2 par un groupe amino ou amino substitué.

**15.** Procédé pour produire de la chaleur dans un milieu comprenant un colorant selon la revendication 1, qui comprend l'exposition d'au moins une partie du milieu à des rayons actiniques infrarouges dont la fréquence est telle qu'ils sont absorbés par le colorant, les rayons étant ainsi absorbés par le colorant et de la chaleur étant ainsi produite dans les parties du milieu exposées aux rayons.

**16.** Procédé selon la revendication 15, dans lequel le milieu renferme en outre une matière sensible à la chaleur, capable de subir une changement de couleur par exposition à la chaleur, le milieu est exposé aux rayons selon l'image, et la chaleur produite par le colorant est suffisante pour que s'effectue un changement de couleur de la matière sensible à la chaleur, ce qui provoque la formation d'une image dans le milieu.

**17.** Milieu pour la formation thermique d'image, comprenant au moins une couche formatrice d'image, la couche formatrice d'image renfermant un composé formateur de couleur, qui subit un changement de couleur par chauffage à une température supérieure à la température formatrice de couleur, pendant une durée permettant la formation de la couleur, la couche formatrice d'image renfermant en outre un colorant selon la revendication 1.

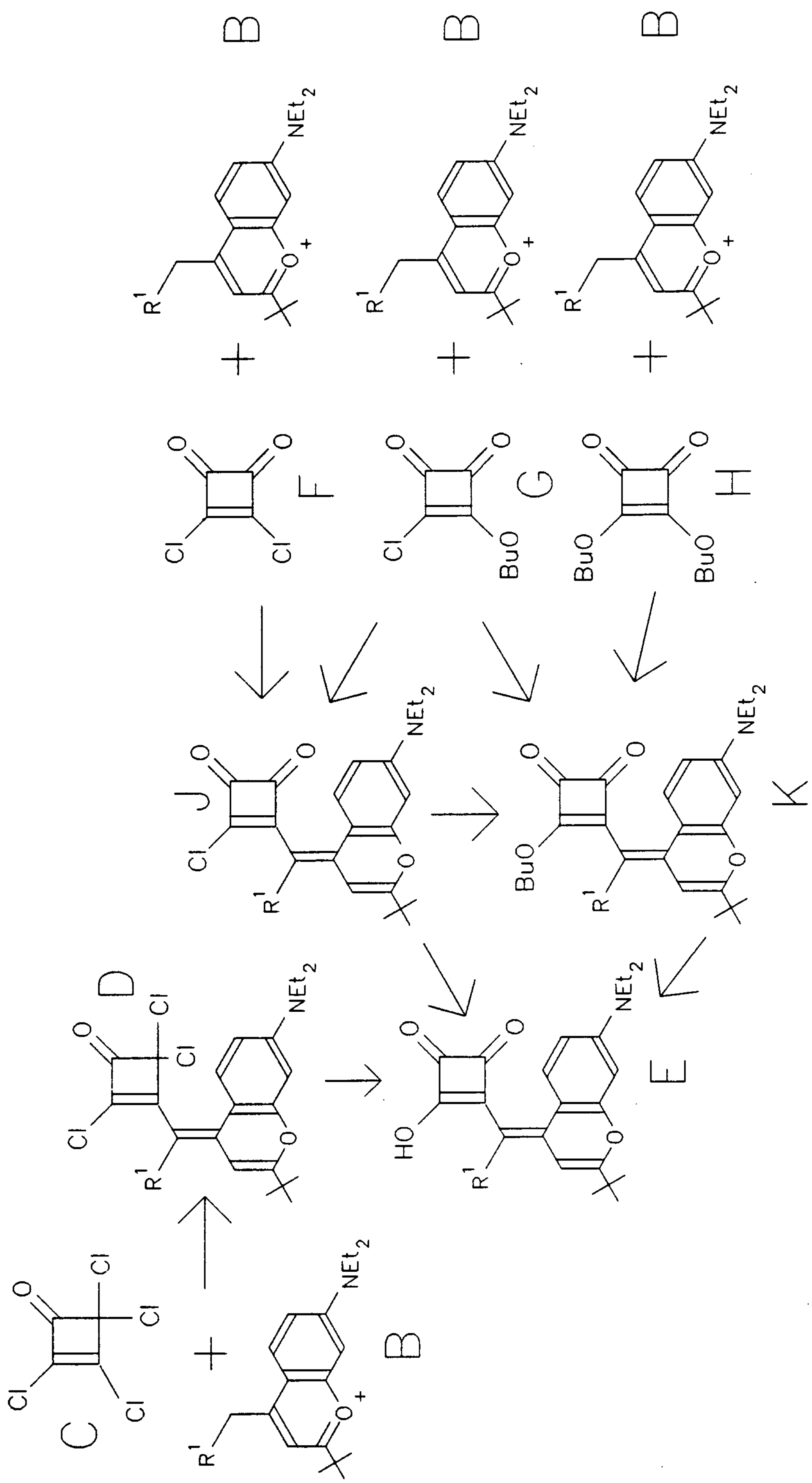

B B B F G H J K C D E B

Figure 1

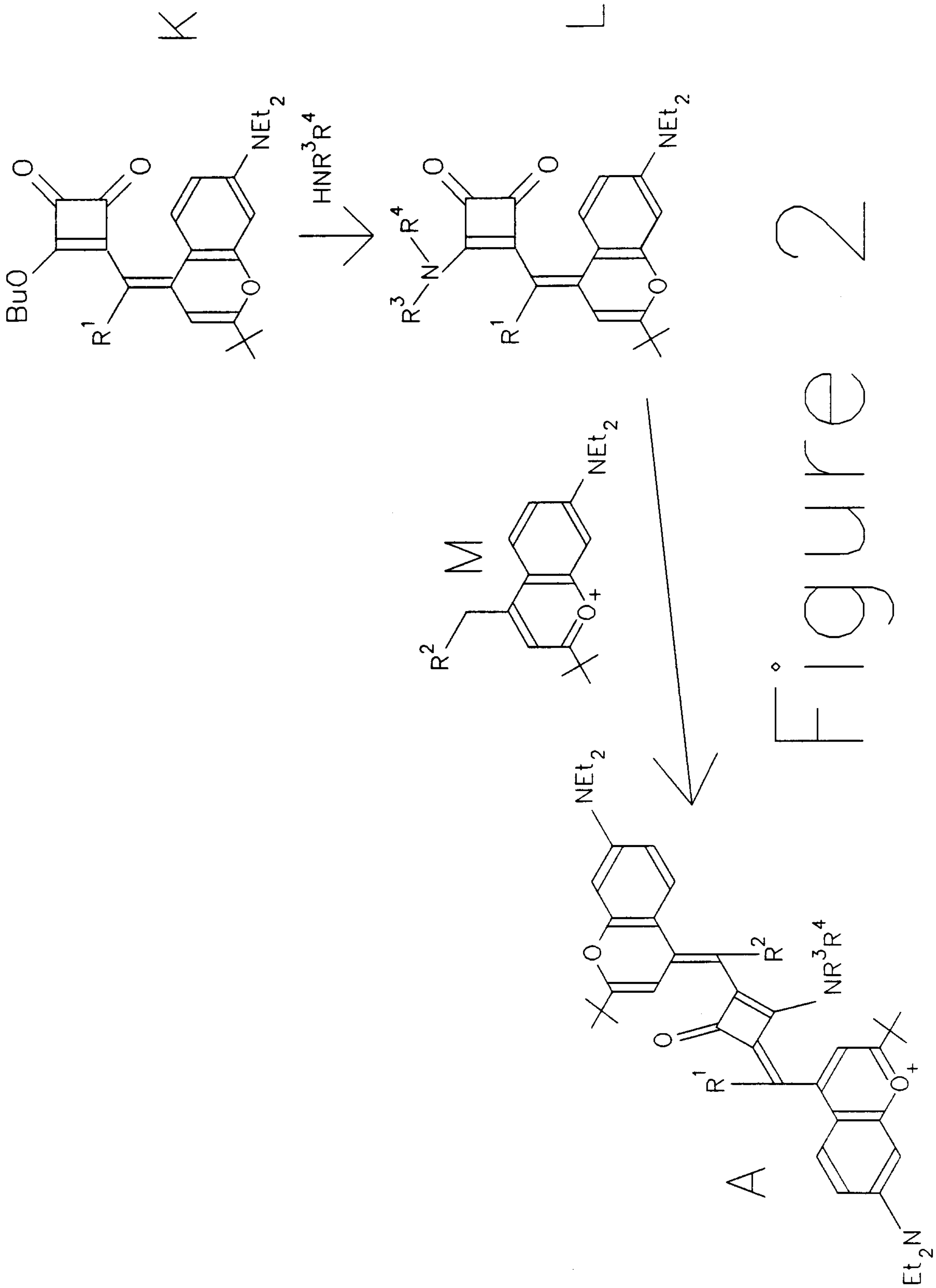


Figure 2

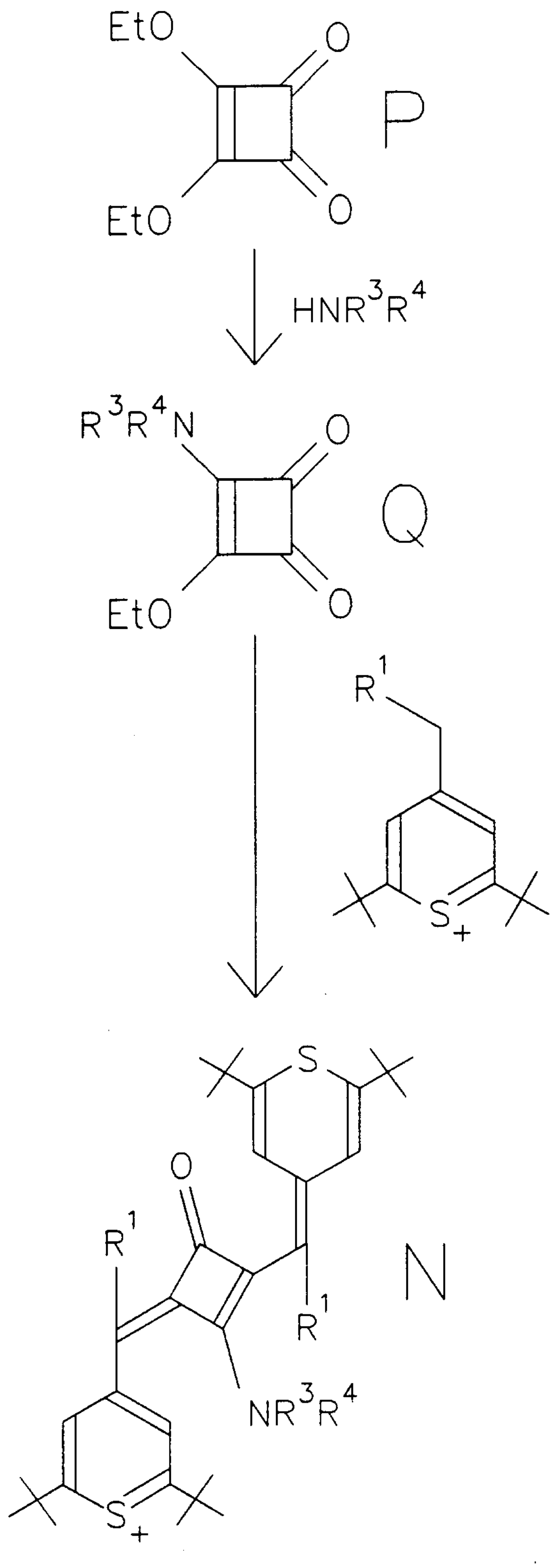


P

Q

R

N

Figure 3

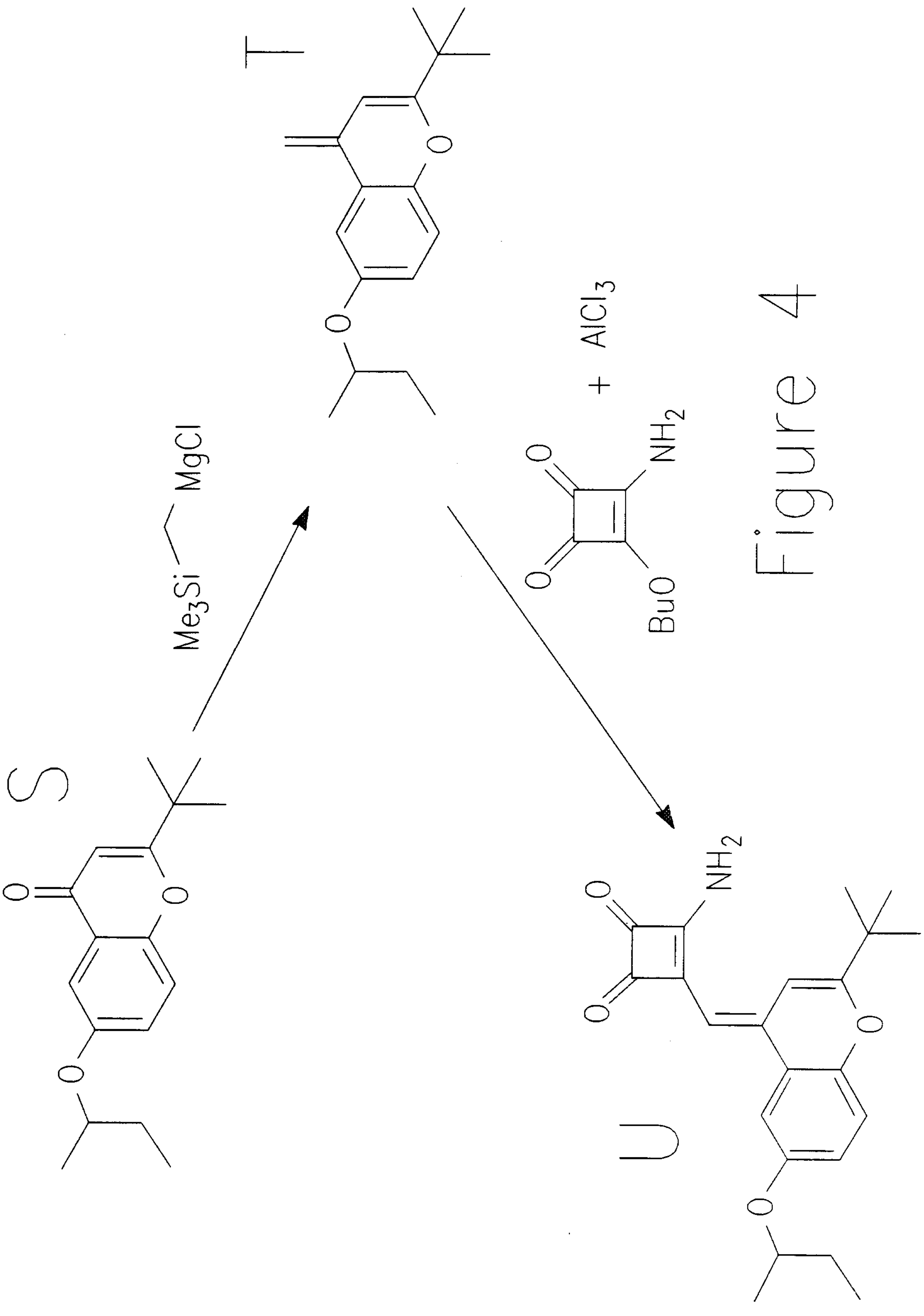


Figure 4

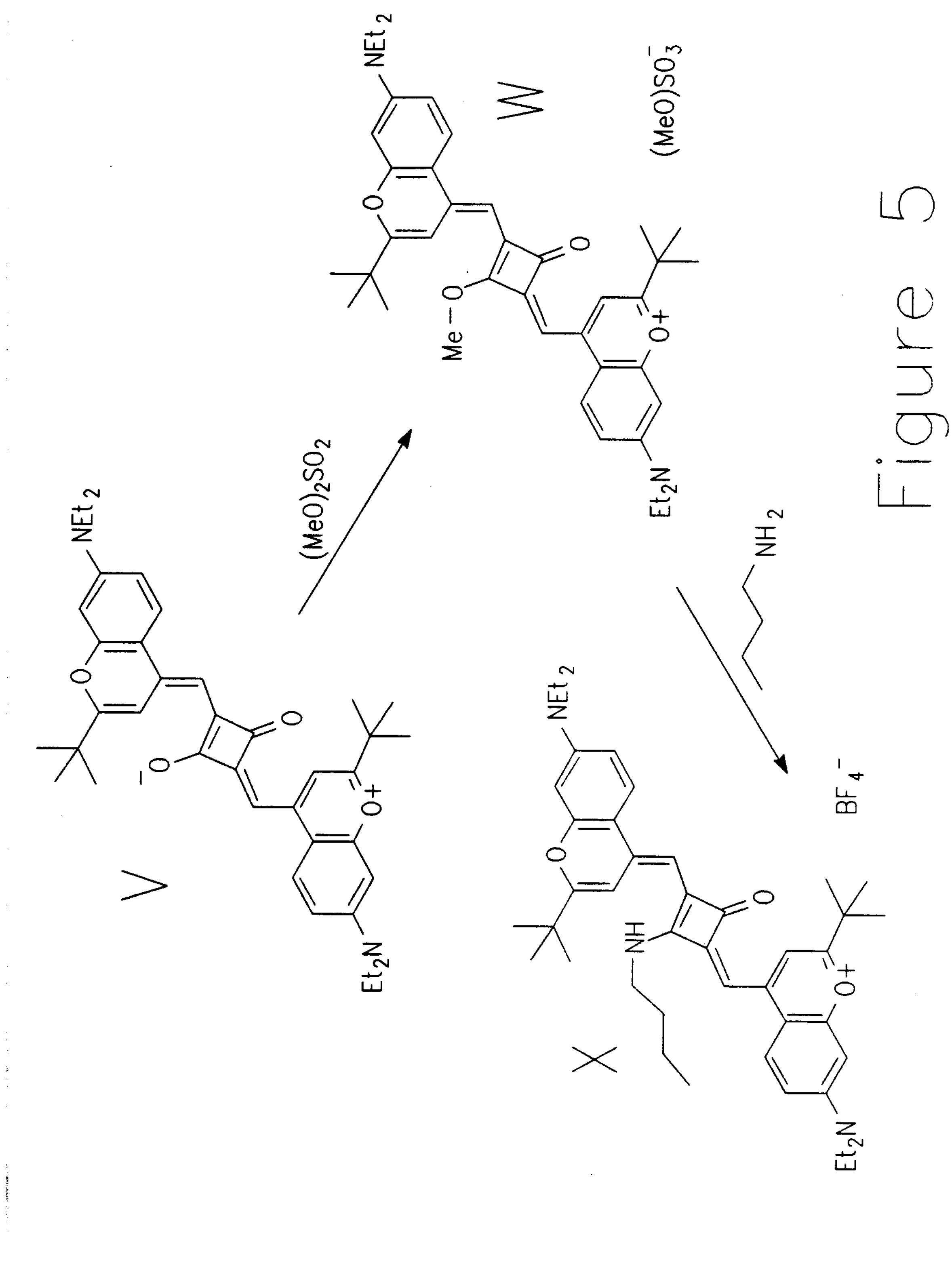


V

W

X

Figure 5

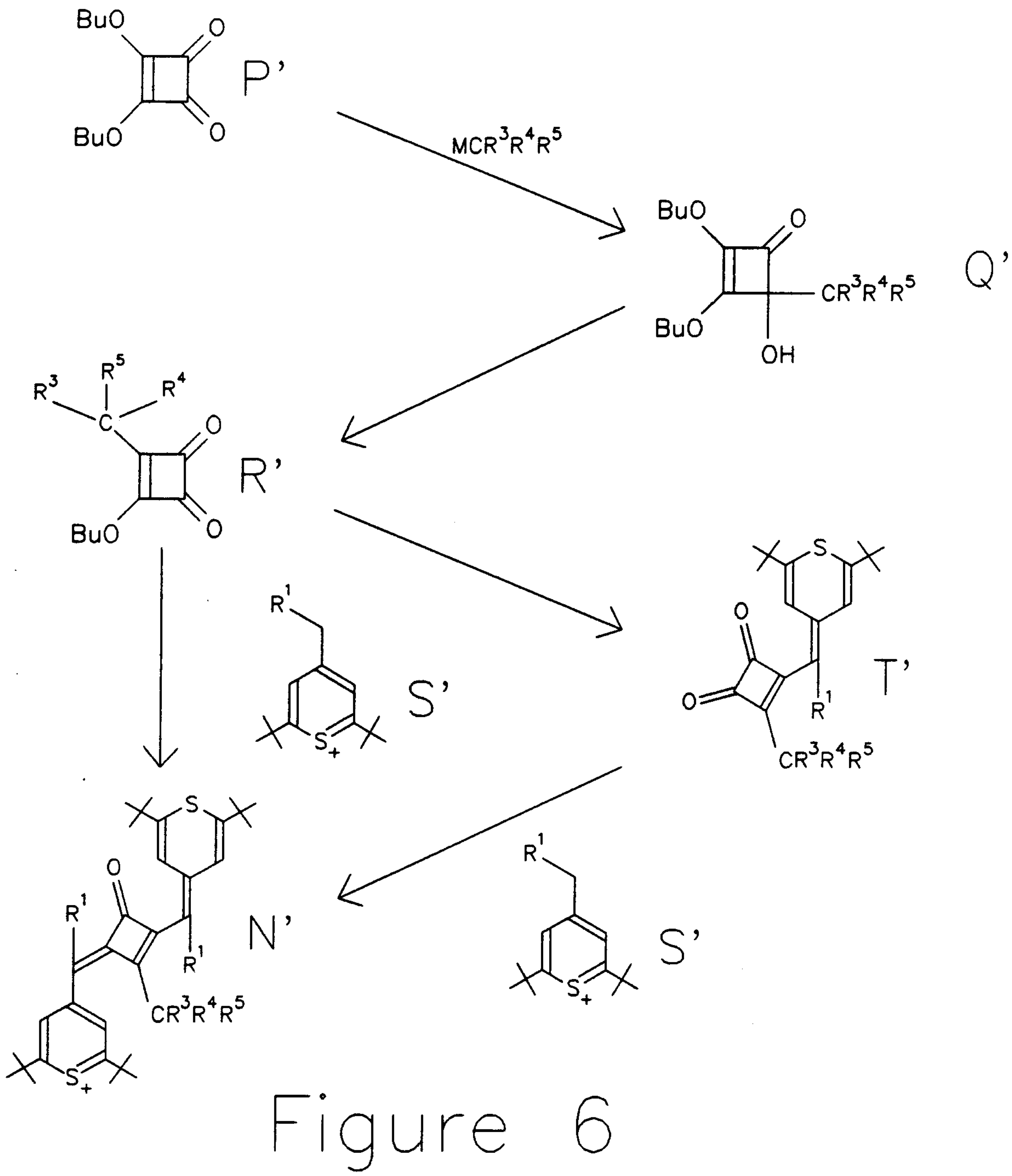


P' Q' R' S' T' N' S'

Figure 6

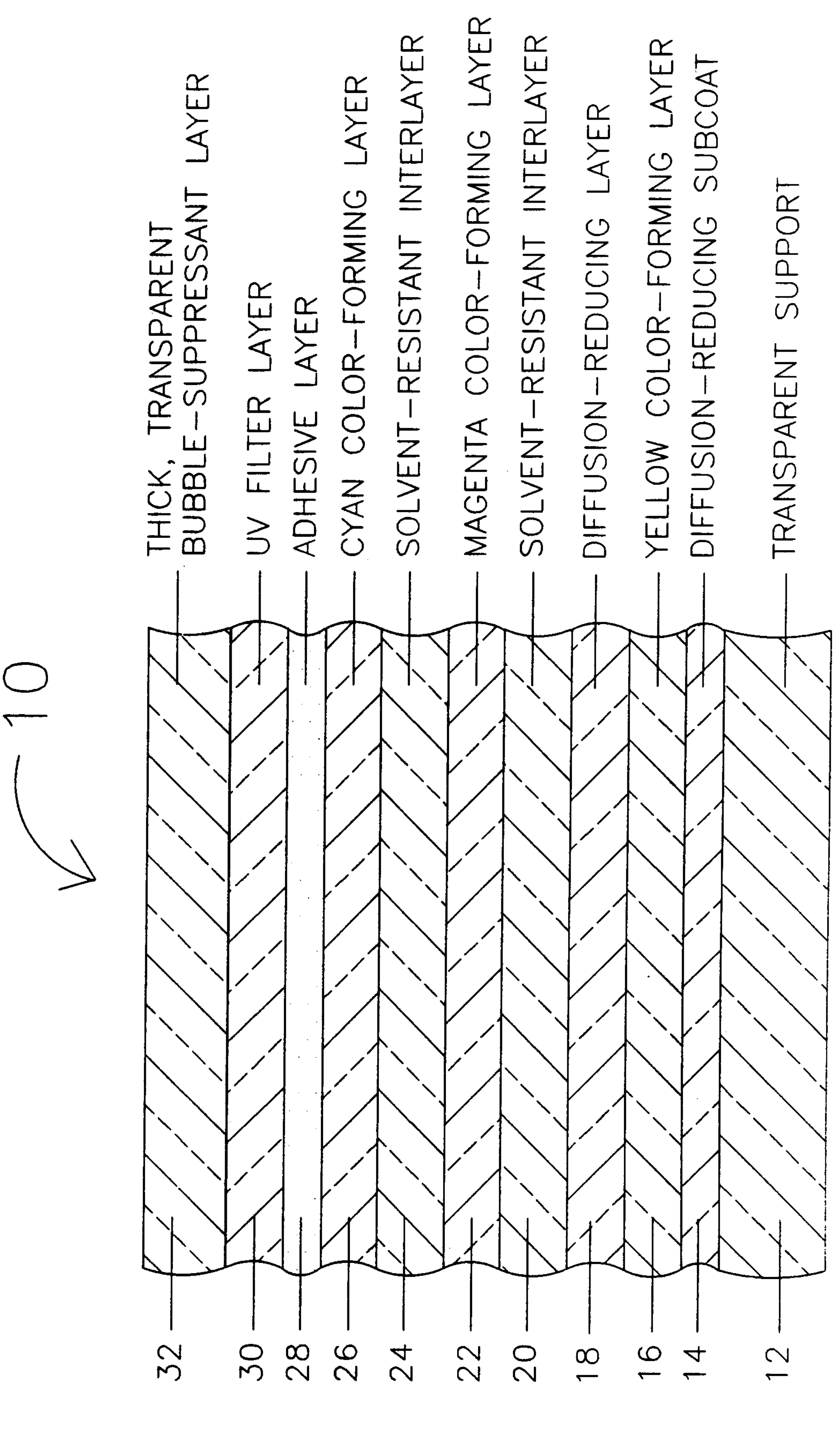
10
THICK, TRANSPARENT BUBBLE–SUPPRESSANT LAYER
UV FILTER LAYER
ADHESIVE LAYER
CYAN COLOR–FORMING LAYER
SOLVENT–RESISTANT INTERLAYER
MAGENTA COLOR–FORMING LAYER
SOLVENT–RESISTANT INTERLAYER
DIFFUSION–REDUCING LAYER
YELLOW COLOR–FORMING LAYER
DIFFUSION–REDUCING SUBCOAT
TRANSPARENT SUPPORT
32
30
28
26
24
22
20
18
16
14
12

Figure 7